(19) Europäisches Patentamt
European Patent Office
Office européen des brevets

(11) EP 4 383 990 A1

(12) EUROPEAN PATENT APPLICATION

(43) Date of publication:
**12.06.2024 Bulletin 2024/24**

(21) Application number: **23215041.7**

(22) Date of filing: **07.12.2023**

(51) International Patent Classification (IPC):
***H10K 85/60*** *(2023.01)* *H10K 30/30* *(2023.01)*

(52) Cooperative Patent Classification (CPC):
**H10K 85/6576; H10K 85/621; H10K 85/657;**
H10K 30/30

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **08.12.2022 US 202263386569 P**
**01.12.2023 US 202318526530**

(71) Applicants:
- **The Regents Of The University Of Michigan**
**Ann Arbor, Michigan 48109 (US)**
- **The University of Southern California**
**Los Angeles, CA 90015 (US)**

(72) Inventors:
- **FORREST, Stephen R.**
**Ann Arbor, MI, 48109 (US)**
- **LI, Yongxi**
**Ann Arbor, MI, 48109 (US)**
- **THOMPSON, Mark E.**
**Los Angeles, CA, 90015 (US)**
- **MENCKE, Austin**
**Los Angeles, CA, 90015 (US)**
- **KANDAPPA, Sunil**
**Los Angeles, CA, 90015 (US)**

(74) Representative: **Maiwald GmbH**
**Elisenhof**
**Elisenstraße 3**
**80335 München (DE)**

(54) **MATERIALS FOR OPTOELECTRONIC APPLICATIONS**

(57) Provided are compounds of Formula I. Also provided are formulations comprising these compounds. Further provided are optoelectronic devices that utilize these compounds.

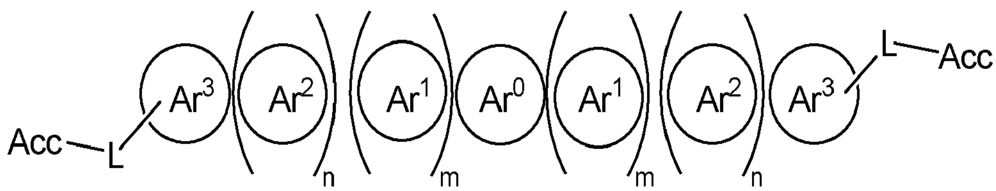

Formula I

EP 4 383 990 A1

## Description

CROSS-REFERENCE TO RELATED APPLICATIONS

The present application claims priority to U.S. Provisional Application No. 63/386,569, filed December 8, 2022, and to U.S. Nonprovisional Application No. 18/526,530, filed December 1, 2023, which are incorporated by reference herein in their entirety.

STATEMENT REGARDING FEDERALLY SPONSORED RESEARCH OR DEVELOPMENT

This invention was made with government support under Grant No. N00014-17-1-2211, awarded by the U.S. Department of the Navy; Grant No. N00014-20-1-2183, awarded by the U.S. Department of the Navy; and Grant No. DE-EE0008561, awarded by the U.S. Department of Energy. The government has certain rights in the invention.

BACKGROUND

Optoelectronic devices rely on the optical and electronic properties of materials to either produce or detect electromagnetic radiation electronically or to generate electricity from ambient electromagnetic radiation. Opto-electronic devices that make use of organic materials are becoming increasingly desirable for a number of reasons. Many of the materials used to make such devices are relatively inexpensive, so organic opto-electronic devices have the potential for cost advantages over inorganic devices. In addition, the inherent properties of organic materials, such as their flexibility, may make them well suited for particular applications such as fabrication on a flexible substrate. Examples of organic opto-electronic devices include organic light emitting diodes/devices (OLEDs), organic phototransistors, organic photovoltaic cells, and organic photodetectors.

Organic photovoltaic cells (OPVs) have sparked considerable interest in recent years owing to their flexibility, light weight, non-toxic nature and semi-transparency that makes them ideal for building integrated and building applied applications. Compared to the commercial solar modules that typically have power conversion efficiencies of PCE = 15-22% of sunlight, organics have improved from 10% in 2016 to over 17% in 2019 for single junction devices. Advances have steadily continued, headed toward single junction thermodynamically limited efficiencies of ~25%, with potentially higher efficiencies based on multijunction cells. This rapid advance has been paced by the development of non-fullerene acceptors (NFAs).

An essential feature of all high-performance photovoltaic devices that exhibit an acceptable level of reproducibility and a long operational lifetime, is that the materials of which are they comprised are of high purity. In crystalline Si photovoltaic cells, the chemical purity of Si has been an important limiting factor in their performance. The introduction of extrinsic impurities can result in significant changes in conductivity by creating lattice strain or by forming interstitial and substitutional defects. As a consequence, the precise control of source material purity is a common strategy for improving the efficiency and reliability of photovoltaics.

In contrast to inorganic photovoltaics, understanding the effects of impurities on organic photovoltaics (OPVs) has received little attention despite their potentially profound impact on performance. In fact, the molecular contaminants left over from synthesis, or that result from decomposition during device operation, can dramatically impact the intrinsic optical and electrical properties of pristine OPV materials (Mateker, W. R. et al. Energy Environ. Sci. 6, 2529-2537 (2013); Salzman, R. F. et al. Org. Electron. 6, 242-246 (2005); Tetreault, et al., Org. Electron. 92, 106091 (2021)). One example is the emergence of photoinduced dimerized products of the fullerene acceptor, $C_{60}$, during OPV operation, which reduces the exciton lifetime and diffusion length, leading to a significant decrease in device lifetime (Wang, N., et al., Sol. Energy Mater. Sol. Cells 125, 170-175 (2014)). Beyond these changes, impurities can disrupt the crystalline order in molecular solids (Forrest, S. R., et al., J. Appl. Phys. 56, 543-551 (1984)), thereby reducing the charge carrier mobility and cell power conversion efficiency (PCE). Therefore, considerable care must be taken to remove contaminants from the source materials, and avoid material decomposition during device preparation and operation.

Ternary blend OPVs comprising one donor and two acceptors, or one acceptor and two donors have attracted great interest in recent years (Lu, L., et al., Nat. Photonics 8, 716-722 (2014); Gasparini, N., et al., Nat. Rev. Mater. 4, 229-242 (2019); Yu, R., et al., Adv. Energy Mater. 8, 1702814 (2018); Li, Y. et al. J. Am. Chem. Soc. 141, 18204-18210 (2019); Li, Y. et al. Adv. Mater. 30, 1804416 (2018)). Compared with conventional binary component devices, the introduction of a third molecular species into the bulk heterojunction (BHJ) layer can increase solar spectral coverage, reduce the energy loss, and significantly improve OPV efficiency. In particular, the rapid development of acceptor (A) - donor (D) - acceptor (A) non-fullerene acceptors (NFAs) has led to ternary blend OPVs comprising two NFAs and a polymer donor with PCE approaching 20% under 1 sun intensity, AM 1.5G illumination (Cui, Y. et al. Adv. Mater. 33, 2102420 (2021); Zuo, L. et al. Nat. Nanotechnol. 17, 53-60 (2022); Zhan, L. et al. Adv. Energy Mater. 12, 2201076 (2022); Hultmark, S. et al. Adv. Funct. Mater. 30, 2005462 (2020)).

## SUMMARY OF THE DISCLOSURE

In one aspect, the present disclosure relates to a compound of Formula I:

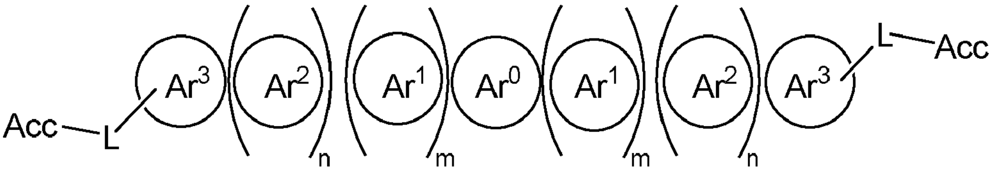

Formula I

wherein $Ar^0$ is an aromatic or non-aromatic ring connected to or fused at two positions to $Ar^1$, $Ar^2$, or $Ar^3$;
each $Ar^1$ is an aromatic ring or a non-aromatic ring that is connected to or fused to $Ar^{\circ}$ and to $Ar^2$, $Ar^3$, or another occurrence of $Ar^1$;
each $Ar^2$ is an aromatic ring or a non-aromatic ring that is connected to or fused to $Ar^1$ or $Ar^{\circ}$ and to $Ar^3$ or another occurrence of $Ar^2$;
$Ar^3$ is an aromatic ring that is connected to or fused to $Ar^2$, $Ar^1$, or $Ar^0$ and is connected to or fused to A;
m is from 0 to 10;
n is from 0 to 10;
Acc is an acceptor group connected or fused to $Ar^3$;
L is a linking group selected from the group consisting of a single bond and 1,2-cyclopentene;
$Ar^{\circ}$ represented by one of the following structures:

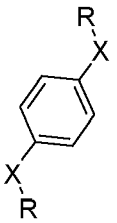 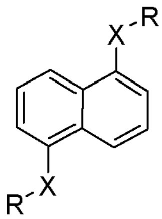 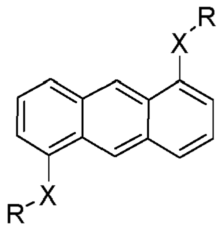 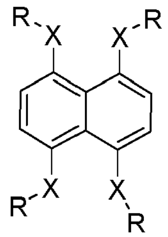 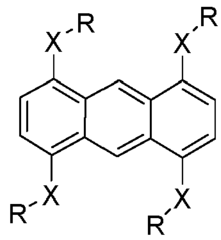

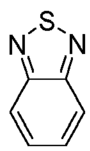 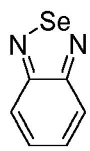 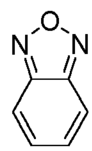 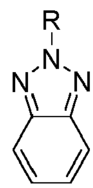 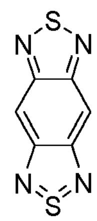 ;

each occurrence of $Ar^1$ is independently represented by one of the following structures:

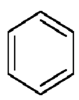 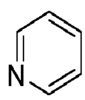 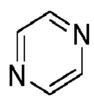 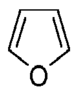 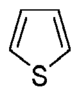 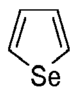 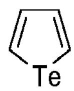

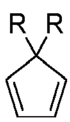 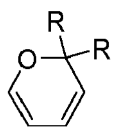 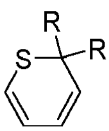 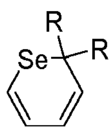 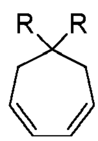

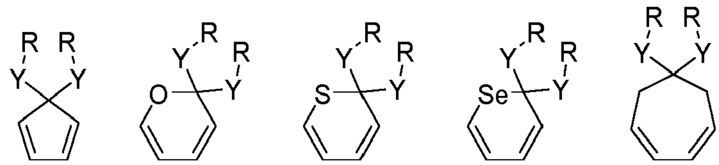

each occurrence of $Ar^2$ is independently represented by one of the following structures:

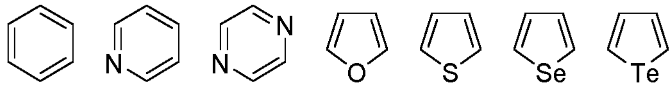

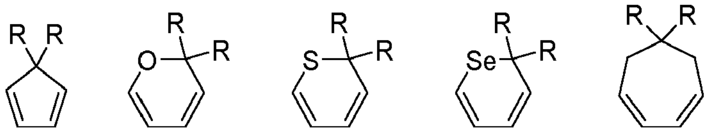

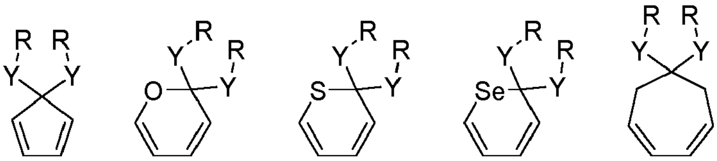
;

each occurrence of Y is represented by one of the following linking groups:

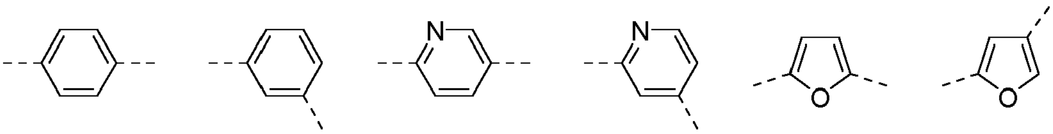

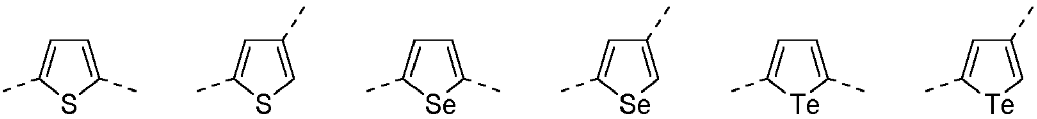

$Ar^3$ is represented by one of the following structures:

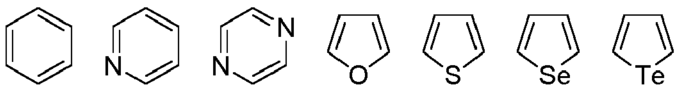

each occurrence of X is a single bond or a heteroatom selected from the group consisting of O, S, Se, and NR; Acc is connected to ring $Ar^3$ by a single bond and is represented by one of the following structures:

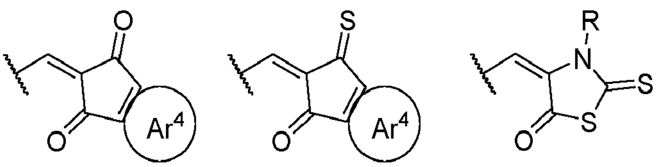
;

or

Acc is fused to ring $Ar^3$ and is represented by one of the following structures, wherein waved lines indicate connection to adjacent carbon atoms on $Ar^3$:

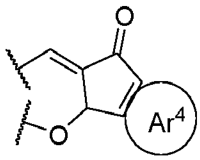 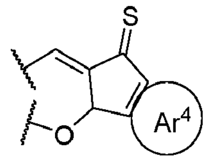 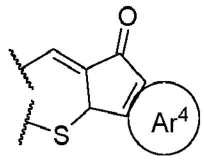 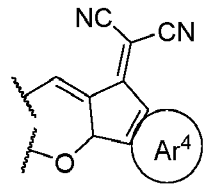

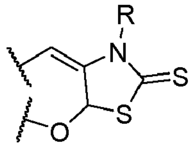 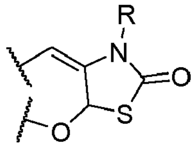 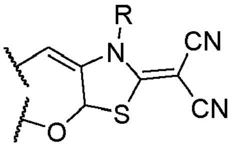

$Ar^4$ is represented by one of the following structures:

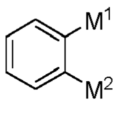 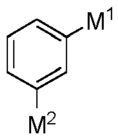 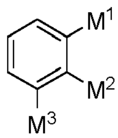 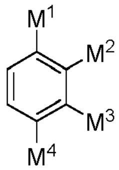 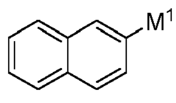 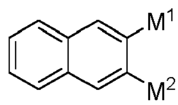 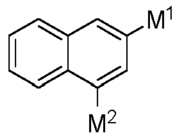

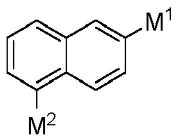 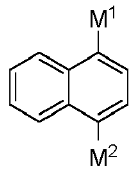 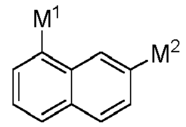 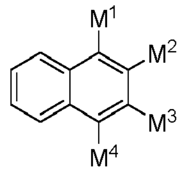

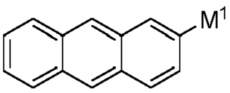 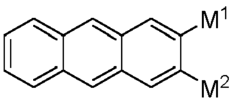 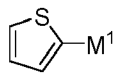 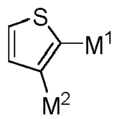 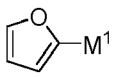 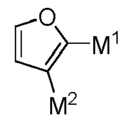

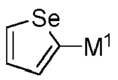 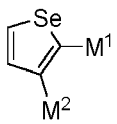 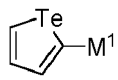 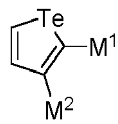 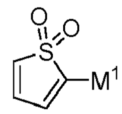 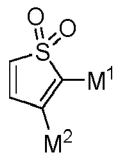

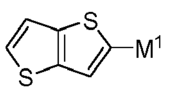 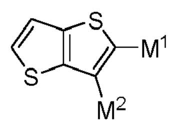 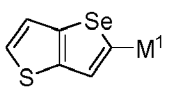 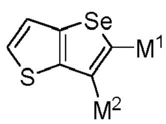 ;

$M^1$, $M^2$, $M^3$, and $M_4$ are individually selected from the group consisting of hydrogen, fluorine, chlorine, bromine, iodine, astatine, and a cyano group, wherein at least one of $M_1$-$M_4$ is a halogen
each R independently represents hydrogen or a substituent selected from the group consisting of deuterium, halogen, alkyl, cycloalkyl, heteroalkyl, heterocycloalkyl, arylalkyl, alkoxy, aryloxy, amino, thioalkyl, silyl, alkenyl, cycloalkenyl, heteroalkenyl, alkynyl, aryl, heteroaryl, acyl, carboxylic acid, ether, ester, nitrile, isonitrile, sulfanyl, sulfinyl, sulfonyl, phosphino, and combinations thereof; wherein any two adjacent substituents optionally join to form a ring.

In another aspect, the present disclosure provides a formulation comprising a compound of Formula I as described herein.

In yet another aspect, the present disclosure provides an optoelectronic device comprising a compound of Formula I as described herein.

BRIEF DESCRIPTION OF THE DRAWINGS

For a more complete understanding of the disclosure, reference is made to the following detailed description and accompanying drawing figures, in which like reference numerals may be used to identify like elements in the figures.

Fig. 1 is a representation of the generality of the end-capping exchange reaction between A-D-A non-fullerene acceptors. The top panel displays the end-capping exchange reaction between two A-D-A non-fullerene acceptors (NFAs). The vinyl groups linking the D and A moieties can dissociate at elevated temperature used during the mixing of the blend prior to forming the bulk heterojunction (BHJ) thin film. The exchange process results in reaction products with both asymmetric and symmetric molecular structures. Consequently, ternary-blend organic photovoltaics (OPVs) based on two NFAs and a donor can comprise up to seven chemical species. The chemical structures of the molecules are displayed in the bottom panel, and listed in Table 1.

Fig. 2 depicts the C=C/C=C exchange reactions between BT-IC and BTIC-4Cl (F) in chlorobenzene (CB) solution in the dark.

Fig. 3 is a comparison of the $^{1}$H NMR spectra of neat BT-IC, BTIC-4Cl, BTIC-2Cl vs. the physical blend solution of BT-IC:BTIC-4Cl (1:1, w/w).

Fig. 4 depicts, at left, thin layer chromatography for neat BTIC-4Cl, BTIC-2Cl and a BT-IC:BTIC-4Cl mixture; and at right, matrix-assisted laser desorption ionization with time-of-flight mass spectrometry (MALDI-TOF-MS) analysis of BT-IC:BTIC-4Cl blend solutions

Fig. 5 shows the $^{19}$F{$^{1}$H} NMR spectra of purified BTIC-4F (panel 1) and BTIC-2F (2), compared to those of BT-IC: BTIC-4F (1:1, w/w) blend solutions and films under different conditions: (3) BT-IC blended with BTIC-4F in the CB solution at $T$ = 65 °C in the dark, (4) the film obtained from the 0 °C solution during blending, then aged at 72 h at 100 °C under nitrogen; (5) BT-IC blended with BTIC-4F under one sun intensity, simulated AM 1.5 G illumination at $T$ = 0 °C for 2.5 h; (6) blend film obtained from the 0 °C solution, then aged 48 h under sun intensity, simulated AM 1.5 G illumination.

Fig. 6 is a comparison of the $^{19}$F{$^{1}$H} NMR spectra of the BT-IC:BTIC-4F (1:1, w/w) mixture after stirring in chlorobenzene for 24h at temperatures ranging from 0 °C to 100 °C

Fig. 7 is a plot of the absorption spectra vs. exposure time in the dark, at $T$ = 65 °C or one sun intensity, simulated AM 1.5 G illumination at 0 °C.

Fig. 8 shows the thin layer chromatography for neat BTIC-4F, IT-IC and IT-IC:BTIC-4F hot mixture and a comparison of the $^{1}$H NMR spectra of neat IT-IC, BTIC-4F, BTIC-2F, ITIC-2F, ITIC-4F, BT-IC and the IT-IC:BTIC-4F hot blend solution.

Fig. 9 shows a comparison of the $^{1}$H NMR spectra of neat ITIC, Y16-4F vs. the ITIC:Y16-4F (1:1, w/w) hot mixture and a comparison of the $^{19}$F{$^{1}$H} NMR spectra of neat Y16-4F, ITIC-2F, ITIC-4F vs. ITIC:Y16-4F (1:1, w/w) hot mixture.

Fig. 10 shows a comparison of the $^{1}$H NMR spectra of neat Y16, Y16-4F vs. the Y6:Y16-4F (1:1, w/w) hot mixture and a comparison of the $^{19}$F{$^{1}$H} NMR spectra of neat Y16-4F vs. the Y6:Y16-4F (1:1, w/w) hot mixture. Note that the $^{1}$H NMR spectrum of Y16 + Y16-4F mixture resembles the signal of the Y16 and Y16-4F spectra, the product is indistinguishable from the blend of reactants. However, the product can be identified by two distinct doublet fluorine (F) peaks.

Fig. 11 shows the thin layer chromatography for neat BPT, IT-IC, cold and hot BTP:IT-IC mixture, and a comparison of the $^{1}$H NMR spectra of neat BPT, IT-IC vs. the hot blend solution.

Fig. 12 shows a comparison of the $^{1}$H NMR spectra of neat 6TIC-4F, BT-IC vs. the blend solution and a comparison of the $^{19}$F{$^{1}$H} NMR spectra of neat 6TIC-4F vs. the blend solution.

Fig. 13 shows a comparison of the $^{1}$H NMR spectra of neat BT-IC, BEIT-4F vs. the blend solution and a comparison of the $^{19}$F{$^{1}$H} NMR spectra of neat BEIT-4F vs. the blend solution.

Fig. 14 shows a comparison of the $^1H$ NMR spectra of neat ITIC-4F, ITIC-DM vs. the blend solution and a comparison of the $^{19}F\{^1H\}$ NMR spectra of neat ITIC-4F vs. the blend solution.

Fig. 15 shows a comparison of the $^1H$ NMR spectra of neat IDTIDT-IC, BTIC-4F vs. the blend solution and a comparison of the $^{19}F\{^1H\}$ NMR spectra of neat BTIC-4F vs. the blend solution

Fig. 16 shows the reaction represented in Fig. 17.

Fig. 17 shows a comparison of the $^{19}F\{^1H\}$ NMR spectra of neat ITIC-4F, ITIC-2F vs. the blend solution of ITIC-4F and Y1 and a comparison of the $^{19}F\{^1H\}$ NMR spectra of neat N3 vs. the blend solution of BT-IC and N3.

Fig. 18 is a schematic of the device structure showing layer thicknesses and compositions for reaction time studies.

Fig. 19 depicts plots of open circuit voltage, $V_{OC}$ and short circuit current density, *Jsc,* vs. reaction time in the dark at $T$ = 65 °C.

Fig. 20 depicts plots of power conversion efficiency, PCE and fill factor, *FF*, vs. reaction time in the dark at $T$ = 65 °C.

Fig. 21 depicts thin layer chromatography for the PCE-10:BT-IC:BTIC-4Cl active layer blend solution held at $T$ = 65 °C for different reaction times. Note that the impurities in the BHJ increase with time, where after 24 h, the presence of BTIC-2Cl is clearly detected.

Fig. 22 depicts the evolution of the solar cell performance parameters over PCE-10:IT-IC:BTIC-4F (1:1:1, w/w/w) blend solution reaction time. Power conversion efficiency, PCE; open circuit voltage, $V_{OC}$; short circuit current, *Jsc* and fill factor, *FF,* plotted vs. reaction time in the dark, at $T$ = 65 °C.

Fig. 23 depicts the evolution of the solar cell performance parameters over PCE-10:IT-IC:Y16-4F (1:1:1, w/w/w) blend solution reaction time. Power conversion efficiency, PCE, $V_{OC}$, *Jsc* and *FF,* plotted vs. reaction time in the dark, at $T$ = 65 °C.

Fig. 24 is a schematic of the device structure for reliability studies. A self-assembled monolayer (IC-SAM) and a 2-nm-thick $C_{70}$ layer prevent chemical changes at the active layer/inorganic interfaces.

Fig. 25 is a plot of normalized PCE and JSC vs. aging time under white light illumination equivalent to 10 ± 1 suns.

Fig. 26 is a plot of Normalized VOC and FF vs. aging time under white light illumination equivalent to 10 ± 1 suns.

Fig. 27 depicts the chemical structures of four model compounds used to investigate the exchange mechanism.

Fig. 28 depicts the $^1H$ NMR spectrum of as-obtained materials, products, and the crude mixture. The mixture obtained by refluxing PhIC and $NMe_2$PhBarb together overnight in CB results in a color change for the solution from orange-yellow to deep red.

Fig. 29 is a comparison of the $^1H$ NMR spectrum of benzaldehyde, 4-dimethylaminobenzaldehyde and the intermediate aldehyde formed in the reaction mixture in hydrous CB.

Fig. 30 depicts the $^1H$ NMR spectra of a crude mixture of PhIC and NMe2PhBarb in anhydrous CB and introducing 1 M equiv. water into the mixture. The mixture was obtained by refluxing PhIC and $NMe_2$PhBarb together overnight. Note that the intermediate aldehyde is formed from the aqueous reaction mixture.

Fig. 31 is a comparison of the $^{19}F\{^1H\}$ NMR spectra of the crude mixture of BTIC and BTIC-4F in anhydrous $CDCl_3$ and introducing 0.3% water into the mixture. The mixture was obtained by refluxing BTIC and BTIC-4F at 50 °C for 16h. Note that the BTIC-2F is formed from the aqueous reaction mixture.

Fig. 32 depicts the $^1H$ NMR spectrum of $NMe_2$PhIC, PhIC, and the crude mixture. The mixture was obtained by refluxing $NMe_2$PhIC and benzaldehyde together for 48 h in anhydrous CB solution. The $^1H$-NMR spectroscopy revealed only the starting material, $NMe_2$PhIC.

Fig. 33 depicts the $^1H$ NMR spectrum of $NMe_2PhIC$, $NMe_2PhBarb$, IC, and the crude mixture. The mixture was obtained by refluxing $NMe_2PhIC$ and 1,3-dimethylbarbiutate together for 48 h in anhydrous CB solution. No trace of aldehyde protons were observed.

Fig. 34 depicts proposed mechanisms for the end-capping exchange reaction through formation of an aldehyde intermediate.

Fig. 35 shows the effect of end-capping units on the reactivity of exchange process. At left are the chemical structures of the investigated molecules. At right is a comparison of the $^{19}F\{^1H\}$ NMR spectra of the crude mixture of BTIC-4F and investigated molecule. The mixture was obtained by stirring in chlorobenzene at 65 °C for 24h.

Fig. 36 depicts the two dimensional (2D) grazing incidence wide-angle x-ray scattering (GIWAXS) patterns of BT-IC (panel 1) and BTIC-2Cl (2) neat films prepared from cold solutions.

Fig. 37 depicts the two dimensional (2D) grazing incidence wide-angle x-ray scattering (GIWAXS) patterns of BTIC-4Cl (3) neat film prepared from cold solution and BT-IC: BTIC-4Cl (1:1, by wt.) blend film prepared from cold solution (4).

Fig. 38 depicts the two dimensional (2D) grazing incidence wide-angle x-ray scattering (GIWAXS) patterns of BT-IC: BTIC-4Cl (1:1, by wt.) blend film prepared from hot solution (5), and BT-IC:BTIC-2Cl:BTIC-4Cl (1:1:1, by wt.) blend film prepared from cold solution (6).

Fig. 39 is plot of in-plane (dotted line) and out-of-plane (solid line) x-ray scattering profiles extracted from 2D GIWAXS data.

Fig. 40 is a plot of azimuthal intensity distributions along $q = 0.54 \pm 0.05$ extracted from 2D GIWAXS data.

Fig. 41 is a plot of the resonant soft X-ray scattering (RSoXS) of BT-IC:BTIC-4Cl cold and hot blends. Here, Q is the scattering vector.

Fig. 42 depicts the 2D grazing incidence wide-angle x-ray scattering (GIWAXS) patterns of PCE-10:BT-IC:BTIC-4Cl: cold, fresh; cold, aged; hot, fresh; and hot, aged blend films. Aging is under 10 sun illumination intensity for 48h.

Fig. 43 is a plot of in-plane (left) and out-of-plane (right) sector-averaged profiles extracted from 2D GIWAXS data, $q$ is the scattering vector.

Fig. 44 depicts the EL spectra of a cold PCE-10: BT-IC: BTIC-4Cl (1:0.75:0.75, by wt.) OPV with Gaussian fits using two binary (PCE-10:BT-IC and PCE-10:BTIC-4Cl) CT state emission peaks.

Fig. 45 depicts the EL spectra of hot ternary (PCE-10: BT-IC: BTIC-4Cl (1 :0.75:0.75, by wt.)) device with Gaussian fits to three binary CT state emission peaks: viz. PCE-10:BT-IC, PCE-10:BTIC-4Cl and PCE-10:BTIC-2Cl.

Fig. 46 depicts EL spectra of (top left) PCE-10: BT-IC: (1:1.5, by wt.) OPV with Gaussians fits using BT-IC exciton peak at 1.46 eV and binary CT peak at 1.36 eV (top right) PCE-10:BTIC-4Cl (1:1.5, by wt.) with Gaussian fits of BTIC-4Cl exciton peak at 1.38 eV and binary CT peak at 1.15 eV (bottom) PCE-10:BTIC-2Cl (1:1.5, by wt.) with Gaussian fits of BTIC-2Cl exciton peak at 1.40 eV and binary CT peak at 1.22 eV

Fig. 47 depicts the EL spectra of cold and hot ternary devices based on PCE-10: BT-IC: BTIC-4Cl (1:0.75:0.75, by wt.) before and after aging for 192h under illumination equivalent to 10 suns intensity.

Fig. 48 depicts the measured changes in open-circuit voltage ($\Delta V_{OC}$) over time for cold and hot ternary devices (data points) along with $\Delta V_{OC}$ calculated from the loss in EL intensity (lines).

Fig. 49 depicts the normalized voltage-dependent short circuit photocurrent of cold and hot ternary devices before and after aging for 265 h under white light illumination equivalent to 10 suns intensity.

Fig. 50 is a graph of charge-extraction efficiency at V = 0 (extracted from the reverse-bias photocurrent data, as described in Methods) plotted against time.

Fig. 51 depicts the 2D grazing incidence wide-angle x-ray scattering (GIWAXS) patterns of BT-IC, BTIC-2Cl and BTIC-4Cl neat films that are prepared from hot solutions.

DETAILED DESCRIPTION

The present disclosure provides non-fullerene acceptors (NFAs) with good photostability and morphological stability. In some aspects, employing these NFAs in the one or more device architectures, OPV device is achieved with improved the operational lifetime.

Various non-limiting examples of OPVs and compositions within various layers of an OPV are described in greater detail below.

Definitions

As used herein, the term "organic" includes polymeric materials as well as small molecule organic materials that may be used to fabricate organic opto-electronic devices. "Small molecule" refers to any organic material that is not a polymer, and "small molecules" may actually be quite large. Small molecules may include repeat units in some circumstances. For example, using a long chain alkyl group as a substituent does not remove a molecule from the "small molecule" class. Small molecules may also be incorporated into polymers, for example as a pendent group on a polymer backbone or as a part of the backbone. Small molecules may also serve as the core moiety of a dendrimer, which consists of a series of chemical shells built on the core moiety. The core moiety of a dendrimer may be a fluorescent or phosphorescent small molecule emitter. A dendrimer may be a "small molecule," and it is believed that all dendrimers currently used in the field of optoelectronic devices are small molecules.

As used herein, "top" means furthest away from the substrate, while "bottom" means closest to the substrate. Where a first layer is described as "disposed over" a second layer, the first layer is disposed further away from substrate. There may be other layers between the first and second layer, unless it is specified that the first layer is "in contact with" the second layer. For example, a cathode may be described as "disposed over" an anode, even though there are various organic layers in between.

As used herein, "solution processable" means capable of being dissolved, dispersed, or transported in and/or deposited from a liquid medium, either in solution or suspension form.

A ligand may be referred to as "photoactive" when it is believed that the ligand directly contributes to the photoactive properties of an emissive material. A ligand may be referred to as "ancillary" when it is believed that the ligand does not contribute to the photoactive properties of an emissive material, although an ancillary ligand may alter the properties of a photoactive ligand.

As used herein, the terms "electrode" and "contact" may refer to a layer that provides a medium for delivering current to an external circuit or providing a bias current or voltage to the device. For example, an electrode, or contact, may provide the interface between the active regions of an organic photosensitive optoelectronic device and a wire, lead, trace or other means for transporting the charge carriers to or from the external circuit. Examples of electrodes include anodes and cathodes, which may be used in a photosensitive optoelectronic device.

As used herein, the term "transparent" may refer to a material that permits at least 50% of the incident electromagnetic radiation in relevant wavelengths to be transmitted through it. In a photosensitive optoelectronic device, it may be desirable to allow the maximum amount of ambient electromagnetic radiation from the device exterior to be admitted to the photoconductive active interior region. That is, the electromagnetic radiation must reach a photoconductive layer(s), where it can be converted to electricity by photoconductive absorption. This often dictates that at least one of the electrical contacts or electrodes should be minimally absorbing and minimally reflecting of the incident electromagnetic radiation. In some cases, such a contact should be transparent or at least semi-transparent. In one embodiment, the transparent material may form at least part of an electrical contact or electrode.

As used herein, the term "semi-transparent" may refer to a material that permits some, but less than 50% transmission of ambient electromagnetic radiation in relevant wavelengths. Where a transparent or semi-transparent electrode is used, the opposing electrode may be a reflective material so that light which has passed through the cell without being absorbed is reflected back through the cell.

As used and depicted herein, a "layer" refers to a member or component of a device, for example an optoelectronic device, being principally defined by a thickness, for example in relation to other neighboring layers, and extending outward in length and width. It should be understood that the term "layer" is not necessarily limited to single layers or sheets of materials. In addition, it should be understood that the surfaces of certain layers, including the interface(s) of such layers with other material(s) or layers(s), may be imperfect, wherein said surfaces represent an interpenetrating, entangled or convoluted network with other material(s) or layer(s). Similarly, it should also be understood that a layer may be discontinuous, such that the continuity of said layer along the length and width may be disturbed or otherwise interrupted by other layer(s) or material(s).

As used herein, a "photoactive region" refers to a region of a device that absorbs electromagnetic radiation to generate excitons. Similarly, a layer is "photoactive" if it absorbs electromagnetic radiation to generate excitons. The excitons may dissociate into an electron and a hole in order to generate an electrical current.

As used herein, the term "cathode buffer" is given its ordinary meaning in the art and generally refers to a material which is disposed between a cathode and a photoactive material. Generally, a cathode buffer material aids in reducing the work function of the cathode interface. Those of ordinary skill in the art will be able to select suitable cathode buffer materials with appropriate work functions for use in the methods and devices described herein.

As used herein, the term "anode buffer" is given its ordinary meaning in the art and generally refers to a material which is disposed between a anode and a photoactive material. Generally, a anode buffer material aids in reducing the work function of the anode interface. Those of ordinary skill in the art will be able to select suitable anode buffer materials with appropriate work functions for use in the methods and devices described herein.

As used herein, the terms "donor" and "acceptor" refer to the relative positions of the highest occupied molecular orbital ("HOMO") and lowest unoccupied molecular orbital ("LUMO") energy levels of two contacting but different organic materials. If the LUMO energy level of one material in contact with another is lower, then that material is an acceptor. Otherwise it is a donor. It is energetically favorable, in the absence of an external bias, for electrons at a donor-acceptor junction to move into the acceptor material, and for holes to move into the donor material.

As used herein, and as would be generally understood by one skilled in the art, a first work function is "greater than" or "higher than" a second work function if the first work function has a higher absolute value. Because work functions are generally measured as negative numbers relative to vacuum level, this means that a "higher" work function is more negative. On a conventional energy level diagram, with the vacuum level at the top, a "higher" work function is illustrated as further away from the vacuum level in the downward direction. Thus, the definitions of HOMO and LUMO energy levels follow a different convention than work functions.

As used herein, the term "band gap" (Eg) of a polymer may refer to the energy difference between the HOMO and the LUMO. The band gap is typically reported in electron volts (eV). The band gap may be measured from the UV-vis spectroscopy or cyclic voltammetry. A "low band gap" polymer may refer to a polymer with a band gap below 2 eV, e.g., the polymer absorbs light with wavelengths longer than 620 nm.

As used herein, the term "excitation binding energy" ($E_B$) may refer to the following formula: $E_B = (M^+ + M^-) - (M^* + M)$, where $M^+$ and $M^-$ are the total energy of a positively and negatively charged molecule, respectively; M* and M are the molecular energy at the first singlet state ($S_1$) and ground state, respectively. Excitation binding energy of acceptor or donor molecules affects the energy offset needed for efficient exciton dissociation. In certain examples, the escape yield of a hole increases as the HOMO offset increases. A decrease of exciton binding energy $E_B$ for the acceptor molecule leads to an increase of hole escape yield for the same HOMO offset between donor and acceptor molecules.

As used herein, "power conversion efficiency" (PCE) ($\eta_\rho$) may be expressed as:

$$\eta_\rho = \frac{V_{OC} * FF * J_{SC}}{P_O}$$

wherein $V_{OC}$ is the open circuit voltage, *FF* is the fill factor, *Jsc* is the short circuit current, and $P_O$ is the input optical power

As used herein, "spin coating" may refer to the process of solution depositing a layer or film of one material (i.e., the coating material) on a surface of an adjacent substrate or layer of material. The spin coating process may include applying a small amount of the coating material on the center of the substrate, which is either spinning at low speed or not spinning at all. The substrate is then rotated at high speed in order to spread the coating material by centrifugal force. Rotation is continued while the fluid spins off the edges of the substrate, until the desired thickness of the film is achieved. The applied solvent is usually volatile, and simultaneously evaporates. Therefore, the higher the angular speed of spinning, the thinner the film. The thickness of the film also depends on the viscosity and concentration of the solution and the solvent.

As used herein, and as would be generally understood by one skilled in the art, a first "Highest Occupied Molecular Orbital" (HOMO) or "Lowest Unoccupied Molecular Orbital" (LUMO) energy level is "greater than" or "higher than" a second HOMO or LUMO energy level if the first energy level is closer to the vacuum energy level. Since ionization potentials (IP) are measured as a negative energy relative to a vacuum level, a higher HOMO energy level corresponds to an IP having a smaller absolute value (an IP that is less negative). Similarly, a higher LUMO energy level corresponds to an electron affinity (EA) having a smaller absolute value (an EA that is less negative). On a conventional energy level diagram, with the vacuum level at the top, the LUMO energy level of a material is higher than the HOMO energy level of the same material. A "higher" HOMO or LUMO energy level appears closer to the top of such a diagram than a "lower" HOMO or LUMO energy level.

As used herein, and as would be generally understood by one skilled in the art, a first work function is "greater than" or "higher than" a second work function if the first work function has a higher absolute value. Because work functions

are generally measured as negative numbers relative to vacuum level, this means that a "higher" work function is more negative. On a conventional energy level diagram, with the vacuum level at the top, a "higher" work function is illustrated as further away from the vacuum level in the downward direction. Thus, the definitions of HOMO and LUMO energy levels follow a different convention than work functions.

The terms "halo," "halogen," and "halide" are used interchangeably and refer to fluorine, chlorine, bromine, and iodine.

The term "pseudohalogen" refers to polyatomic analogues of halogens, whose chemistry, resembling that of the true halogens, allows them to substitute for halogens in several classes of chemical compounds. Exemplary pseudohalogens include, but are not limited to, nitrile, cyaphide, isocyanide, cyanate, isocyanate, fulminate, thiocyanate, isothiocyanate, selenocyanate, tellurocyanate, azide, tetracarbonylcobaltate, trinitromethanide, and tricyanomethanide groups.

The term "acyl" refers to a substituted carbonyl radical ($C(O)-R_s$).

The term "ester" refers to a substituted oxycarbonyl ($-O-C(O)-R$, or $-C(O)-O-R_s$) radical.

The term "ether" refers to an $-OR_s$ radical.

The terms "sulfanyl" or "thio-ether" are used interchangeably and refer to a -SR, radical.

The term "sulfinyl" refers to a $-S(O)-R_s$ radical.

The term "sulfonyl" refers to a $-SO_2-R_s$ radical.

The term "phosphino" refers to a $-P(R_s)_3$ radical, wherein each $R_s$ can be same or different.

The term "silyl" refers to a $-Si(R_s)_3$ radical, wherein each $R_s$ can be same or different.

The term "boryl" refers to a $-B(R_s)_2$ radical or its Lewis adduct -B(Rs)3 radical, wherein Rs can be same or different.

In each of the above, $R_s$ can be hydrogen or a substituent selected from the group consisting of deuterium, halogen, alkyl, cycloalkyl, heteroalkyl, heterocycloalkyl, arylalkyl, alkoxy, aryloxy, amino, silyl, alkenyl, cycloalkenyl, heteroalkenyl, alkynyl, aryl, heteroaryl, and combination thereof. Preferred $R_s$ is selected from the group consisting of alkyl, cycloalkyl, aryl, heteroaryl, and combination thereof.

The term "alkyl" refers to and includes both straight and branched chain alkyl radicals. Preferred alkyl groups are those containing from one to fifteen carbon atoms and includes methyl, ethyl, propyl, 1-methylethyl, butyl, 1-methylpropyl, 2-methylpropyl, pentyl, 1-methylbutyl, 2-methylbutyl, 3-methylbutyl, 1,1-dimethylpropyl, 1,2-dimethylpropyl, 2,2-dimethylpropyl,and the like. Additionally, the alkyl group is optionally substituted.

The term "cycloalkyl" refers to and includes monocyclic, polycyclic, and spiro alkyl radicals. Preferred cycloalkyl groups are those containing 3 to 12 ring carbon atoms and includes cyclopropyl, cyclopentyl, cyclohexyl, bicyclo[3. 1. 1]heptyl, spiro[4.5]decyl, spiro[5.5]undecyl, adamantyl, and the like. Additionally, the cycloalkyl group is optionally substituted.

The terms "heteroalkyl" or "heterocycloalkyl" refer to an alkyl or a cycloalkyl radical, respectively, having at least one carbon atom replaced by a heteroatom. Optionally the at least one heteroatom is selected from O, S, N, P, B, Si and Se, preferably, O, S or N. Additionally, the heteroalkyl or heterocycloalkyl group is optionally substituted.

The term "alkenyl" refers to and includes both straight and branched chain alkene radicals. Alkenyl groups are essentially alkyl groups that include at least one carbon-carbon double bond in the alkyl chain. Cycloalkenyl groups are essentially cycloalkyl groups that include at least one carbon-carbon double bond in the cycloalkyl ring. The term "heteroalkenyl" as used herein refers to an alkenyl radical having at least one carbon atom replaced by a heteroatom. Optionally the at least one heteroatom is selected from O, S, N, P, B, Si, and Se, preferably, O, S, or N. Preferred alkenyl, cycloalkenyl, or heteroalkenyl groups are those containing two to fifteen carbon atoms. Additionally, the alkenyl, cycloalkenyl, or heteroalkenyl group is optionally substituted.

The term "alkynyl" refers to and includes both straight and branched chain alkyne radicals. Preferred alkynyl groups are those containing two to fifteen carbon atoms. Additionally, the alkynyl group is optionally substituted.

The terms "aralkyl" or "arylalkyl" are used interchangeably and refer to an alkyl group that is substituted with an aryl group. Additionally, the aralkyl group is optionally substituted.

The term "heterocyclic group" refers to and includes aromatic and non-aromatic cyclic radicals containing at least one heteroatom. Optionally the at least one heteroatom is selected from O, S, N, P, B, Si, and Se, preferably, O, S, or N. Hetero-aromatic cyclic radicals may be used interchangeably with heteroaryl. Preferred hetero-non-aromatic cyclic groups are those containing 3 to 7 ring atoms which includes at least one hetero atom, and includes cyclic amines such as morpholino, piperidino, pyrrolidino, and the like, and cyclic ethers/thio-ethers, such as tetrahydrofuran, tetrahydropyran, tetrahydrothiophene, and the like. Additionally, the heterocyclic group may be optionally substituted.

The term "aryl" refers to and includes both single-ring aromatic hydrocarbyl groups and polycyclic aromatic ring systems. The polycyclic rings may have two or more rings in which two carbons are common to two adjoining rings (the rings are "fused") wherein at least one of the rings is an aromatic hydrocarbyl group, e.g., the other rings can be cycloalkyls, cycloalkenyls, aryl, heterocycles, and/or heteroaryls. Preferred aryl groups are those containing six to thirty carbon atoms, preferably six to twenty carbon atoms, more preferably six to twelve carbon atoms. Especially preferred is an

aryl group having six carbons, ten carbons or twelve carbons. Suitable aryl groups include phenyl, biphenyl, triphenyl, triphenylene, tetraphenylene, naphthalene, anthracene, phenalene, phenanthrene, fluorene, pyrene, chrysene, perylene, and azulene, preferably phenyl, biphenyl, triphenyl, triphenylene, fluorene, and naphthalene. Additionally, the aryl group is optionally substituted.

The term "heteroaryl" refers to and includes both single-ring aromatic groups and polycyclic aromatic ring systems that include at least one heteroatom. The heteroatoms include, but are not limited to O, S, N, P, B, Si, and Se. In many instances, O, S, or N are the preferred heteroatoms. Hetero-single ring aromatic systems are preferably single rings with 5 or 6 ring atoms, and the ring can have from one to six heteroatoms. The hetero-polycyclic ring systems can have two or more rings in which two atoms are common to two adjoining rings (the rings are "fused") wherein at least one of the rings is a heteroaryl, e.g., the other rings can be cycloalkyls, cycloalkenyls, aryl, heterocycles, and/or heteroaryls. The hetero-polycyclic aromatic ring systems can have from one to six heteroatoms per ring of the polycyclic aromatic ring system. Preferred heteroaryl groups are those containing three to thirty carbon atoms, preferably three to twenty carbon atoms, more preferably three to twelve carbon atoms. Suitable heteroaryl groups include dibenzothiophene, dibenzofuran, dibenzoselenophene, furan, thiophene, benzofuran, benzothiophene, benzoselenophene, carbazole, indolocarbazole, pyridylindole, pyrrolodipyridine, pyrazole, imidazole, triazole, oxazole, thiazole, oxadiazole, oxatriazole, dioxazole, thiadiazole, pyridine, pyridazine, pyrimidine, pyrazine, triazine, oxazine, oxathiazine, oxadiazine, indole, benzimidazole, indazole, indoxazine, benzoxazole, benzisoxazole, benzothiazole, quinoline, isoquinoline, cinnoline, quinazoline, quinoxaline, naphthyridine, phthalazine, pteridine, xanthene, acridine, phenazine, phenothiazine, phenoxazine, benzofuropyridine, furodipyridine, benzothienopyridine, thienodipyridine, benzoselenophenopyridine, and selenophenodipyridine, preferably dibenzothiophene, dibenzofuran, dibenzoselenophene, carbazole, indolocarbazole, imidazole, pyridine, triazine, benzimidazole, 1,2-azaborine, 1,3-azaborine, 1,4-azaborine, borazine, and aza-analogs thereof. Additionally, the heteroaryl group is optionally substituted.

Of the aryl and heteroaryl groups listed above, the groups of triphenylene, naphthalene, anthracene, dibenzothiophene, dibenzofuran, dibenzoselenophene, carbazole, indolocarbazole, imidazole, pyridine, pyrazine, pyrimidine, triazine, and benzimidazole, and the respective aza-analogs of each thereof are of particular interest.

The terms alkyl, cycloalkyl, heteroalkyl, heterocycloalkyl, alkenyl, cycloalkenyl, heteroalkenyl, alkynyl, aralkyl, heterocyclic group, aryl, and heteroaryl, as used herein, are independently unsubstituted, or independently substituted, with one or more general substituents.

In many instances, the general substituents are selected from the group consisting of deuterium, halogen, alkyl, cycloalkyl, heteroalkyl, heterocycloalkyl, arylalkyl, alkoxy, aryloxy, amino, silyl, alkenyl, cycloalkenyl, heteroalkenyl, alkynyl, aryl, heteroaryl, acyl, carboxylic acid, ether, ester, nitrile, isonitrile, sulfanyl, sulfinyl, sulfonyl, phosphino, and combinations thereof.

In some instances, the preferred general substituents are selected from the group consisting of deuterium, fluorine, alkyl, cycloalkyl, heteroalkyl, alkoxy, aryloxy, amino, silyl, alkenyl, cycloalkenyl, heteroalkenyl, aryl, heteroaryl, nitrile, isonitrile, sulfanyl, and combinations thereof.

In some instances, the preferred general substituents are selected from the group consisting of deuterium, fluorine, alkyl, cycloalkyl, alkoxy, aryloxy, amino, silyl, aryl, heteroaryl, sulfanyl, and combinations thereof.

In yet other instances, the more preferred general substituents are selected from the group consisting of deuterium, fluorine, alkyl, cycloalkyl, aryl, heteroaryl, and combinations thereof.

The terms "substituted" and "substitution" refer to a substituent other than H that is bonded to the relevant position, e.g., a carbon or nitrogen. For example, when R' represents mono-substitution, then one R' must be other than H (i.e., a substitution). Similarly, when R' represents di-substitution, then two of $R^1$ must be other than H. Similarly, when $R^1$ represents no substitution, R', for example, can be a hydrogen for available valencies of ring atoms, as in carbon atoms for benzene and the nitrogen atom in pyrrole, or simply represents nothing for ring atoms with fully filled valencies, e.g., the nitrogen atom in pyridine. The maximum number of substitutions possible in a ring structure will depend on the total number of available valencies in the ring atoms.

As used herein, "combinations thereof' indicates that one or more members of the applicable list are combined to form a known or chemically stable arrangement that one of ordinary skill in the art can envision from the applicable list. For example, an alkyl and deuterium can be combined to form a partial or fully deuterated alkyl group; a halogen and alkyl can be combined to form a halogenated alkyl substituent; and a halogen, alkyl, and aryl can be combined to form a halogenated arylalkyl. In one instance, the term substitution includes a combination of two to four of the listed groups. In another instance, the term substitution includes a combination of two to three groups. In yet another instance, the term substitution includes a combination of two groups. Preferred combinations of substituent groups are those that contain up to fifty atoms that are not hydrogen or deuterium, or those which include up to forty atoms that are not hydrogen or deuterium, or those that include up to thirty atoms that are not hydrogen or deuterium. In many instances, a preferred combination of substituent groups will include up to twenty atoms that are not hydrogen or deuterium.

The "aza" designation in the fragments described herein, i.e. aza-dibenzofuran, aza-dibenzothiophene, etc. means that one or more of the C-H groups in the respective aromatic ring can be replaced by a nitrogen atom, for

example, and without any limitation, azatriphenylene encompasses both dibenzo[*f,h*]quinoxaline and dibenzo[*f,h*]quinoline. One of ordinary skill in the art can readily envision other nitrogen analogs of the aza-derivatives described above, and all such analogs are intended to be encompassed by the terms as set forth herein.

As used herein, "deuterium" refers to an isotope of hydrogen. Deuterated compounds can be readily prepared using methods known in the art. For example, U.S. Pat. No. 8,557,400, Patent Pub. No. WO 2006/095951, and U.S. Pat. Application Pub. No. US 2011/0037057, which are hereby incorporated by reference in their entireties, describe the making of deuterium-substituted organometallic complexes. Further reference is made to Ming Yan, et al., Tetrahedron 2015, 71, 1425-30 and Atzrodt et al., Angew. Chem. Int. Ed. (Reviews) 2007, 46, 7744-65, which are incorporated by reference in their entireties, describe the deuteration of the methylene hydrogens in benzyl amines and efficient pathways to replace aromatic ring hydrogens with deuterium, respectively.

It is to be understood that when a molecular fragment is described as being a substituent or otherwise attached to another moiety, its name may be written as if it were a fragment (e.g. phenyl, phenylene, naphthyl, dibenzofuryl) or as if it were the whole molecule (e.g. benzene, naphthalene, dibenzofuran). As used herein, these different ways of designating a substituent or attached fragment are considered to be equivalent.

In some instance, a pair of adjacent substituents can be optionally joined or fused into a ring. The preferred ring is a five, six, or seven-membered carbocyclic or heterocyclic ring, includes both instances where the portion of the ring formed by the pair of substituents is saturated and where the portion of the ring formed by the pair of substituents is unsaturated. As used herein, "adjacent" means that the two substituents involved can be on the same ring next to each other, or on two neighboring rings having the two closest available substitutable positions, such as 2, 2' positions in a biphenyl, or 1, 8 position in a naphthalene, as long as they can form a stable fused ring system.

Unless otherwise specified, any of the layers of the various embodiments may be deposited by any suitable method. For the organic layers, preferred methods include thermal evaporation, ink-jet, such as described in U.S. Pat. Nos. 6,013,982 and 6,087,196, which are incorporated by reference in their entireties, organic vapor phase deposition (OVPD), such as described in U.S. Pat. No. 6,337,102 to Forrest et al., which is incorporated by reference in its entirety, and deposition by organic vapor jet printing (OVJP), such as described in U.S. Pat. No. 7,431,968, which is incorporated by reference in its entirety. Other suitable deposition methods include spin coating and other solution based processes. Solution based processes are preferably carried out in nitrogen or an inert atmosphere. For the other layers, preferred methods include thermal evaporation. Preferred patterning methods include deposition through a mask, cold welding such as described in U.S. Pat. Nos. 6,294,398 and 6,468,819, which are incorporated by reference in their entireties, and patterning associated with some of the deposition methods such as ink-jet and organic vapor jet printing (OVJP). Other methods may also be used. The materials to be deposited may be modified to make them compatible with a particular deposition method. For example, substituents such as alkyl and aryl groups, branched or unbranched, and preferably containing at least 3 carbons, may be used in small molecules to enhance their ability to undergo solution processing. Substituents having 20 carbons or more may be used, and 3-20 carbons is a preferred range. Materials with asymmetric structures may have better solution processability than those having symmetric structures, because asymmetric materials may have a lower tendency to recrystallize. Dendrimer substituents may be used to enhance the ability of small molecules to undergo solution processing.

Devices fabricated in accordance with embodiments of the present invention may further optionally comprise a barrier layer. One purpose of the barrier layer is to protect the electrodes and organic layers from damaging exposure to harmful species in the environment including moisture, vapor and/or gases, etc. The barrier layer may be deposited over, under or next to a substrate, an electrode, or over any other parts of a device including an edge. The barrier layer may comprise a single layer, or multiple layers. The barrier layer may be formed by various known chemical vapor deposition techniques and may include compositions having a single phase as well as compositions having multiple phases. Any suitable material or combination of materials may be used for the barrier layer. The barrier layer may incorporate an inorganic or an organic compound or both. The preferred barrier layer comprises a mixture of a polymeric material and a non-polymeric material as described in U.S. Pat. No. 7,968,146, PCT Pat. Application Nos. PCT/US2007/023098 and PCT/US2009/042829, which are herein incorporated by reference in their entireties. To be considered a "mixture", the aforesaid polymeric and non-polymeric materials comprising the barrier layer should be deposited under the same reaction conditions and/or at the same time. The weight ratio of polymeric to non-polymeric material may be in the range of 95:5 to 5:95. The polymeric material and the non-polymeric material may be created from the same precursor material. In one example, the mixture of a polymeric material and a non-polymeric material consists essentially of polymeric silicon and inorganic silicon.

Devices fabricated in accordance with embodiments of the invention can be incorporated into a wide variety of electronic component modules (or units) that can be incorporated into a variety of electronic products or intermediate components.

The materials and structures described herein may have applications in devices other than organic solar cells. For example, other optoelectronic devices such as organic electroluminescent devices (OLEDs) and organic photodetectors may employ the materials and structures. More generally, organic devices, such as organic transistors, may

employ the materials and structures.

According to one aspect, the present disclosure relates to a compound of Formula I:

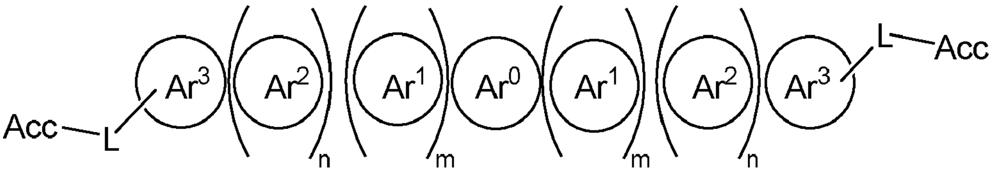

Formula I

wherein $Ar^0$ is an aromatic or non-aromatic ring connected to or fused at two positions to $Ar^1$, $Ar^2$, or $Ar^3$;
each $Ar^1$ is an aromatic ring or a non-aromatic ring that is connected to or fused to Ar° and to $Ar^2$, $Ar^3$, or another occurrence of $Ar^1$;
each $Ar^2$ is an aromatic ring or a non-aromatic ring that is connected to or fused to $Ar^1$ or Ar° and to $Ar^3$ or another occurrence of $Ar^2$;
$Ar^3$ is an aromatic ring that is connected to or fused to $Ar^2$, $Ar^1$, or $Ar^0$ and is connected to or fused to A;
m is from 0 to 10;
n is from 0 to 10;
Acc is an acceptor group connected or fused to $Ar^3$;
L is a linking group selected from the group consisting of a single bond and 1,2-cyclopentene;
Ar° represented by one of the following structures:

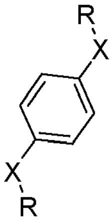 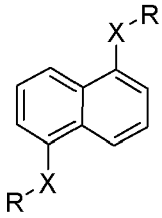 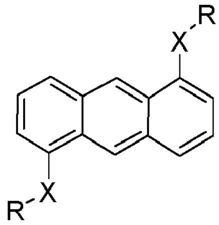 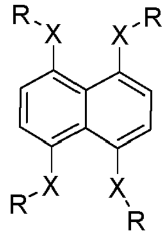 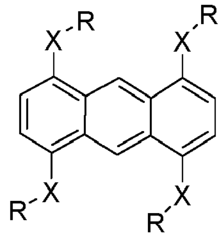

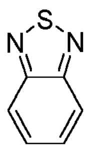 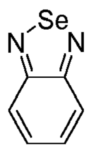 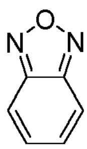 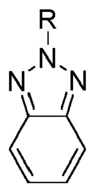 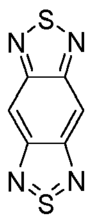 ;

each occurrence of $Ar^1$ is independently represented by one of the following structures:

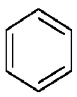 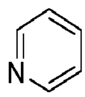 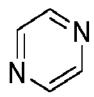 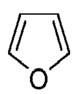 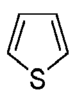 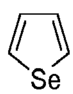 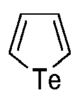

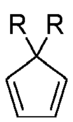 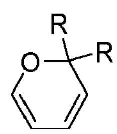 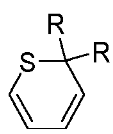 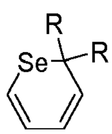 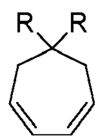

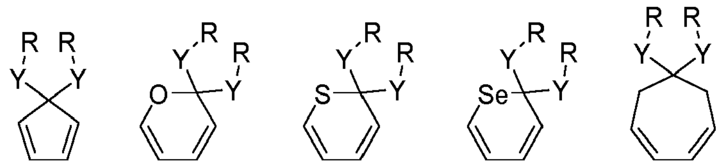

each occurrence of $Ar^2$ is independently represented by one of the following structures:

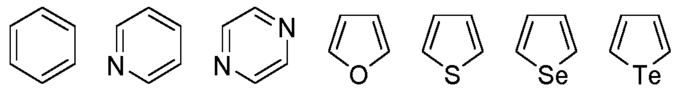

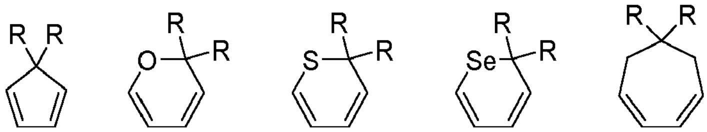

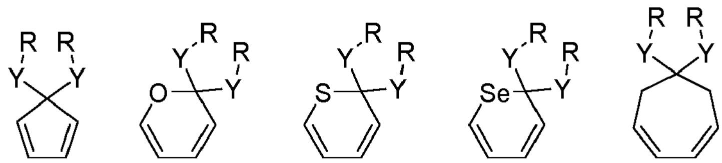

;

each occurrence of Y is represented by one of the following linking groups:

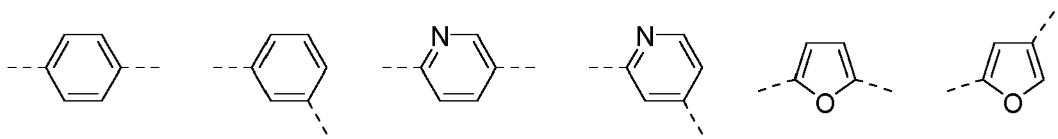

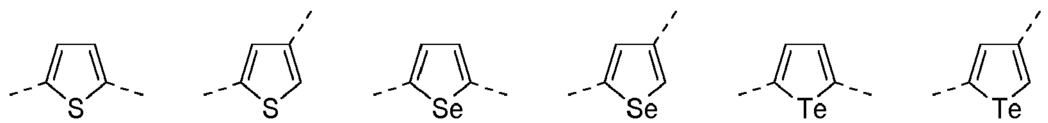

$Ar^3$ is represented by one of the following structures:

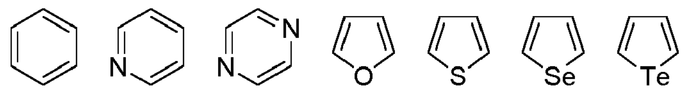

each occurrence of X is a single bond or a heteroatom selected from the group consisting of O, S, Se, and NR; Acc is connected to ring $Ar^3$ by a single bond and is represented by one of the following structures:

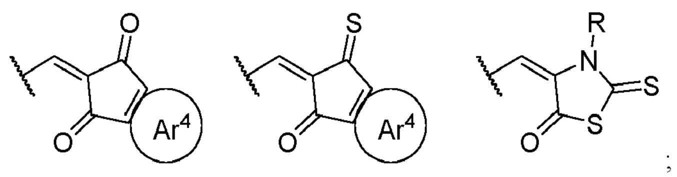

;

or

Acc is fused to ring $Ar^3$ and is represented by one of the following structures, wherein waved lines indicate connection to adjacent carbon atoms on $Ar^3$:

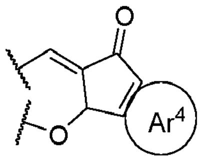 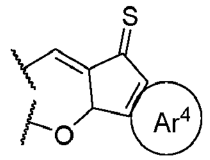 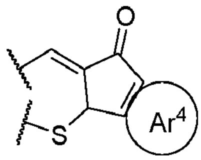 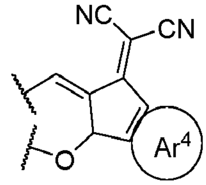

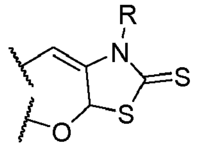 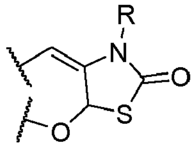 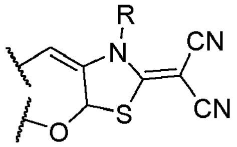

$Ar^4$ is represented by one of the following structures:

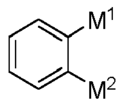 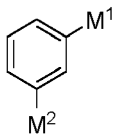 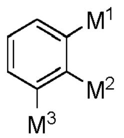 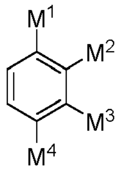 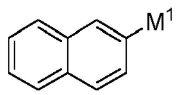 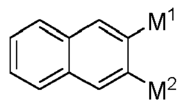 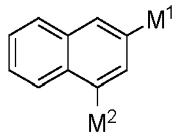

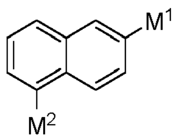 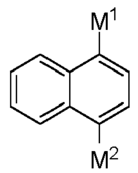 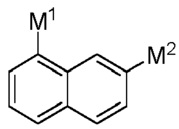 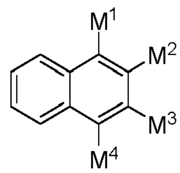

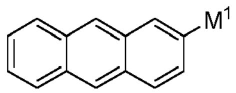 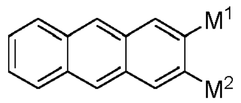 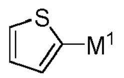 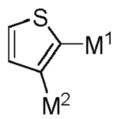 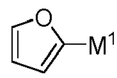 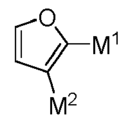

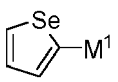 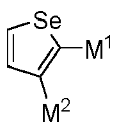 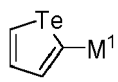 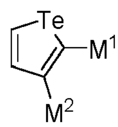 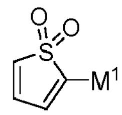 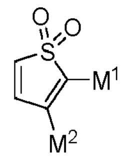

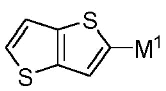 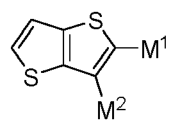 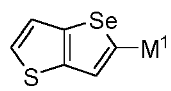 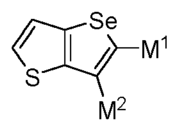 ;

$M^1$, $M^2$, $M^3$, and $M_4$ are individually selected from the group consisting of hydrogen, fluorine, chlorine, bromine, iodine, astatine, and a cyano group, wherein at least one of $M_1$-$M_4$ is a halogen
each R independently represents hydrogen or a substituent selected from the group consisting of deuterium, halogen, alkyl, cycloalkyl, heteroalkyl, heterocycloalkyl, arylalkyl, alkoxy, aryloxy, amino, thioalkyl, silyl, alkenyl, cycloalkenyl, heteroalkenyl, alkynyl, aryl, heteroaryl, acyl, carboxylic acid, ether, ester, nitrile, isonitrile, sulfanyl, sulfinyl, sulfonyl, phosphino, and combinations thereof; wherein any two adjacent substituents optionally join to form a ring.

In one embodiment, the compound is represented by Formula II, Formula III, Formula IV, or Formula V:

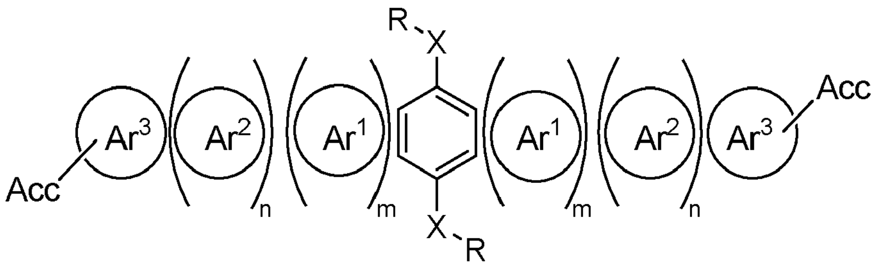

Formula II

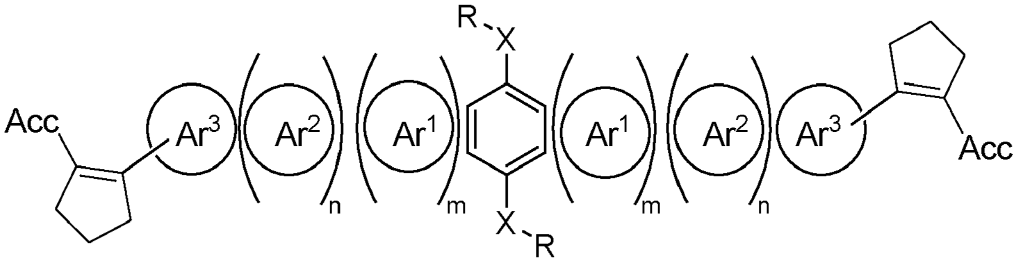

Formula III

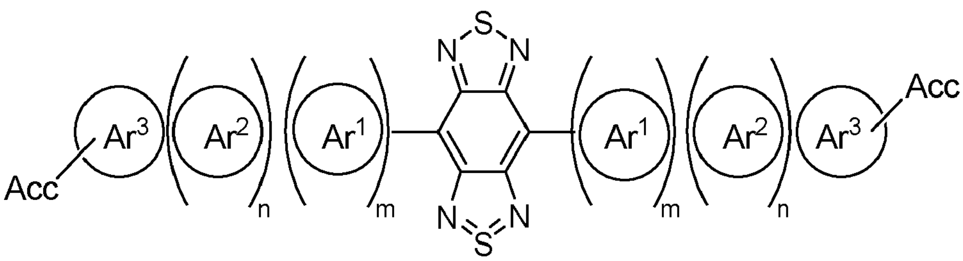

Formula IV

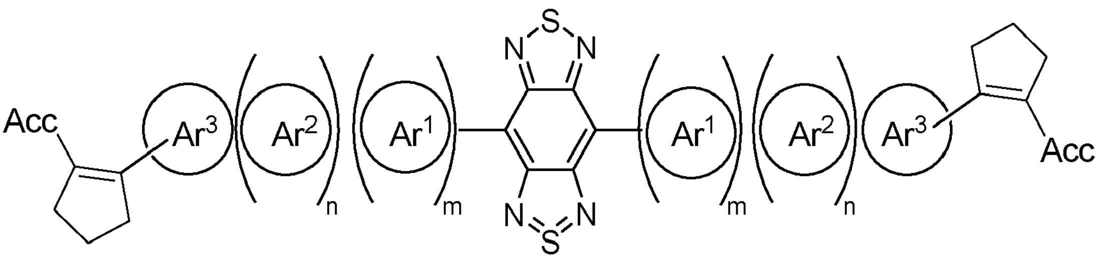

Formula V.

In one embodiment, the compound is represented by one of the following structures:

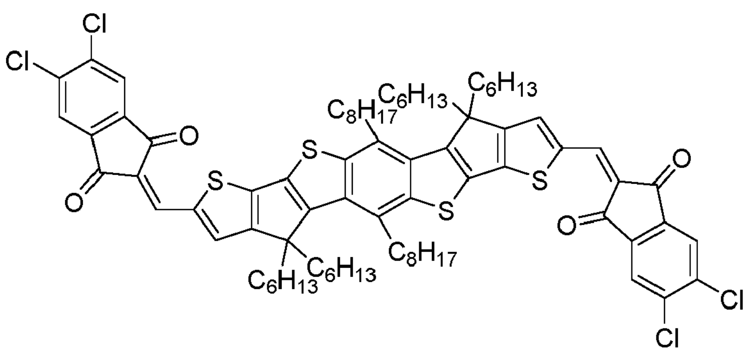

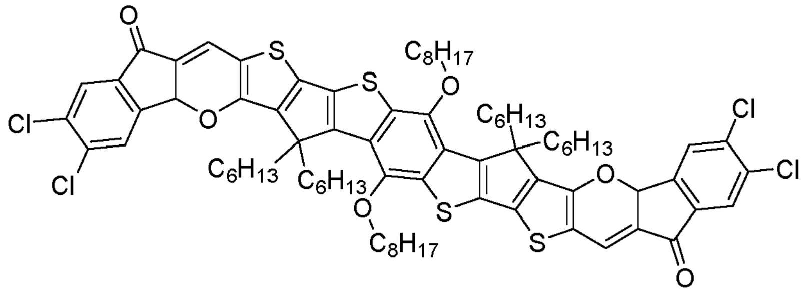

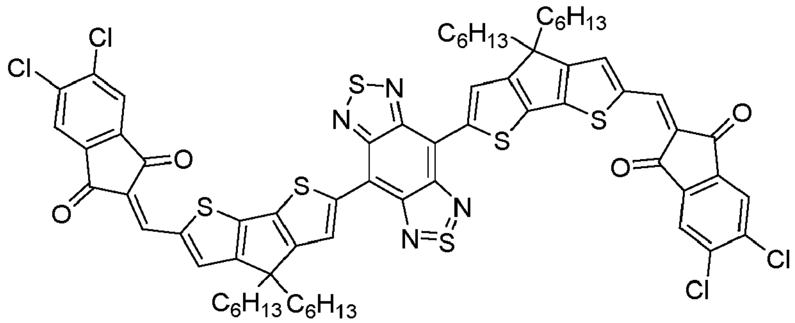

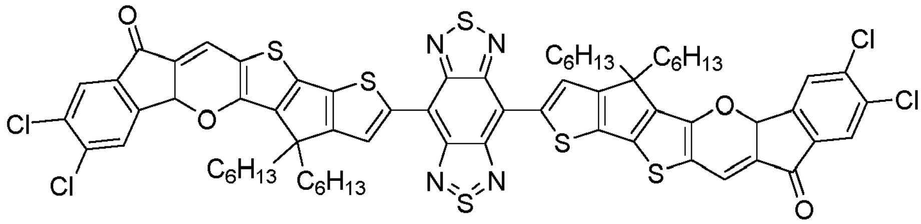

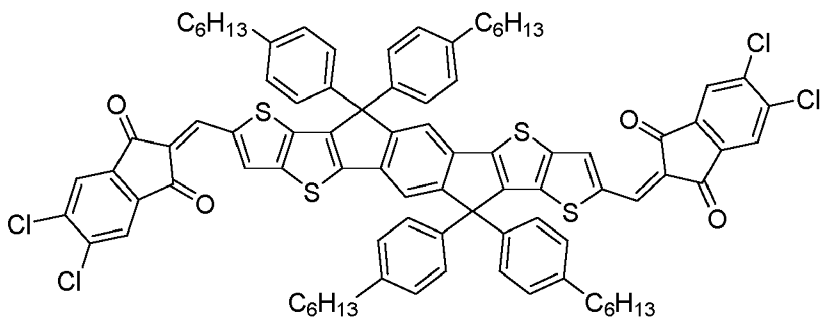

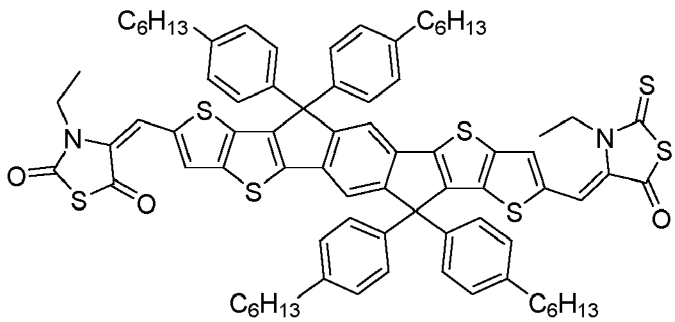.

According to another aspect, a formulation comprising a compound described herein is also disclosed.

Organic Photovoltaic Cells

In one aspect, the invention relates to an OPV device comprising a compound of the disclosure. In one embodiment, the OPV device includes an anode; a cathode; and an active material positioned between the anode and cathode, wherein the active material comprises an acceptor and a donor.

In one embodiment, the OPV device comprises a single-junction organic photovoltaic device. In one embodiment, the OPV device comprises two electrodes having an anode and a cathode in superposed relation, at least one donor composition, and at least one acceptor composition, wherein the donor-acceptor material or active layer is positioned between the two electrodes. In one embodiment, one or more intermediate layers may be positioned between the anode and the active layer. Additionally, or alternatively, one or more intermediate layers may be positioned between the active layer and cathode.

In one embodiment, the anode comprises a conducting oxide, thin metal layer, or conducting polymer. In one embodiment, the anode comprises a conductive metal oxide. Exemplary conductive metal oxides include, but are not limited to, indium tin oxide (ITO), tin oxide (TO), gallium indium tin oxide (GITO), zinc oxide (ZO), and zinc indium tin oxide (ZITO). In one embodiment, the anode comprises a metal layer. Exemplary metals for the metal layer include, but are not limited to, Ag, Au, Pd, Pt, Ti, V, Zn, Sn, Al, Co, Ni, Cu, Cr, and combinations thereof. In one embodiment, the metal layer comprises a thin metal layer. In one embodiment, the anode 102 comprises a conductive polymer. Exemplary conductive polymers include, but are not limited to, polyanaline (PANI), or 3,4-polyethyl-enedioxythiophene:polystyrenesulfonate (PEDOTPSS). In one embodiment, thickness of the anode is between about 0.1-100 nm. In one embodiment, thickness of the anode is between about 1-10 nm. In one embodiment, thickness of the anode is between about 0.1-10 nm. In one embodiment, thickness of the anode is between about 10-100 nm. In one embodiment, anode comprises a transparent or semi-transparent conductive material.

In one embodiment, the cathode comprises a conducting oxide, a metal layer, or conducting polymer. Exemplary conducting oxide, metal layers, and conducting polymers are described elsewhere herein. In one embodiment, the cathode comprises a thin metal layer. In one embodiment, the cathode comprises a metal or metal alloy. In one embodiment, the cathode may comprise Ca, Al, Mg, Ti, W, Ag, Au, or another appropriate metal, or an alloy thereof. In one embodiment, the thickness of the cathode is between about 0.1-100 nm. In one embodiment, the thickness of the cathode is between about 1-10 nm. In one embodiment, the thickness of the cathode is between about 0.1-10 nm. In one embodiment, the thickness of the cathode is between about 10-100 nm. In one embodiment, cathode comprises a transparent or semi-transparent conductive material.

In one embodiment, the OPV device may comprise one or more charge collecting/transporting intermediate layers positioned between an electrode and the active region or layer. In one embodiment, the OPV device comprises one or more intermediate layers. In one embodiment, the intermediate layer comprises a metal oxide. Exemplary metal oxides include, but are not limited to, MoOs, $MoO_x$, $V_2O_5$, ZnO, and $TiO_2$. In one embodiment, the first intermediate layer has the same composition as the second intermediate layer. In one embodiment, the first intermediate layer and the second intermediate layer have different compositions. In one embodiment, the thickness of the intermediate layers are each independently between about 0.1-100 nm. In one embodiment, the thickness of the intermediate layers are each independently between about 1-10 nm. In one embodiment, the thickness of the intermediate layers are each independently between about 0.1-10 nm. In one embodiment, the thickness of the intermediate layers are each independently between about 10-100 nm.

In one embodiment, the OPV device comprises various layers of a tandem or multi-junction photovoltaic device. In one embodiment, the OPV device comprises two electrodes having an anode and a 204 in superposed relation, at least one donor composition, and at least one acceptor composition positioned within a plurality of active layers or regions between the two electrodes. Additional active layers or regions are also possible. In one embodiment, the anode and the cathode each independently comprise a conducting oxide, thin metal layer, or conducting polymer. Exemplary conducting oxides, metal layers, and conducting polymers are described elsewhere herein.

In one embodiment, the OPV device comprises one or more intermediate layers positioned between the anode and a first active layer. Additionally, or alternatively, at least one intermediate layer may be positioned between the second active layer and cathode. In one embodiment, the OPV device comprises one or more intermediate layers positioned between the first active layer and the second active layer. In one embodiment, the OPV device comprises a first intermediate layer. In one embodiment, the OPV device comprises a second intermediate layer. In one embodiment, the OPV device comprises a third intermediate layer. In one embodiment, the OPV device comprises both first and second intermediate layers. In one embodiment, the OPV device comprises both first and third intermediate layers. In one embodiment, the OPV device comprises both second and third intermediate layers. In one embodiment, the OPV device comprises first, second, and third intermediate layers. In one embodiment, the first, second, and/or third intermediate layer comprises a metal oxide. Exemplary metal oxides are described elsewhere herein.

EXPERIMENTAL EXAMPLES

Small molecules are more reliable materials for organic photovoltaics (OPVs) compared to polymers, due to their reproducible synthesis and facile purification. Especially for device fabrication involving vapor deposition techniques, small molecules are obvious candidates compared to polymers.

Fabrication employing ternary blends can result in the generation of impurities that affect both the reproducibility of the performance and long-term reliability of the cells. In particular, the vinyl groups linking the D and A moieties can dissociate at room temperature by the exchange of the end-capping groups between two NFAs (Fig. 1) (Li, Y. et al. J. Am. Chem. Soc. 139, 17114-17119 (2017); Li, Y. et al. Energy Environ. Sci. 10, 1610-1620 (2017); Lin, Y. et al. Adv. Mater. 27, 1170-1174 (2015); Dai, S. et al. Adv. Mater. 30, 1706571 (2018); Yuan, J. et al. Nat. Commun. 10, 570 (2019); Liu, Q. et al. Joule 5, 2365-2379 (2021); Huang, X. et al. Appl. Phys. Lett. 116, 153501 (2020)) during the mixing of the blend used in forming the BHJ thin film. This exchange process results in reaction products with both asymmetric dipolar and symmetric non-polar molecular structures. As a consequence, ternary-blend OPVs based on two NFAs and a donor can unintentionally comprise up to seven chemical species. These impurities participate in photoinduced charge transfer, affecting cell performance and reproducibility of devices produced in different processing batches. Additionally, the asymmetric dipolar molecules introduce packing disorder and instabilities that make the blend vulnerable to photodegradation. As a consequence, the OPV PCE falls to less than 65% of its initial value within 265 h, and the time for a decrease in PCE to 80% from its initial value of $T_{80}$ = 40 h when exposed to illumination of 10 suns intensity, compared with $T_{80}$ > 265 h for devices where the impurities are absent. Importantly, the end-capping exchange reaction can be suppressed by appropriate design of the NFA molecules and the blending process, which is critical to enhancing the performance of ternary OPVs.

Results

These studies of end-capping exchange center on two archetype NFAs shown in Fig. 2, namely BT-IC and BT-CIC (BTIC-4Cl), which are among the best-performing materials systems for semitransparent organic solar cells (Li, Y. et al. Nat. Commun. 12, 1-9 (2021); Huang, X., et al., Joule, 6, 1581-1589 (2022); Li, Y, et al. Nat. Rev. Mater. 8, 186-201 (2023)). Both BT-IC and BTIC-4Cl have $C_2$ symmetry about their molecular backbones, and thus have small dipole moments (1.1 D). The OPV devices are typically prepared by spin coating mixtures of donor and acceptor materials to form the bulk heterojunction. Chlorobenzene (CB) is a common solvent for such solutions. Stirring a CB solution of BT-IC and BTIC-4Cl at 65 °C in ultrapure $N_2$ gas (with $O_2$ < 0.1 ppm and $H_2O$ <0.1 ppm) for 15 h leads to the unintentional formation of a new compound.

To verify the chemical composition of the blend, the product was isolated via column chromatography and characterized by nuclear magnetic resonance (NMR) spectroscopy (Fig. 3), matrix-assisted laser desorption ionization with time-of-flight mass spectrometry (MALDI-TOF-MS), and thin layer chromatography (TLC) (Fig. 4). The product of the reaction of BT-IC and BTIC-4Cl is BTIC-2Cl, Fig. 2, the result of exchanging end groups between the two reactants. BTIC-2Cl has a significantly larger dipole moment (4.8 D) than the two precursor materials due to chlorination of only a single end group. We find that this reaction occurs in a mixture containing only BT-IC and BTIC-4Cl, as well as in solutions where a donor polymer has been added to the mixture, suggesting that the end-capping exchange reaction is an intrinsic property of A-D-A type NFAs. Furthermore, a solution of the asymmetric BTIC-2Cl was stirred at 65 °C for 15 h, thereby producing BT-IC and BTIC-4Cl were produced (Fig. 4, left), suggesting that the exchange reaction is reversible. Note that the reaction occurs even at room temperature after stirring in CB for over 3h in the dark.

Since the $^1$H NMR spectrum of BTIC-2Cl resembles a superposition of the BT-IC and BTIC-4Cl spectra, it is impossible to distinguish the product from the blend of the reactants (Fig. 3). Therefore, fluorinated materials BTIC-4F and BTIC-2F were used to quantitatively characterize the reaction conditions via $^{19}$F{$^1$H} NMR spectroscopy. As shown in Fig. 5, BTIC-2F is identified by two distinct doublet fluorine (F) peaks (panel 2) that are distinguished from those of BTIC-4F found down field (panel 1).

The reaction kinetics of the end-capping exchange are also studied by $^{19}$F{H} NMR spectroscopy. The BT-IC is blended with BTIC-4F in a CB solution for 24h at temperatures ranging from 0 °C to 100 °C. As shown in Fig. 6, the conversion ratio (*r*) increases monotonically with temperature, from *r* = 0 at *T* = 0 °C, to 17% at *T* = 25 °C, increasing to 88% at *T* = 100 °C (see Methods). Thus, the end-capping exchange reaction is suppressed at low temperatures. Rather than the 65 °C CB solution mixture that generates BTIC-2F with *r* = 33% (panel 3), no BTIC-2F is detected in the BT-IC:BTIC-4F blend film prepared at low temperature after aging 72 h at 100 °C under nitrogen (see Fig. 5, panel 4), indicating that the exchange process is largely suppressed in the solid state.

The influence of illumination on the reaction is studied by exposing both the BT-IC:BTIC-4F blend film and solution to one sun intensity, simulated AM1.5 G illumination at 0 °C for 48h and 2.5 h, respectively. Surprisingly, in addition to the peaks of BTIC-2F, two new doublets emerge in the $^{19}$F{$^1$H} NMR spectra from the exposed BT-IC:BTIC-4F solution (Fig. 5, panel 5), indicating that a chemical reaction process other than end-group exchange has occurred.

It has been reported that this new reaction may be an intramolecular electrocyclic reaction between the end-group and the thienyl backbone (Che, Y, et al. Angew. Chemie Int. Ed. 60, 24833-24837 (2021)). In contrast, no new chemical species are detected after illuminating the blend film (panel 6). This conclusion is further supported by the evolution of the absorption spectra of a BT-IC:BTIC-4F solution over time (see Fig. 7). Light soaking for 2.5 h significantly bleaches the BT-IC:BTIC-4F mixture whose absorption peak is centered at a wavelength of 750 nm. Again, the spectral shape and intensity of the mixture show no changes after 40 h in the dark at 65 °C, although its composition changes due to the end-capping exchange.

A number of representative NFA molecules are selected to investigate the influence of molecular structure on the end-capping exchange reaction (Fig. 1). The reaction can occur in the most efficient A-D-A NFA families reported thus far, independent of the composition of the central fused aromatic ring units, and that of the end-capping moieties. For example, a mixture of IT-IC and BTIC-4F generates the four expected constituents (ITIC-2F, ITIC-4F, BTIC-2F and BT-IC) (Li, M., et al. J. Mater. Chem. A7, 8889-8896 (2019); Zhao, W. et al. J. Am. Chem. Soc. 139, 7148-7151 (2017)) upon end-capping exchange after stirring at 65 °C for 24 h under ultrapure $N_2$ (Fig. 8). Analogously, the same results are obtained for Y-series and pyrene-series NFAs (see Fig. 1). Detailed characterization data are provided in Figs. 9-17.

To systematically study the effects of impurities produced by the end-capping exchange reaction, ternary OPVs were fabricated with the device structure: indium tin oxide (ITO)/ ZnO (30 nm) / PCE-10:BT-IC:BTIC-4Cl (1:0.75:0.75, w/w/w, 80 nm) / $MoO_x$ (10 nm) / Ag (100 nm) as shown in Fig. 18. The constituents in one group of OPVs ("hot") were blended at $T$ = 65 °C for different reaction times, while a second, identical group of cells ("cold") were prepared at $T$ = 25 °C for 0.5 h to impede the reaction. Note that the hot solution is cooled to room temperature before fabricating the OPVs. The evolution of the solar cell performance parameters vs. blend solution reaction time for both cold and hot devices are plotted in Fig. 19 and Fig. 20. Here, the cold device corresponds to a reaction time of $t$ = 0 h. As expected, impurities in the BHJ increase with reaction time (see Fig. 21), where the presence of BTIC-2Cl is clearly detected in the active layer mixture after 24 h. Contrary to expectations, the open circuit voltage ($V_{OC}$) of hot ternary OPVs is pinned to the same value even though unintended reaction products are present in the BHJ layer. However, there is a decrease in *PCE* and fill factor (*FF*) for the hot ternary OPV devices. Compared to the cold device, the hot OPV shows a decreased fill factor (*FF*, 0.645 $\pm$ 0.004 vs. 0.673 $\pm$ 0.005) and *PCE* (9.65 $\pm$ 0.30 % vs. 10.21 $\pm$ 0.30 %) after blending for 24 h.

Ternary blend OPVs based on PCE-10:IT-IC:BTIC-4F (1:1:1, w/w/w) and PCE-10:IT-IC:Y16-4F (1:1:1, w/w/w) were also evaluated versus reaction time to test the generality of the effect on their performance, with results shown in Figs. 22 and 23. In contrast to the BT-IC:BTIC-4Cl mixture which produced only a single impurity molecule, both the IT-IC:BTIC-4F and IT-IC:Y16-4F mixtures generate multiple reaction products. As a consequence, a significant decrease in both *PCE* (10.13 $\pm$ 0.25 % vs. 8.46 $\pm$ 0.16 % for PCE-10:IT-IC:BTIC-4F; and 10.32 $\pm$ 0.41 % vs. 9.38 $\pm$ 0.59% for PCE-10:IT-IC:Y16-4F) and *FF* (0.665 $\pm$ 0.006 vs. 0.544 $\pm$ 0.009; and 0.621 $\pm$ 0.004 vs. 0.605 $\pm$ 0.008) are observed, while the short circuit current density ($J_{SC}$) and $V_{OC}$ remain unchanged.

The influence of impurities on the reliability of ternary blend OPVs was studied by exposing both the hot and cold devices to white light illumination at an intensity as high as 10 suns (see Methods). To prevent chemical changes known to occur at the organic/inorganic interfaces over time, $C_{70}$ and IC-SAM buffer layers are inserted between the BHJ and charge transporting layers (see Fig. 24) (Ding, K., et al. ACS Appl. Mater. \& Interfaces 14, 5692-5698 (2022)). Compared to the cold ternary device where PCE is reduced by 15% from its initial value after 265 h, that of the hot ternary device falls to less than 65% over the same period due to a rapid decrease in $J_{SC}$ and *FF* (see Fig. 25 and Fig. 26). This is consistent with previous results where the presence of impurities in OPVs were implicated in exciton quenching, leading to correspondingly shorter device operational lifetimes.

## Discussion

While the chemical stability of the exocyclic vinyl double bonds in A-D-A type NFAs has been extensively studied (Jiang, Y. et al. Mater. Horiz. 6, 1438-1443 (2019); Liu, Z.-X. et al. Nat. Commun. 12, 3049 (2021)), little is known about thermally induced olefin exchange reactions. For example, Aldrich, et. al reported a previously unrecognized end-capping redistribution process of IT-IC based NFAs (Aldrich, T. J. et al. J. Am. Chem. Soc. 141, 3274 (2019)). This unexpected finding highlights the chemical instability of IT-IC-based NFAs and has deep implications on the chemistry of IT-IC molecules. The authors propose an "unanticipated" [2+2] cycloaddition/cyclization reversal mechanism, which provides valuable insight into the chemical behavior of these materials. However, the inclusion of a 1,8-diiodooctane additive and polymer donor in their system can obscure the interpretation of the mechanisms and their generality. The end group rearrangement in other systems and how to shift this equilibrium is still unclear. In our work, the C=C/C=C exchange process is clarified by synthesis and study of four model D-A compounds (PhIC, $NMe_2$PhBarb, $NMe_2$PhIC and PhBarb) with different end-capping groups (Fig. 27, compounds 1-4, respectively). The molecules are distinguished by their different $^1$HNMR spectra (Fig. 28, panels 1-4), allowing for easy identification of end capping exchange products. Refluxing PhIC and $NMe_2$PhBarb together in CB overnight reveals a ratio between the starting materials and products of 3:2 (Fig. 28, panel 5). Importantly, peaks at 10.02 and 9.73 ppm correspond to the aldehyde proton peaks of 4-dimethylamino-benzaldehyde (Fig. 29, panel 1) and benzaldehyde (Fig. 29, panel 2), respectively, suggesting the exchange reaction may proceed the dissociative mechanism involving hydrolysis.

To verify that the C=C/C=C exchange reaction is a water-assisted retro-Knoevenagel condensation reaction, the reaction of PhIC and $NMe_2$PhBarb was repeated with anhydrous CB that was further dried over 3 Å molecular sieves. Contrary to the experiment conducted in as-obtained CB, no color change or observable peaks of products are found in the $^1$H NMR spectra (Fig. 28, panel 6). However, introducing 1 M equivalent water into the mixture and refluxing for the same duration results in NMR peaks corresponding to trace amounts of products, as well as those of benzaldehyde (Fig. 30). This indicates that the C=C/C=C exchange process undergoes a $H_2O$-catalyzed dissociative retro-Knoevenagel condensation reaction to generate the aldehyde and free methylene compounds, which then reform either the original benzylidenes, or exchange to form new products. This conclusion is also supported by the reaction between two archetype NFAs BTIC and BTIC-4F (see Fig. 31).

Reactions of $NMe_2$PhIC with both benzaldehyde (BD, intermediate) and 1,3-dimethylbarbituric acid (DMB, intermediate) were studied in anhydrous CB to determine whether either free donors or acceptors can directly exchange with a D-A molecule (Fig. 32 and Fig. 33). Interestingly, reacting $NMe_2$PhIC with DMB results in the formation of $NMe_2$Barb and free IC. However, BD failed to exchange with the $NMe_2$PhIC.

Given these results, proposed mechanisms for end-capping exchange are shown in Fig. 34. The reaction begins with a Michael addition reaction by a nucleophilic attack of the water oxygen on the highly polarized exocyclic C=C double bond (Zhu, X. et al. ACS Appl. Energy Mater. 2, 7602-7608 (2019); Xu, S. et al. Angew. Chemie Int. Ed. 61, e202202492 (2022)), followed by the departure of the aldehyde and formation of an activated methylene intermediate. The activated methylene adds to the double bond of PhIC (1) to form a geminal intermediate, which can then eliminate IC to form the end-capping exchange product PhBarb (4). IC is then added to $NMe_2$PhBarb (2), followed by elimination of DMB to generate $NMe_2$PhIC (3). Alternatively, condensation of the released activated methylene or barbiturate with the foreign aldehyde generates the end-capping exchange products. In either case, the electrophilic behavior of the β-carbon in the polarized C=C double bond plays a crucial role, as the nucleophilic attack of oxygen on the C=C double bond is the first step of the mechanism (Zhu, X. et al. ACS Appl. Energy Mater. 2, 7602-7608 (2019)).

Although the capping exchange reaction can be suppressed by using ultra-high-quality anhydrous solvents or controlling the temperature of the mixed solution, this might incur significant costs in the large-scale industrial production of such purified materials. Therefore, new material design rules that can reduce the reactivity between NFAs are preferred. Since the reactivity of the exchange is correlated with the polarizability of C=C double bonds, the effects of end-capping on the reactivity of four archetype NFAs have been examined (IT-IC, IT-CIC, IT-COC and IT-R whose molecular structures are shown in Fig. 35) (Zhang, H. et al. Adv. Mater. 30, 1800613 (2018); Cui, Y et al. Nat. Energy 4, 768-775 (2019)). The Mulliken charge of the β-carbon is determined using density functional theory to evaluate the tendency to gain electrons (Mulliken, R. S. J. Chem. Phys. 23, 1833-1840 (1955)). The qualitative order of Mulliken charge was found to be IT-IC > IT-CIC > IT-COC > IT-R, which is consistent with their relative reactivities. The reaction of BTIC-4F with IT-IC gives the highest conversion ratio of $r$ = 141 ±6%, where $r$ is the integral of the $^{19}F\{^1H\}$NMR spectral peak of the product relative to that of the starting material. On the other hand, no observable products are found in a mixture of BTIC-4F and IT-R due to its low probability of initiating a nucleophilic attack reaction. This indicates that the end-capping exchange between NFAs can be suppressed by reducing the Mulliken charge of the β-carbon in the exocyclic vinyl double bond.

Recent studies have shown that OPVs comprising a physical mixture of two A-D-A type NFAs in ternary OPVs exhibit electronic and physical properties different from its components (e.g. melting point, morphology etc.) (Xiao, L. et al. Adv. Funct. Mater. 31, 2105304 (2021); Cai, Y. et al. Adv. Mater. 33, 2101733 (2021)). The formation of "molecular alloys" or "organic co-crystals" caused by electrostatic van der Waals forces have been attributed to these changes. In contrast to earlier reports, this study demonstrates that there is a thermally induced, solution-phase end-capping exchange reaction occurring between NFAs during materials preparation and device fabrication. This reaction contaminates the source materials, thereby altering the electronic and physical properties of the resulting films.

To understand the effects of impurities on hot ternary OPVs, the molecular packing and crystallization of the blend film was characterized using grazing incidence wide angle X-ray scattering (*GIWAXS*). The two-dimensional (2D) diffraction patterns, and 1D line cuts of several films are provided in Figs. 36-38. The neat films of three individual acceptors show significantly different diffraction patterns due to the influence of the chlorination on molecular packing and crystallization. BT-IC (Fig. 36, panel 1) exhibits a rich diffraction pattern, indicating long range crystalline order. In contrast, the neat, dipolar BTIC-2Cl film (panel 2) shows considerably less well-defined diffraction features than BT-IC, with BTIC-4Cl (panel 3), exhibiting intermediate ordering. BTIC-2Cl and BTIC-4Cl both exhibit a pronounced and broad out-of-plane scattering feature at high $q$ indicative of out-of-plane π-π stacking. On the other hand, the GIWAXS patterns of the hot and cold blend films of BT-IC:BTIC-4Cl (1:1, w/w) are remarkably different. In the absence of chemical reactions, the packing structure of the cold blend film (panel 4) is dominated by BT-IC, as evident by the similar (h00) in-plane diffraction peaks of BT-IC at 0.69, 0.97, 1.37 and 1.68 $Å^{-1}$ (see Fig. 37). In contrast, the hot blend film (panel 5) shows several diffraction features found only in BTIC-2Cl (white arrows) ascribed to the BTIC-2Cl generated in the reaction.

This is further supported by the azimuthal distribution analysis of the diffraction feature at $q$ = 0.54 Å$^{-1}$ (Fig. 38), where the two intense peaks appearing at radial angles $\chi = \pm 26°$ are only observed in the neat BTIC-2Cl and the hot blend films. Also, BTIC-2Cl appears to dominate the crystalline phase in the blends. The introduction of an equivalent amount of BTIC-2Cl into a cold mixture of BT-IC:BTIC-4Cl (1:1, w/w) (panel 6) shows the same pattern as neat BTIC-2Cl, indicating that this asymmetric molecule is source of disorder (also see Fig. 41). Figs. 42 and 43 show the morphological stability of cold ternary blend films of PCE-10:BT-IC:BTIC-4Cl (1:0.75:0.75, w/w/w) after aging for 48 h under 10 sun intensity. White light illumination shows no significant changes in their GIWAXS patterns of the cold film. In contrast, significant degradation is observed for the hot film due to the emergence of dipolar reaction products, resulting in the disappearance of the (010) peak at 1.8 Å$^{-1}$.

Since the frontier molecular orbital of BTIC-2Cl differs from both BT-IC and BTIC-4Cl, its presence is expected to influence the charge transfer (CT) properties of the blends, and thus the charge photogeneration properties of OPVs in which these compounds are employed. To investigate CT state energetics in both cold and hot blends, their electroluminescence (EL) spectra due to CT$\rightarrow$S$_0$ transitions of PCE-10:BT-IC:BTIC-4Cl (1:0.75:0.75 by wt.)-based ternary OPVs are shown in Figs. 44 and 45, respectively. Parameters used to fit the individual binary CT state emission peaks (solid lines) are provided in the Supplementary Figs. 46 and Table 1. The ternary EL spectra are fit with linear combinations of the binary CT spectra by maintaining their same peak positions and full width half maxima (FWHM), while adjusting only their relative intensities. The EL spectrum of the cold ternary OPV is fit with two Gaussian peaks: one due to the PCE-10:BT-IC binary CT state at 1.36 $\pm$ 0.02 eV, and the other due to the PCE-10:BTIC-4Cl binary CT state at 1.15 $\pm$ 0.01 eV. On the other hand, three Gaussian peaks are required to fit the hot ternary OPVEL spectrum comprising both the PCE-10:BT-IC and PCE-10:BTIC-4Cl binary CT peaks, as well as an additional PCE-10:BTIC-2Cl binary CT peak at 1.23 $\pm$ 0.01 eV produced by the end capping reaction, suggesting the reaction product is active in photogeneration in the hot ternary OPV The change in CT properties of hot ternary OPVs is attributed to the linear superposition of the constituent binary molecules, rather than the emergence of new electronic states due to the generation of deep levels or the formation of molecular alloys (Huang, X., et al. Mater. Horiz. 7, 244-251 (2020)).

**Table 1.** Charge transfer state emission peaks for binary and ternary OPVs obtained from spectral fitting.

| Binary CT state | CT peak position (eV) | CT peak FWHM (eV) | Intensity fraction in cold ternary | Intensity fraction in hot ternary |
|---|---|---|---|---|
| PCE-10:BT-IC | 1.36 $\pm$ 0.02 | 0.17 $\pm$ 0.01 | 16.7% | 6.3% |
| PCE-10:BTIC-4Cl | 1.15 $\pm$ 0.01 | 0.28 $\pm$ 0.02 | 83.3% | 78.4% |
| PCE-10:BTIC-2Cl | 1.22 $\pm$ 0.01 | 0.23 $\pm$ 0.01 | - | 15.4% |

To track changes in the non-radiative recombination in the OPVs, the EL spectra are periodically collected for both cold and hot devices aged under white light illumination at 10 suns intensity. As shown in Fig. 47, the EL intensity of the hot ternary OPV degrades by 25%, which is faster than for cold devices (16%). Additionally, the peak intensity decays rapidly within the first 96 h, qualitatively matching the initial losses observed in $V_{OC}$ and $FF$ (Fig. 25 and Fig. 26). The observed degradation in CT state EL emission can be quantitatively linked to changes in $V_{OC}$ via:

$$\Delta V_{OC} = V_{OC}(t) - V_{OC}(0) = \frac{mk_BT}{q}\left[\ln\left(\frac{\eta_{EL}(t)}{\eta_{EL}(0)}\right)\right] \quad (1)$$

where $k_B$ is Boltzmann's constant, $T$ is the temperature, $q$ is the electron charge and $\eta_{EL}$ is the EL external quantum efficiency. The factor $m$ reveals the region where the degradation occurs in the device. The lower bound of $m$ = 1 corresponds to degradation in the BHJ itself, and $m$ > 1 indicates losses outside the active layer (Ameson, C. et al. Appl. Phys. Lett. 118, 63301 (2021)). As shown in Fig. 48, by fitting (dashed lines) Eq. (1) to the changes in $\eta_{EL}$ and $\Delta V_{OC}$ over time (data points), obtain $m$ = 1.00 $\pm$ 0.05 is obtained for both cold and hot devices. This suggests that the increased nonradiative recombination loss in hot ternary devices occurs solely in the BHJ. This loss, originating from the reaction products generated in the hot ternary BHJ, leads to a rapid decrease in $V_{OC}$.

The asymmetric dipolar products of the end-capping exchange reaction have large polarizabilities; i.e. $\alpha = 1.25 \times 10^{-21}$ cm$^3$ for BTIC-2Cl > $\alpha = 4.56 \times 10^{-22}$ cm$^3$ for BT-IC > $\alpha = 2.90 \times 10^{-22}$ cm$^3$ for BTIC-4Cl (see Methods). This may result in dipole reorientation over time that decreases the internal electric field across the BHJ, and hence reduces

the charge extraction efficiency. The voltage-dependent photocurrent before and after 265 h aging under an illumination intensity of 10 suns normalized to the photocurrent at $V = 0$, are shown in Fig. 49. From these measurements, the charge extraction efficiency at $V = 0$ was calculated over the aging period (see Fig. 50). The hot ternary device experienced a 16 ± 2% decrease in charge-extraction efficiency, whereas only a 2.0 ± 10% decrease is observed in the cold ternary device. Therefore, the decrease in $J_{SC}$ and *FF* of the hot ternary device is due to its decreased charge extraction efficiency (Bartesaghi, D. et al. Nat. Commun. 6, 7083 (2015); Dibb, G. F. A., et al. J. Phys. Chem. Lett. 4, 803-808 (2013)).

While the dipolar reaction products have a significant impact on the reliability of ternary OPVs, their presence can also influence device reproducibility, possibly explaining the poor reproducibility of organic ternary OPVs despite using the same materials prepared under similar conditions (Huang, J. et al. Energy Environ. Sci. 14, 3010-3018 (2021); He, L., et al. IEEE J. Photovoltaics 9, 1290-1296 (2019)). Additionally, in contrast to earlier reports, almost all NFAs used in high efficiency OPV cells undergo $H_2O$-catalyzed dissociative reactions during blending that create a plethora of reaction intermediates, such as dissociated end-capping units. This result is consistent with an observation that a redistribution of end-capping units in the BHJ toward the BHJ/$MoO_x$ interface occurs during thermal aging. Therefore, in addition to the ternary blend devices discussed here, this result can also significantly impact the performance, reliability, and reproducibility of many reported binary blend OPVs based on similar A-D-A type NFAs.

In summary, ternary blend OPVs consisting of two A-D-A type NFAs and a donor are susceptible to end-capping exchange reactions between NFAs during material blending prior to film deposition, even at room temperature in the dark. The reactions are considerably accelerated at elevated temperatures customarily used in material preparation. The reaction is shown to undergo $H_2O$-catalyzed dissociative retro-Knoevenagel condensation. Additionally, the reaction products feature significant ground state dipole moments that are linked to a decrease in the crystallization of the BHJ nanostructure, and that increase non-radiative charge recombination when employed in OPVs, thereby reducing both the device reproducibility and long-term stability. This end-capping exchange between NFAs can be reduced by decreasing the reactivity of the β-carbon in the exocyclic vinyl double bond. These results provide new insights for understanding and improving the performance of a large and general class of materials when used in ternary blend OPVs.

Methods

**Materials.** Details of molecular structures and nomenclatures of NFAs studied in this work can be found in Table 2. The cathode buffer material 4-((1,3-dioxo-1,3-dihydro-2H-inden-2-ylidene)methyl)benzoic acid, IC-SAM, and NFA molecules, BT-IC, BTIC-4Cl (BT-ClC), IT-IC, ITIC-4F, ITIC-DM, BETIC-4F, 6TIC-4F, IDTIDT-IC and N3 are synthesized as previously. Other materials are purchased from commercial suppliers: PCE-10 (1-Materials), Y16 (1-Materials), Y16-4F (1-Materials), Y1 (1-Materials), IO-4Cl (Brilliant Materials), ITIC-4Cl (Brilliant Materials), MoOs (Acros Organics), Zinc acetate dihydrate (Sigma Aldrich), Ag (Alfa Aesar) and Al (Alfa Aesar). Chlorobenzene (HPLC grade, 99.9%) is purchased from Sigma Aldrich.

Table 2: Chemical structures and common names of NFA molecules.

| Chemical Structures | References |
|---|---|
| 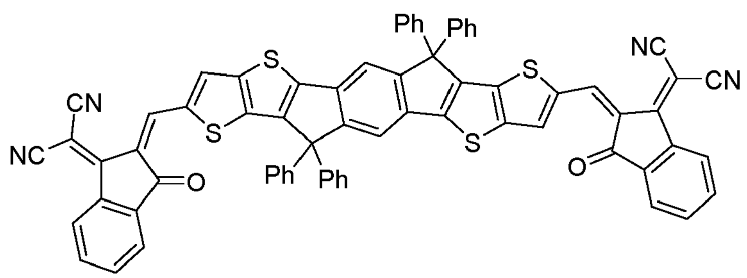 IT-IC | Lin, Y. et al., Adv. Mater. 2015, 27, 1170. |

(continued)

| Chemical Structures | References |
|---|---|
| 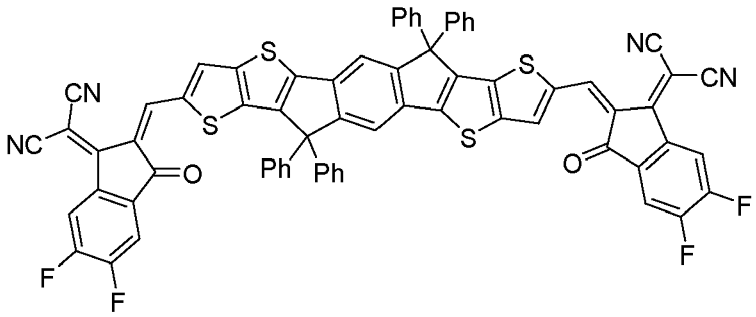 ITIC-4F | Zhao, W. et al., J. Am. Chem. Soc., 2017, 139, 7148-7151. |
| 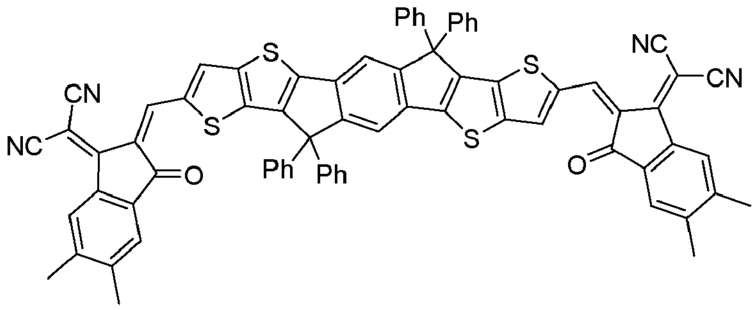 ITIC-DM | Li, S. et al., Adv. Mater. 2016, 28, 9423. |
| 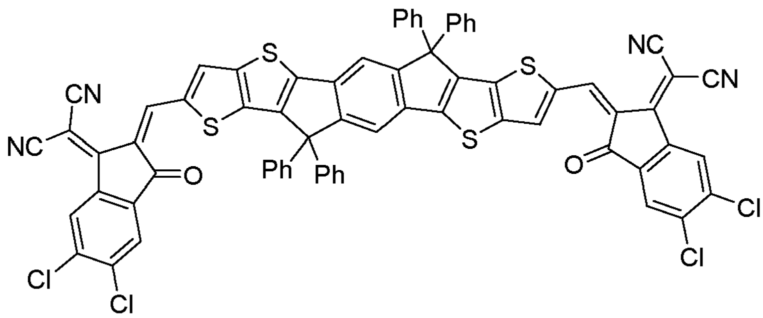 IT-CIC | Zhang, H. et al., Adv. Mater. 2018, 30, 1800613. |
| 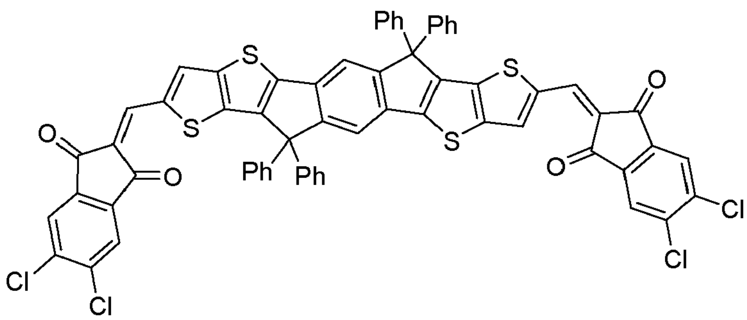 IT-COC | Cui, Y. et al., Nat. Energy 2019, 4, 768. |

(continued)

| Chemical Structures | References |
|---|---|
| 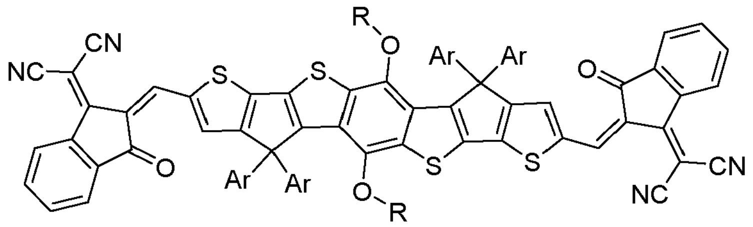 BT-IC | Li, Y. et al., Energy Environ. Sci. 2017, 10 (7), 1610-1620. |
| 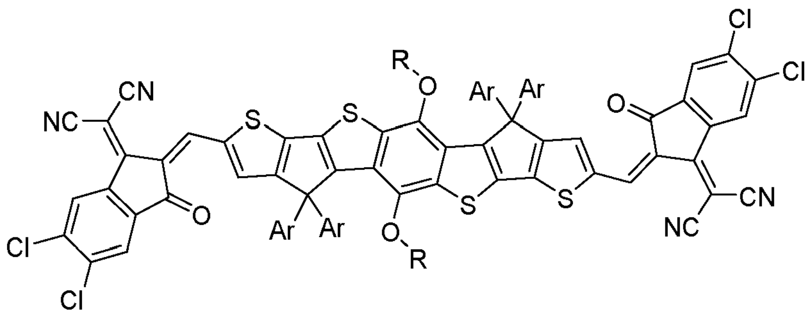 BT-CIC (BTIC-4Cl) | Li, Y. et al., J. Am. Chem. Soc., 2017, 139,17114. |
| 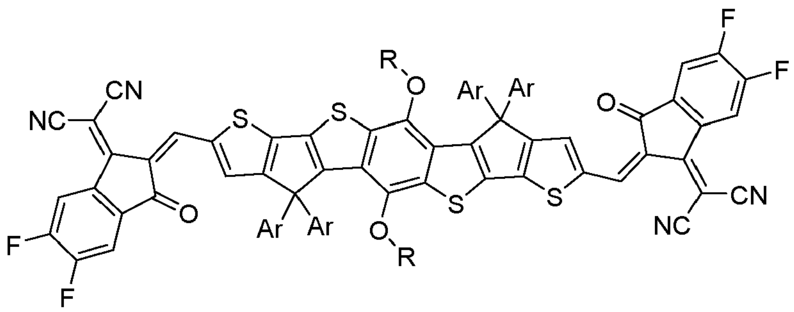 BTIC-4F | - |
| 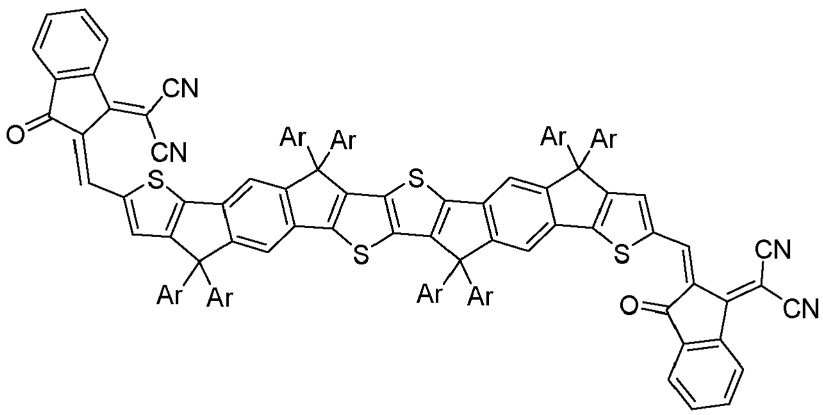 IDTIDT-IC | Li, Y. et al., J. Mater. Chem. A, 2016, 4, 5890 . |

(continued)

| Chemical Structures | References |
|---|---|
| 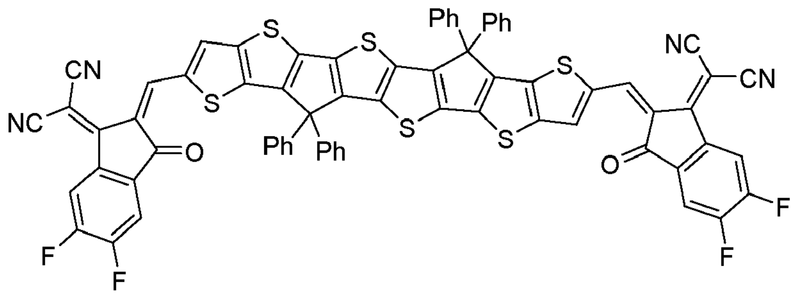 6TIC-4F | Shi, X. et al., Adv. Funct. Mater. 2018, 28, 1802324. |
| 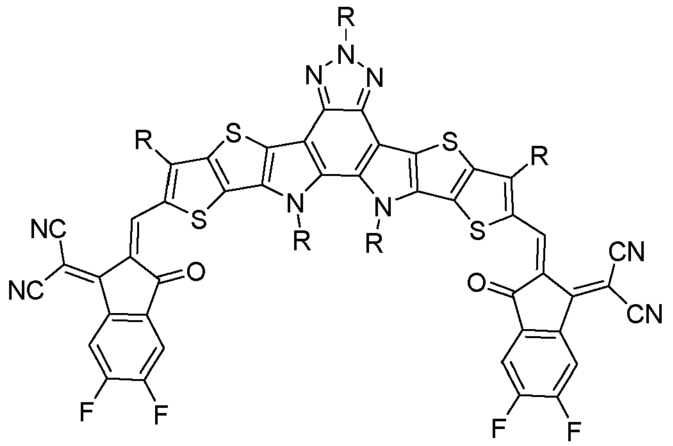 Y16-4F | Liu, S. et al., Nat. Photonics 2020, 14, 300 . |
| 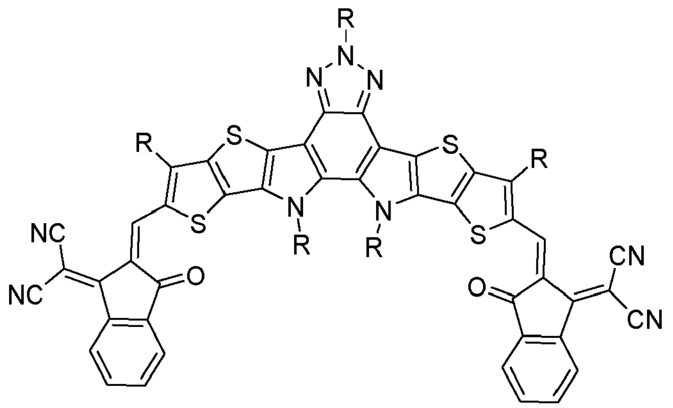 Y16 | Luo, M. et al., J. Energy Chem. 2020,42, 169. |

(continued)

| Chemical Structures | References |
|---|---|
| 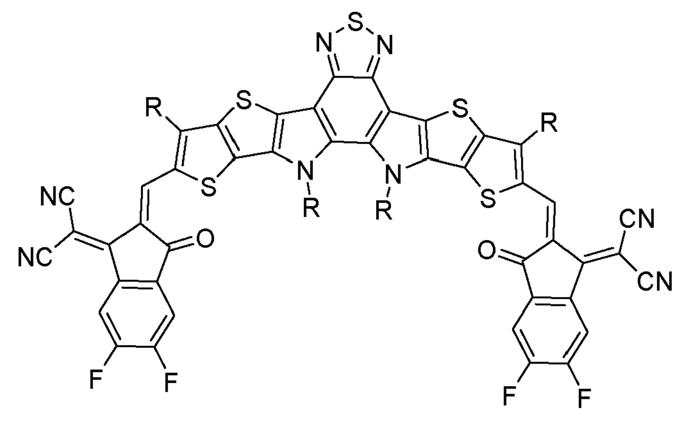 N3 | Jiang, K. et al., Joule 2019, 3, 3020. |
| 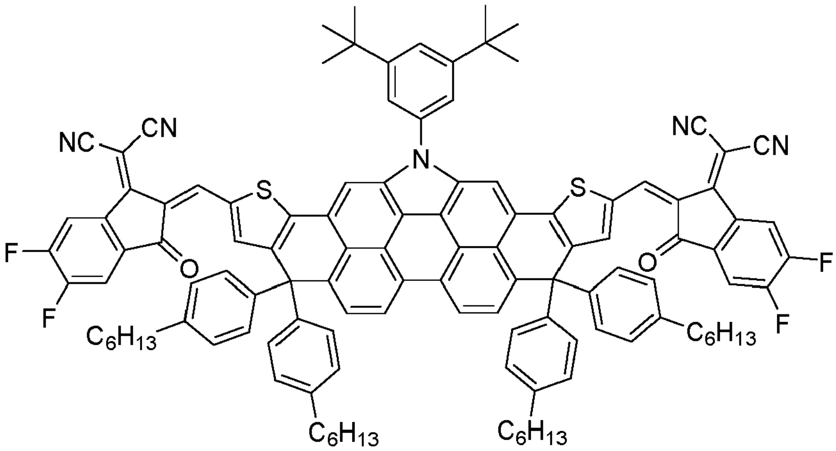 BEIT-4F | Huang, X. et al., Appl. Phys. Lett, 2020, 116 (15), 153501. |

**Solar cell fabrication.** Pre-patterned ITO-coated glass substrates with sheet resistances of 15 Q/sq were purchased from Luminescence Technology Corp. Prior to thin film deposition, the substrate surface was detergent and solvent cleaned with acetone and isopropanol, followed by $CO_2$ snow cleaning and exposure to ultraviolet-ozone for 15 min. The ZnO layer (ca. 30 nm) was spin cast from a ZnO precursor solution onto the substrates and then thermally annealed at 150 °C for 30 min in air. The IC-SAM was dissolved in methanol with a concentration of 1 mg/ml and spin coated at 4000 rpm for 60s, followed by thermal annealing at 110 °C for 10 min. The IC-SAM coated substrate was washed with methanol to remove residues. The "hot" ternary blend active layer solution was prepared in 20 mg/ml PCE-10:BT-IC:BTIC-4Cl (1:0.75:0.75, w/w/w) in CB at 65 °C for 15 h. The solution was cooled to room temperature and filtered once with a 0.45 μm polytetrafluoroethylene (PTFE) syringe filter prior to use, and then spin-coated onto the substrate at 2000 rpm for 90 s to achieve a thickness of 80-90 nm. The cold ternary blend solution was prepared by dissolving PCE-10 in a hot CB and then cooled to room temperature and mixing with BT-IC and BTIC-4Cl in a 1:0.75:0.75 ratio. The solution was stirred for 0.5 h and filtered before fabricating the OPV The samples were transferred into a vacuum chamber connected to glove boxes filled with ultrapure $N_2$ ($O_2$, $H_2O$ < 0.1 ppm). The $C_{70}$, MoOs and Al films were deposited at 0.2 Å /s in a high vacuum chamber with a base pressure of $10^{-7}$ torr. The deposition rates and thicknesses were measured using quartz crystal monitors and calibrated post-growth by variable-angle spectroscopic ellipsometry. Device areas of 2 mm × 2 mm were defined by the overlap of the ITO anode and the Al cathode with a 50 μm thick shadow mask.

**Solar cell characterization.** The current density-voltage (*J-V*) characteristics of the cells were measured in a glovebox filled with ultrapure $N_2$. Light from a Xe lamp filtered to achieve a simulated AM 1.5G spectrum (ASTM G173-03) was used as the source for *J- V* measurements. The lamp intensity is varied using neutral density filters, and calibrated with a Si reference cell certified by the National Renewable Energy Laboratory. Each cell was measured under six

different light intensities from 0.001 sun to 1 sun (100 mW/cm$^2$). The *Jsc* were calculated from the EQE spectra, with < 7% relative mismatch of the measured *Jsc* from the *J-V* characteristics. The error bars quoted in the tables take into account both random and systematic errors.

**Device stability measurements.** All devices were encapsulated by bonding a glass cover slide to the glass substrate using an ultraviolet-curable epoxy in a glovebox filled with ultrahigh purity nitrogen. All aging in the light soaking chamber was performed on the encapsulated devices in air under 10 ± 1.2 suns illumination intensity.

High-intensity illumination from light emitting diodes (LEDs) operating at various intensities was concentrated onto the OPVs with Ag-coated reflective light pipes. The LED intensity that produced a photocurrent equivalent to *Jsc* under AM1.5G illumination ($J_{SC}$, AM1.5G) was equivalent to 1-sun intensity. To calibrate higher LED intensities, a neutral-density filter was used to ensure that the OPVs remained in their linear regime. The neutral-density filter was 10% transmissive, thus the equivalent solar intensity (in units of suns) of the LEDs was calculated using the following formula (Burlingame, Q. et al. Nature 573, 394-397 (2019)).

$$Equivalent\ solar\ intensity = \frac{10\, J_{photon,LED}}{J_{SC,AM\ 1.5G}} \qquad (1)$$

To avoid excessive heating at high intensity, the OPV cells were actively water cooled to 33 ± 5 °C at 10 ± 1.2 suns with a closed-loop chiller.

**Molecular structure characterization.** NMR spectra were collected using a Varian MR400 spectrometer (400 MHz, $^1$H; 101 MHz, $^{13}$C; 376 MHz, $^{19}$F) in deuterated chloroform solution. with trimethylsilane (TMS) as reference.

Laser desorption/ionization time of flight mass spectra were collected using a Bruker AutoFlex Speed MALDI-TOF instrument calibrated with Peptide Calibration Standard II. The samples used for mass spectral measurements were prepared in 1 mg/ml dichloromethane. Acid matrix used was prepared with a-cyano hydrocinnamic acid and basic matrix prepared with 1,5-diamino naphthalene.

**Grazing Incidence Wide Angle X-ray Scattering (GIWAXS) measurements:** The thin films on Si wafer substrates were measured at Beamline 11 of the National Synchrotron Light Source II (NSLS-II), Brookhaven National Lab (BNL). The X-ray energy was 13.5 keV and the scattering patterns were recorded on a 2D image plate (Pilatus 800K) with a beam size of 200 μm. The samples were ~10 mm long in the direction of the beam path, and the detector was located at a distance of 259 mm from the sample center (distance calibrated by a AgB reference). The incidence angle was 0.1° and exposure time was 20s.

**Resonant soft X-ray scattering (RSoXS) measurements:** For RSoXS samples, poly(3,4-ethylenedioxythiophene) polystyrene sulfonate (PEDOT:PSS) films were cast on Si substrates followed by the organic films under test. The samples were immersed in deionized water to dissolve the PEDOT:PSS. The floated organic films were then picked up by a 2 mm × 2 mm $SiN_x$ membrane window (Norcada NX5200C) and mounted onto an Al rack. The carbon-edge RSoXS profiles were measured at beamline 11.0.1.2 of the Advanced Light Source (ALS), Lawrence Berkeley National Lab (LBNL).

**Electroluminescence measurements.** The EL spectra were measured under 2V and 10 mA forward bias for fresh devices. The spectra were collected using a fiber-coupled monochromator (Princeton Instruments SP-2300i) equipped with a Si charge-coupled device (CCD) (PIXIS:400) for spectra between 700 nm and 950 nm, and an InGaAs photodiode array (PyLoN-IR:1024) from 950 nm to 1400 nm.

**Dipole moment and polarizability calculations.** The dipole moments and polarizabilities of the molecules are calculated using Materials Studio 7.0 and GAUSSIAN 09W package 1. The molecular geometries are first preliminarily optimized with the Forcite module in Materials Studio 7.0. GAUSSIAN 09W was then used for further geometry optimization and dipole moment calculation with the B3LYP functional and the 6-31G(d) basis set. The polarizable continuum solvation model (PCM) is used with a dielectric constant of 3.24 to account for the effect of surrounding molecules in the thin film.

**Charge-extraction efficiency measurements** The OPVs have a relatively thin organic active layer (less than 100 nm) that can be easily damaged at high reverse bias. For this reason, the voltage-dependent photocurrent, $J_{photo}$(V), was measured to a voltage of -2.0 V, and a fitting function was used to determine the reverse bias saturation photocurrent ($J_{photo,sat}$), namely[49]:

$$J_{photo}(V) = J_{photo,sat} + c_1 e^{V/c_2}$$

where $c_1$ and $c_2$ are constants. The charge extraction efficiency at $V$ = 0 was equal to the ratio: $J_{photo}$ ($V$ = 0) / $J_{photo,sat}$.

**Optical characterization.** The absorbance of dilute solutions was collected using a calibrated PerkinElmer

Lambda 1050 ultraviolet-visible spectrometer.

Laser desorption/ionization time of flight mass spectra were collected using a Bruker AutoFlex Speed MALDI-TOF instrument calibrated with Peptide Calibration Standard II. The samples used for mass spectral measurements were prepared in 1 mg/ml dichloromethane. Acid matrix used was prepared with a-cyano hydrocinnamic acid and basic matrix prepared with 1,5-diamino naphthalene.

Synthesis of BTIC-2Cl.

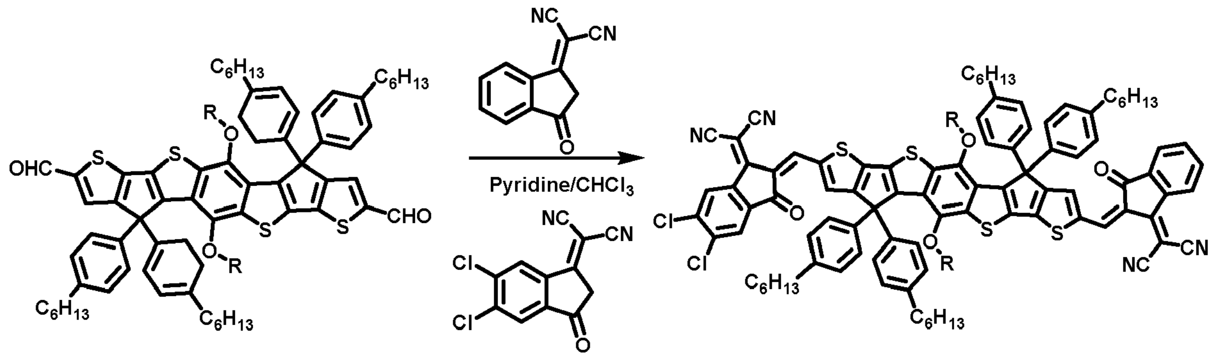


BT-CHO

BTIC-2Cl

R: 2-ethylhexyl

(4,4,10,10-tetrakis(4-hexylphenyl)-5,11-(2-ethylhexyloxy)-4,10-dihydro-dithienyl[1,2-b:4,5b']benzodi-thiophene-2,8-diyl)bis(formaldehyde) (BT-CHO) was synthesized according to previously reported methods. 3-(dicyanomethylidene)-5,6-dichloro-indan-1-one (105 mg, 0.4 mmol) and 3-(dicyanomethylidene)indan-1-one (78 mg, 0.4 mmol) were added into the mixture of compound BT-CHO (400 mg, 0.3 mmol) in anhydrous chloroform with pyridine (1 mL), the reaction was deoxygenated with nitrogen for 30 min and then refluxed for 10 h. After cooling to room temperature, the solution was poured into methanol and the precipitate was filtered off. Then it was extracted with DCM and washed with water. The crude product was purified by silica gel column using a mixture of hexane/DCM (3:2) as the eluent to give a purple solid (150 mg, 29%). $^1$H NMR (δ): 8.81 (s, 2H), 8.74 (s, 1H), 8.66 (d, *J* = 7.5 Hz, 1H), 7.90 (s, 1H), 7.88 (s, 1H), 7.78 - 7.67 (m, 2H), 7.50 (s, 1H), 7.49 (s, 1H), 7.29 (m, 8H), 7.08 (d, *J* = 8.2 Hz, 8H), 3.47 (m, 4H), 2.65 - 2.40 (m, 8H), 1.67 - 1.54 (m, 8H), 1.43 - 1.09 (m, 42H), 0.95 (t, *J* = 7.2 Hz, 6H), 0.84 (m, 18H). $^{13}$C NMR (δ):188.5, 186.2, 164.8, 164.5, 160.2, 158.2, 158.1, 157.1, 155.7, 153.7, 146.5, 146.2, 142.3, 142.2, 140.8, 139.9, 139.5, 139.2, 139.0, 138.8, 138.6, 138.4, 138.2, 136.8, 136.1, 136.0, 135.9, 135.7, 135.0, 134.3, 128.7, 128.5, 128.3, 128.1, 126.9, 125.3, 125.0, 123.6, 121.4, 120.2, 114.8, 114.4, 68.8, 68.5, 63.9, 39.4, 35.6, 31.7, 31.3, 29.6, 29.2, 28.8, 23.3, 22.7, 22.6, 14.2, 14.1, 10.8.

Synthesis of 2-(2-benzylidene-3-oxo-2,3-dihydro-1H-inden-1-ylidene)malononitrile (PhIC).

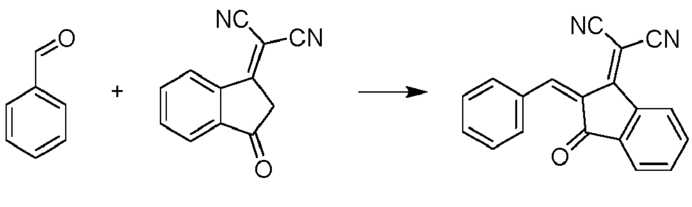

The compound was synthesized based on a modified literature procedure. Under an inert atmosphere of nitrogen using standard Schlenk techniques, 1-(Dicyanomethylene)-3-indanone (183.2 mg, 943.39 mmol) was dissolved in 20 mL of toluene. Benzaldehyde (0.09 mL, 0.9 g, 900 mmol) and 3 drops of pyridine as a catalyst was added and the reaction was heated to reflux for four h. The solvent was removed *in vacuo* to give a black solid. It was washed 3x with sat. $K_2CO_{3(aq)}$, 3x with methanol, and 3x with ether, affording a pure green powder (63.4 mg, 224.6 mmol, 25.4% yield).

Synthesis of 2-(2-(4-(dimethylamino)benzylidene)-3-oxo-2,3-dihydro-1H-inden-1-ylidene)malononitrile ($NMe_2$PhIC).

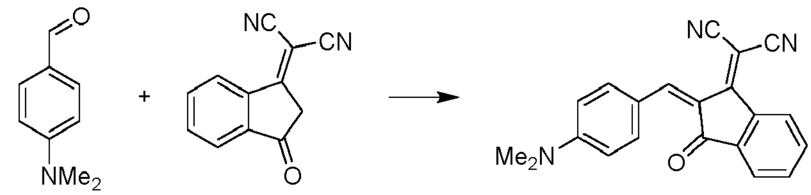

1-(Dicyanomethylene)-3-indanone (100.6 mg, 518.04 mmol) and (74.5 mg, 499.35 mg) and 4-dimethylaminobenzaldehyde were dissolved in 5 mL ethanol. The solution quickly turned from grey to purple. 10 mL piperidine was added and the solution turned red. The solution was then refluxed for 4 h. The solvent was removed *in vacuo* to give an orange solid. The solid was washed 3x with cold ethanol and 2x with ether, affording a dark purple powder (101.2 mg, 311.03 mmol, 62.3 %).

Synthesis of 5-benzylidene-1,3-dimethylpyrimidine-2,4,6(1H,3H,5H)-trione (PhBarb).

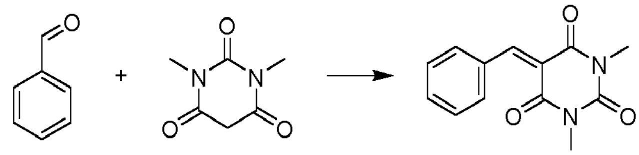

NN'-dimethylbarbituric acid (641.9 mg, 4.11 mmol) was dissolved in 25 mL $H_2O$. Benzaldehyde (0.5 mL, 5 mg, 5 mmol) was added and the solution was left to stir for 2 h in which time a precipitate was formed. The precipitate was washed 3x with ethanol and 3x with ether to afford a white powder (977 mg, 3.40 mmol, 69.4%). $^1$H NMR (δ)8.58 (s, 1H), 8.11 - 8.00 (m, 2H), 7.58 - 7.43 (m, 3H), 3.40 (d, *J* = 19.5 Hz, 6H).

Synthesis of 5-(4-(dimethylamino)benzylidene)-1,3-dimethylpyrimidine-2,4,6-(1H,3H,5H)-trione (NMe2PhBarb).

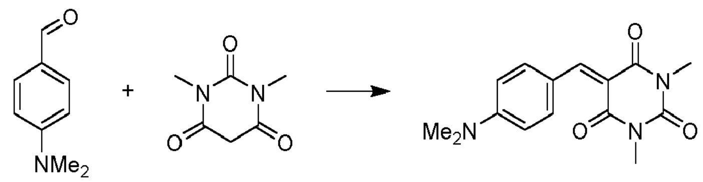

NN'-dimethylbarbituric acid (641.9 mg, 4.11 mmol) and 4-dimethylbenzaldehyde (493.6 mg, 3.31 mmol) were dissolved in 10 mL ethanol. The solution immediately turned orange and precipitate began to form (confirmed by dissolving the two materials in ethanol individually which formed translucent solutions and then adding them together to generate an orange solution which began precipitating). The solution was refluxed for 6 h. The solution was then cooled in an ice bath and washed 3x with cold ethanol and 2x with ether to afford a salmon-colored powder (870.5 mg, 3.03 mmol, 91.5%). $^1$H NMR (δ) 8.44 (s, 1H), 8.43 - 8.36 (m, 2H), 6.77 - 6.65 (m, 2H), 3.40 (d, *J* = 3.3 Hz, 6H), 3.15 (s, 6H).

Hydrous Exchange of Endcaps reaction.

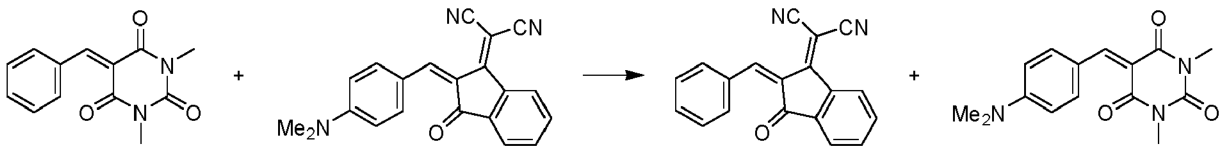

Under an inert atmosphere of nitrogen using standard Schlenk techniques, PhIC (10.6 mg, 43.40 mmol) and

NMe$_2$PhBarb (10.7 mg, 32.89 mmol) were added to 10 mL of hydrous chlorobenzene to form an orange-yellow solution. After refluxing 16 h, the solution turned dark red. The solvent was removed *in vacuo.* $^1$H-NMR spectroscopy revealed a mixture of product and reactants in a 1:1.44 ratio, as well as peaks at 10.02 and 9.73 belonging to the aldehyde proton of benzaldehyde and 4-methylamino-benzaldehyde respectively. Only the aldehyde peaks were observed due to the sharpness of these peaks granting them the ability to stand above the baseline.

Anhydrous Non-exchange of Donor groups between NMe$_2$PhIC and Benzaldehyde.

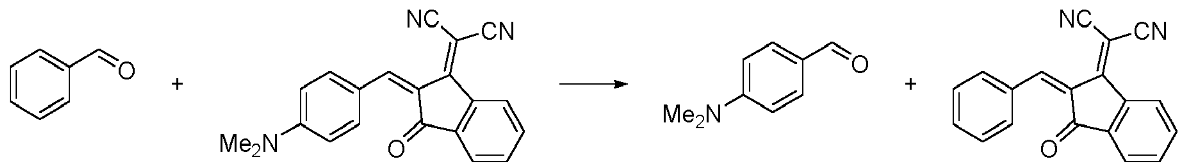

Under an inert atmosphere of nitrogen using standard Schlenk techniques, NMe2PhIC (10.3 mg, 31.7 mmol) and benzaldehyde (0.05 mL, 52.0 mg, 490 mmol) were added to 10 mL of dry chlorobenzene taken form an anhydrous sealed bottled and further dried over 3 Å molecular sieves to make a purple solution. After refluxing for 48 h, the solution remained purple. The solvent was removed in vacuo. $^1$H-NMR spectroscopy revealed only starting material.

Anhydrous exchange of acceptor groups between NMe$_2$PhIC and 1,3-dimethylbarbituic acid.

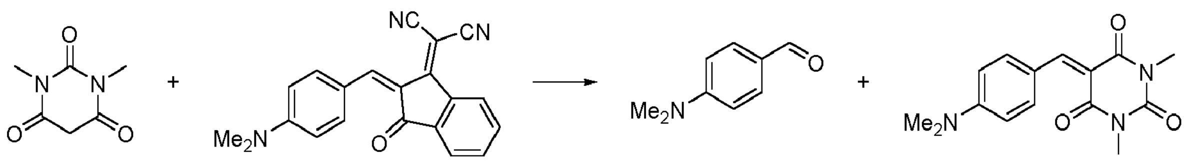

Under an inert atmosphere of nitrogen using standard Schlenk techniques, NMe$_2$PhIC (10.6 mg, 32.5 mmol) and 1,3-dimethylbarbiutate (5.6 mg, 36 mmol) were added to 10 mL of dry chlorobenzene taken form an anhydrous sealed bottled and further dries over 3 Å molecular sieves to make a purple solution. After refluxing for 48 h, the solution turned deep red. The solvent was removed in vacuo. $^1$H-NMR spectroscopy revealed a mixture of products and reactants in a 1:1 ratio. Furthermore, no trace of aldehyde protons were observed.

**Characterization of end-capping exchange between BT-IC and BTIC-4Cl.** The hot ternary blend solution was prepared by the mixture of BT-IC:BTIC-4Cl (1:1, w/w) in CB at 65 °C for 15 h with a concentration of 16 mg/ml. After reaction, the product was isolated via the column chromatography and characterized by NMR spectroscopy. Note that the $^1$H NMR spectrum of BTIC-2Cl (green line) resembles a superposition of the BT-IC and BTIC-4Cl spectra, it is difficult to distinguish the product from the blend of reactants.

**The reaction kinetics of the end-capping exchange.** A 16 mg/ml solution of BT-IC:BTIC-4Cl (1:1, w/w) in CB was prepared at temperatures ranging from 0 °C to 100 °C for 24 h. The solvent was removed, and the crude mixture was dissolved in CDCl$_3$ and analyzed by $^{19}$F{$^1$H} NMR spectroscopy. The conversion ratio (*r*) was calculated by integration of the $^{19}$F{$^1$H} NMR spectral peak of the product relative to the peak of the original material.

The reaction between two archetype NFAs BTIC and BTIC-4F is carried out in CDCl$_3$ at 50 °C for 16h, and the formation of the corresponding product, BTIC-2F is monitored by $^{19}$F{$^1$H} NMR spectroscopy. BTIC-2F was absent when starting from anhydrous solvent (Fig. 31). In contrast, for the solvent containing 0.3% water, a significant amount of BTIC-2F is produced.

Reactions of NMe$_2$PhIC with both benzaldehyde and 1,3-dimethylbarbituric acid (DMB) in anhydrous CB were studied to determine free donors or acceptors can directly exchange with a D-A molecule. In the absence of water, benzaldehyde failed to exchange with the 4-dimethylaminobenzene moiety of NMe$_2$PhIC to form PhIC and 4-dimethyl-aminobenzaldehyde, however reacting NMe$_2$PhIC with 1,3-dimethylbarbituric acid did result in the formation of NMe$_2$Barb and free IC.

Two possible mechanisms are proposed. The first involves two compounds, $D_1$-$A_1$ and $D_2$-$A_2$, both undergoing retro-Knoevenagel syntheses to form two different aromatic aldehydes and two different activated methylenes. These components are then allowed to freely react to form a total of four Knoevenagel compounds, the two original D-A molecules, and two new molecules, $D_1$-$A_2$ and $D_2$-$A_1$, not originally present.

Another mechanism involves $D_m$-$A_m$ undergoing retro-Knoevenagel condensation to form an aromatic aldehyde and activated methylene. The activated methylene adds to the double bond of $D_n$-$A_n$ to form a geminal intermediate.

This intermediate then eliminates $A_n$ to reform $D_n$-$A_m$. $A_n$ then reacts with either the aromatic aldehyde form of $D_m$ (via mechanism 1) or add to $D_m$-$A_m$ followed by elimination of $A_m$. In either case, the product molecule is of the form $D_1$-$A_2$.

To eliminate the possibility of morphological changes coming from individual molecules in the hot fabrication process, GIWAXS patterns of the neat films with and without heating were compared, with no obvious changes observed (Fig. 51). This provides additional evidence that molecular packing changes observed in the hot blend film is due to the generation of BTIC-2Cl in the mixture.

The resonant soft X-ray scattering (RSoXS) spectra of the cold blend of BT-IC:BTIC-4Cl (1:1, w/w) shows a narrow and sharp scattering peak at $Q$ = 0.01 Å$^{-1}$, corresponding to a domain size of 63 nm (see Fig. 41), whereas no obvious peaks can be found in the hot blend, providing further support that BTIC-2Cl reduces the crystallinity of the blends.

It is understood that the various embodiments described herein are by way of example only, and are not intended to limit the scope of the disclosure. For example, many of the materials and structures described herein may be substituted with other materials and structures without deviating from the spirit of the disclosure. The present compounds as disclosed may therefore include variations from the particular examples and preferred embodiments described herein, as will be apparent to one of skill in the art. It is understood that various theories as to why the embodiments work are not intended to be limiting.

## Claims

1. A compound of Formula I:

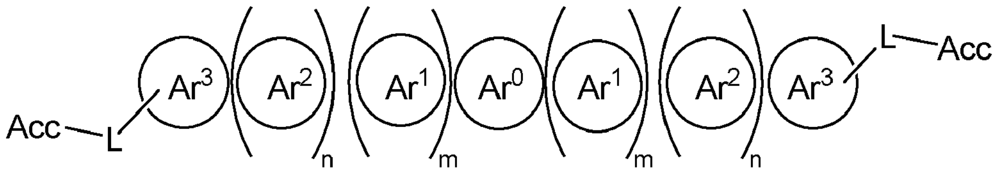

Formula I

wherein $Ar^0$ is an aromatic or non-aromatic ring connected to or fused at two positions to $Ar^1$, $Ar^2$, or $Ar^3$;
each $Ar^1$ is an aromatic ring or a non-aromatic ring that is connected to or fused to Ar° and to $Ar^2$, $Ar^3$, or another occurrence of $Ar^1$;
each $Ar^2$ is an aromatic ring or a non-aromatic ring that is connected to or fused to $Ar^1$ or Ar° and to $Ar^3$ or another occurrence of $Ar^2$;
$Ar^3$ is an aromatic ring that is connected to or fused to $Ar^2$, $Ar^1$, or $Ar^0$ and is connected to or fused to A; m is from 0 to 10;
n is from 0 to 10;
Acc is an acceptor group connected or fused to $Ar^3$;
L is a linking group selected from the group consisting of a single bond and 1,2-cyclopentene;
Ar° represented by one of the following structures:

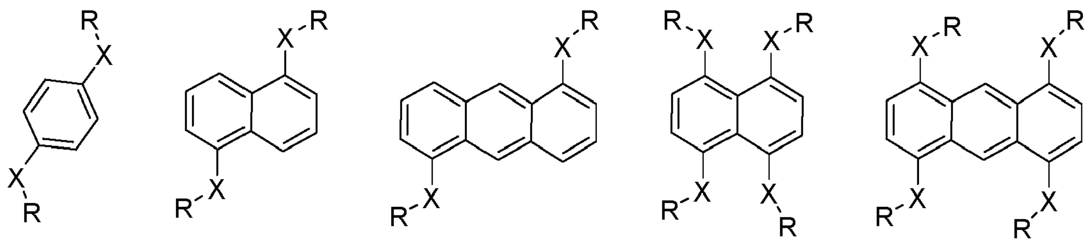

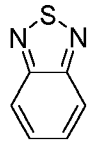 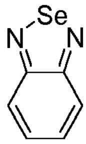 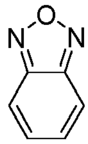 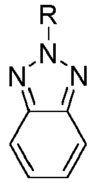 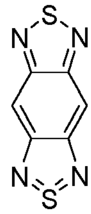 ;

each occurrence of $Ar^1$ is independently represented by one of the following structures:

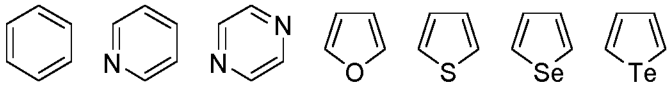

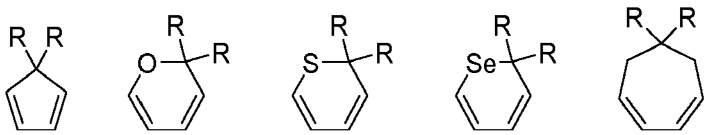

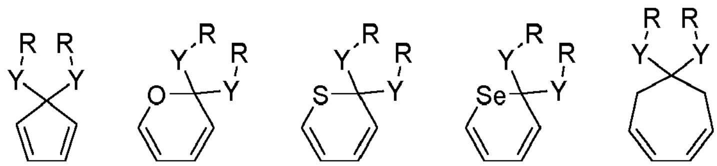

each occurrence of $Ar^2$ is independently represented by one of the following structures:

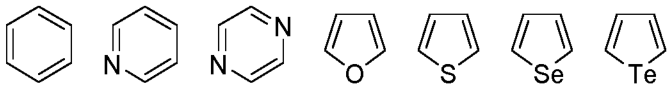

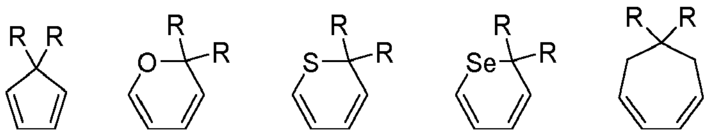

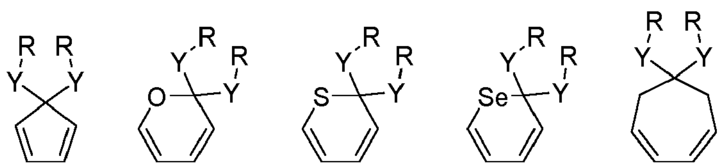 ;

each occurrence of Y is represented by one of the following linking groups:

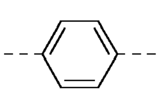 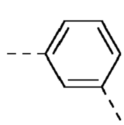 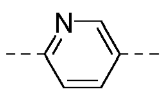 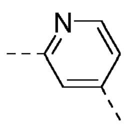 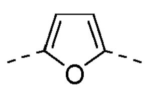 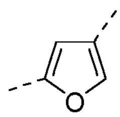

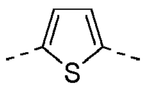 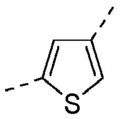 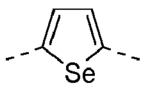 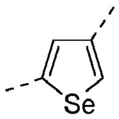 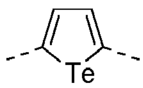 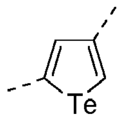

$Ar^3$ is represented by one of the following structures:

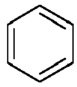 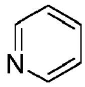 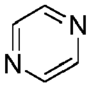 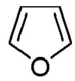 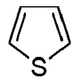 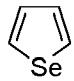 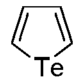

each occurrence of X is a single bond or a heteroatom selected from the group consisting of O, S, Se, and NR; Acc is connected to ring $Ar^3$ by a single bond and is represented by one of the following structures:

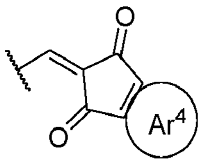 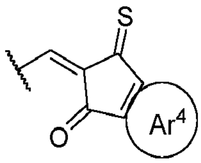 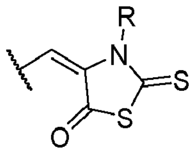 ;

or

Acc is fused to ring $Ar^3$ and is represented by one of the following structures, wherein waved lines indicate connection to adjacent carbon atoms on $Ar^3$:

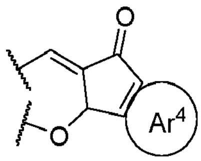 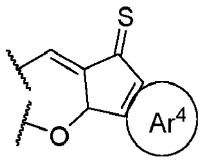 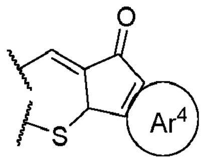 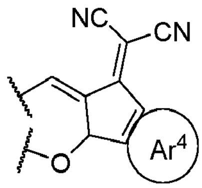

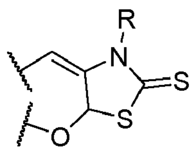 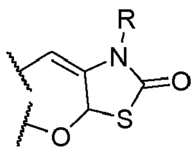 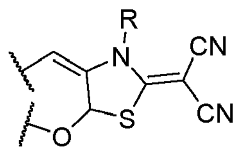

$Ar^4$ is represented by one of the following structures:

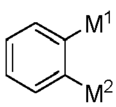 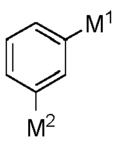 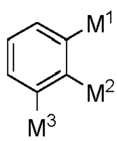 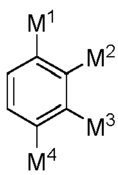 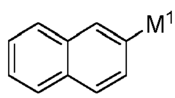 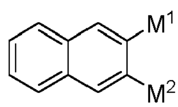 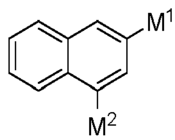

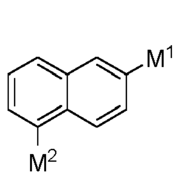 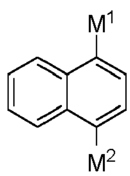 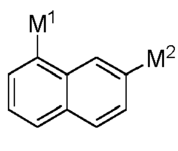 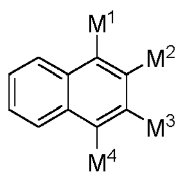

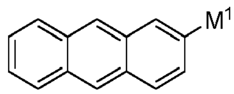 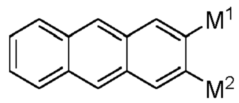 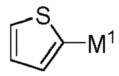 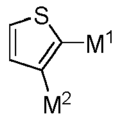 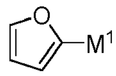 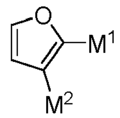

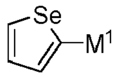 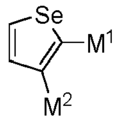 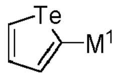 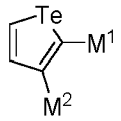 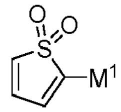 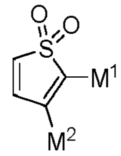

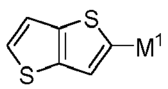 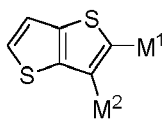 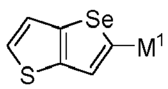 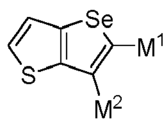 ;

$M^1$, $M^2$, $M^3$, and $M_4$ are individually selected from the group consisting of hydrogen, fluorine, chlorine, bromine, iodine, astatine, and a cyano group, wherein at least one of $M_1$-$M_4$ is a halogen

each R independently represents hydrogen or a substituent selected from the group consisting of deuterium, halogen, alkyl, cycloalkyl, heteroalkyl, heterocycloalkyl, arylalkyl, alkoxy, aryloxy, amino, thioalkyl, silyl, alkenyl, cycloalkenyl, heteroalkenyl, alkynyl, aryl, heteroaryl, acyl, carboxylic acid, ether, ester, nitrile, isonitrile, sulfanyl, sulfinyl, sulfonyl, phosphino, and combinations thereof; wherein any two adjacent substituents optionally join to form a ring.

**2.** The compound of claim 1, wherein the compound is represented by Formula II, Formula III, Formula IV, or Formula V:

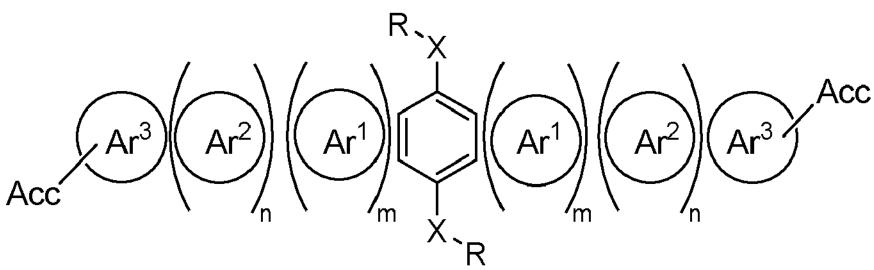

Formula II

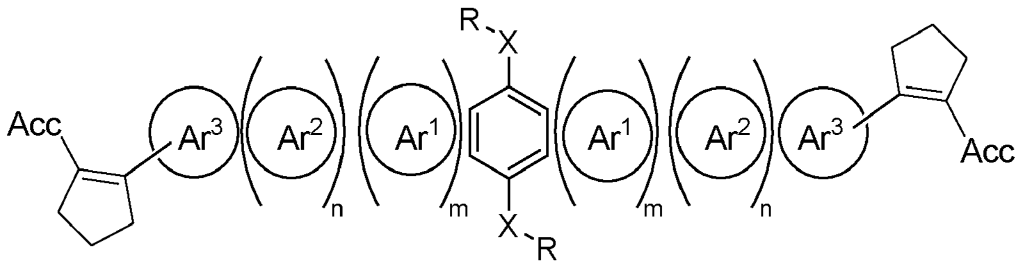

Formula III

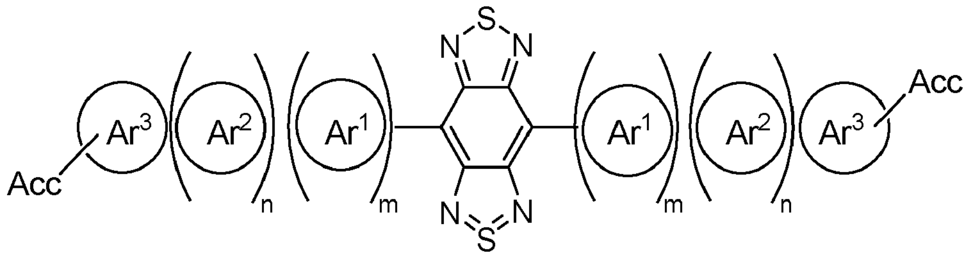

Formula IV

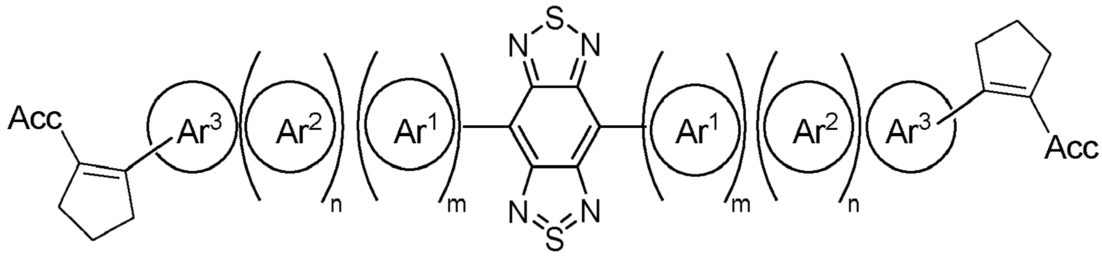

Formula V.

**3.** The compound of claim 1, wherein the compound is represented by one of the following structures:

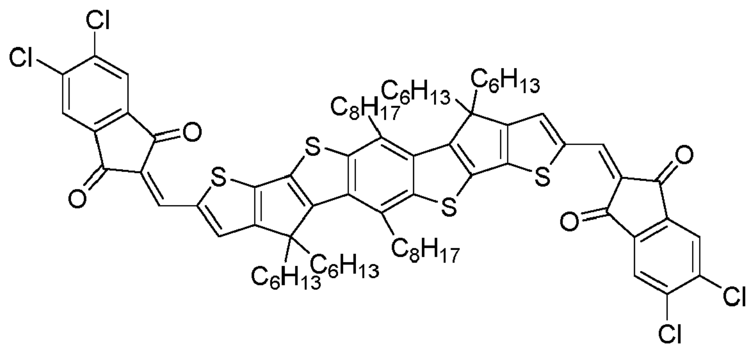

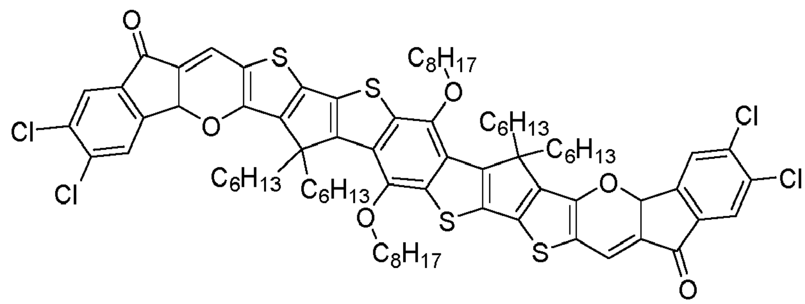

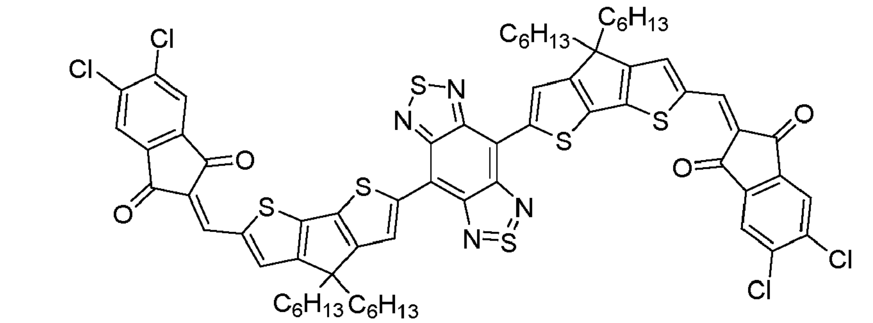

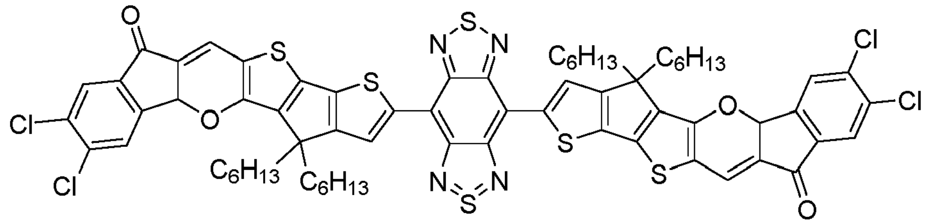

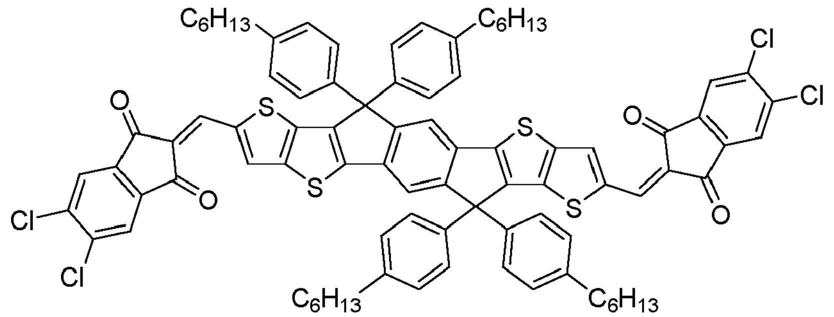

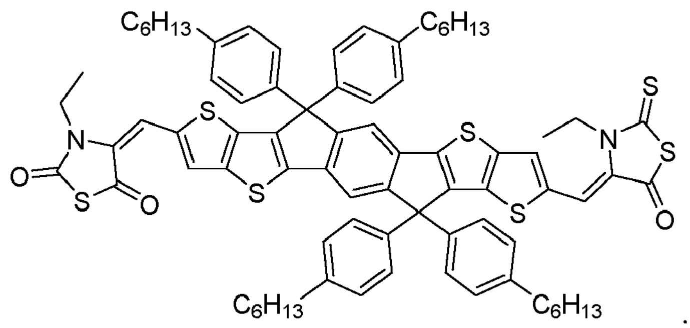

.

**4.** An optoelectronic device comprising a compound of any one of the previous claims, or of Formula I:

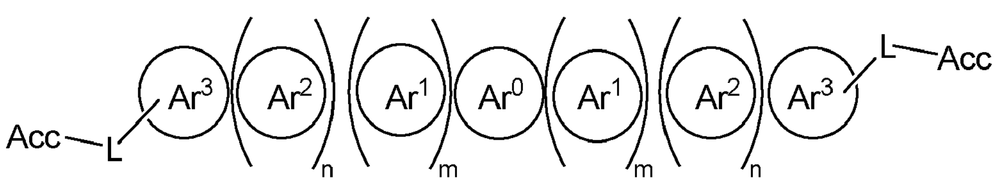

Formula I

wherein $Ar^0$ is an aromatic or non-aromatic ring connected to or fused at two positions to $Ar^1$, $Ar^2$, or $Ar^3$; each $Ar^1$ is an aromatic ring or a non-aromatic ring that is connected to or fused to Ar° and to $Ar^2$, $Ar^3$, or another

occurrence of $Ar^1$;
each $Ar^2$ is an aromatic ring or a non-aromatic ring that is connected to or fused to $Ar^1$ or Ar° and to $Ar^3$ or another occurrence of $Ar^2$;
$Ar^3$ is an aromatic ring that is connected to or fused to $Ar^2$, $Ar^1$, or $Ar^0$ and is connected to or fused to A; m is from 0 to 10;
n is from 0 to 10;
Acc is an acceptor group connected or fused to $Ar^3$;
L is a linking group selected from the group consisting of a single bond and 1,2-cyclopentene;
Ar° represented by one of the following structures:

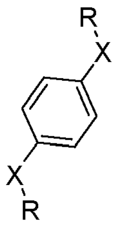 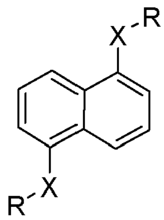 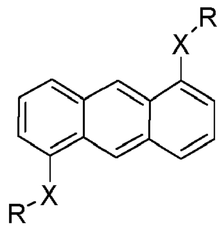 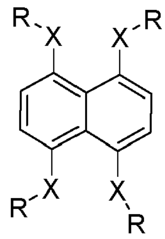 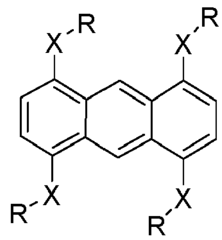

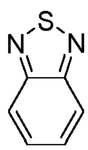 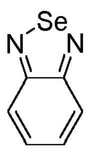 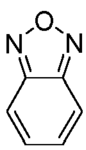 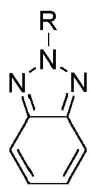 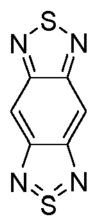 ;

each occurrence of $Ar^1$ is independently represented by one of the following structures:

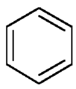 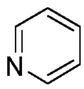 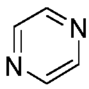 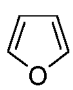 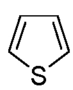 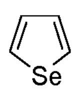 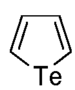

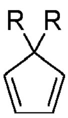 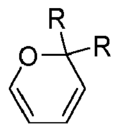 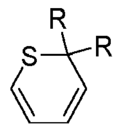 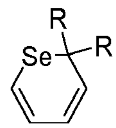 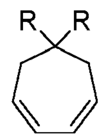

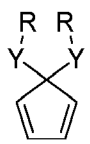 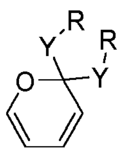 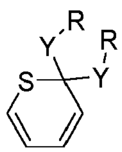 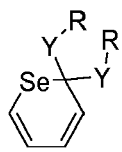 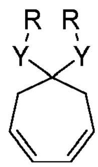

each occurrence of $Ar^2$ is independently represented by one of the following structures:

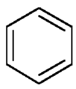 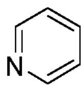 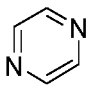 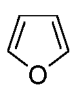 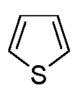 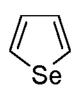 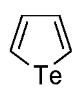

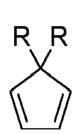 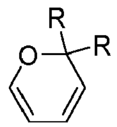 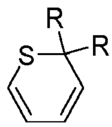 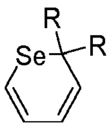 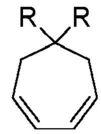

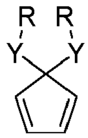 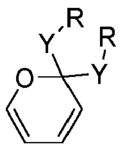 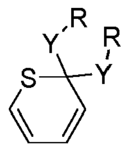 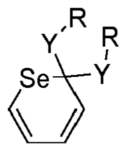 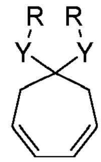 ;

each occurrence of Y is represented by one of the following linking groups:

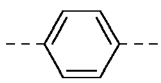 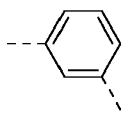 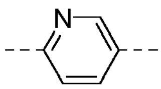 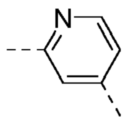 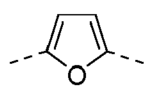 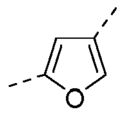

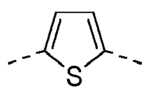 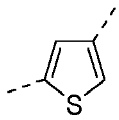 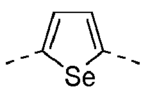 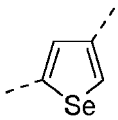 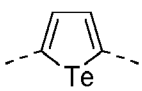 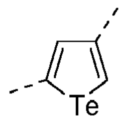

$Ar^3$ is represented by one of the following structures:

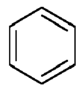 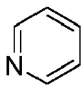 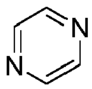 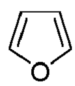 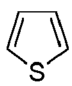 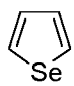 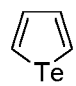

each occurrence of X is a single bond or a heteroatom selected from the group consisting of O, S, Se, and NR; Acc is connected to ring $Ar^3$ by a single bond and is represented by one of the following structures:

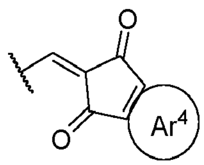 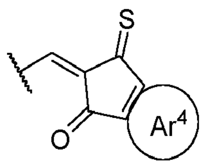 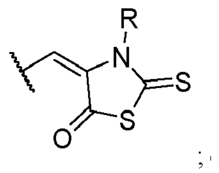 ;

or

Acc is fused to ring $Ar^3$ and is represented by one of the following structures, wherein waved lines indicate connection to adjacent carbon atoms on $Ar^3$:

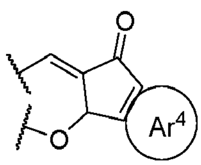 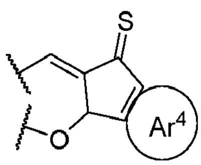 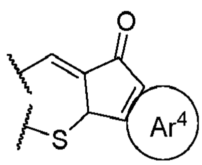 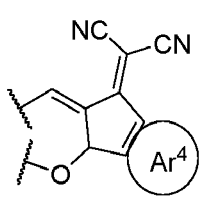

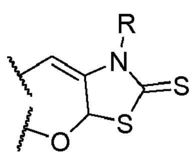 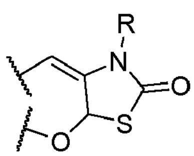 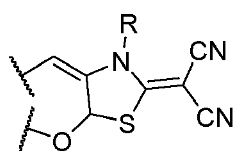

$Ar^4$ is represented by one of the following structures:

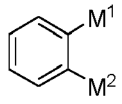 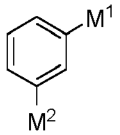 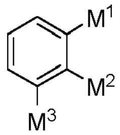 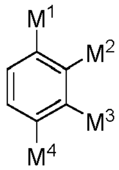 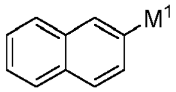 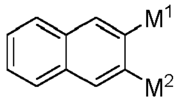 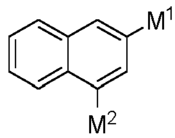

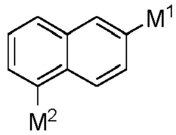 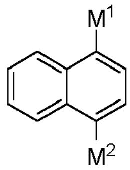 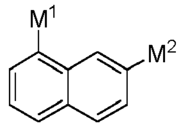 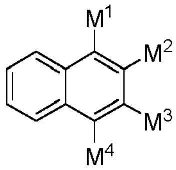

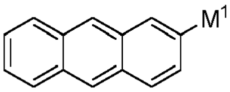 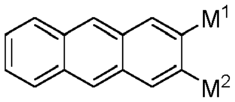 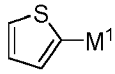 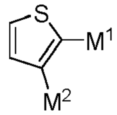 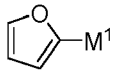 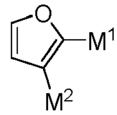

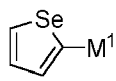 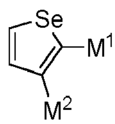 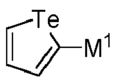 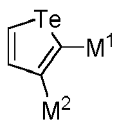 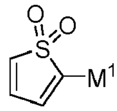 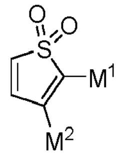

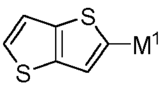 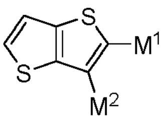 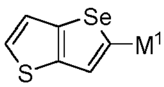 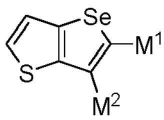 ;

$M^1$, $M^2$, $M^3$, and $M_4$ are individually selected from the group consisting of hydrogen, fluorine, chlorine, bromine, iodine, astatine, and a cyano group, wherein at least one of $M_1$-$M_4$ is a halogen
each R independently represents hydrogen or a substituent selected from the group consisting of deuterium, halogen, alkyl, cycloalkyl, heteroalkyl, heterocycloalkyl, arylalkyl, alkoxy, aryloxy, amino, thioalkyl, silyl, alkenyl, cycloalkenyl, heteroalkenyl, alkynyl, aryl, heteroaryl, acyl, carboxylic acid, ether, ester, nitrile, isonitrile, sulfanyl, sulfinyl, sulfonyl, phosphino, and combinations thereof; wherein any two adjacent substituents optionally join to form a ring.

**5.** The optoelectronic device of claim 4, wherein the optoelectronic device is selected from the group consisting of an organic light-emitting device (OLED), organic phototransistor, organic photovoltaic cell, and organic photodetector.

**6.** The optoelectronic device of claim 4, wherein the optoelectronic device is a photovoltaic cell.

**7.** A consumer product comprising the optoelectronic device of claim 4 or 5.

**8.** A formulation comprising the compound of any one of claims 1 to 3.

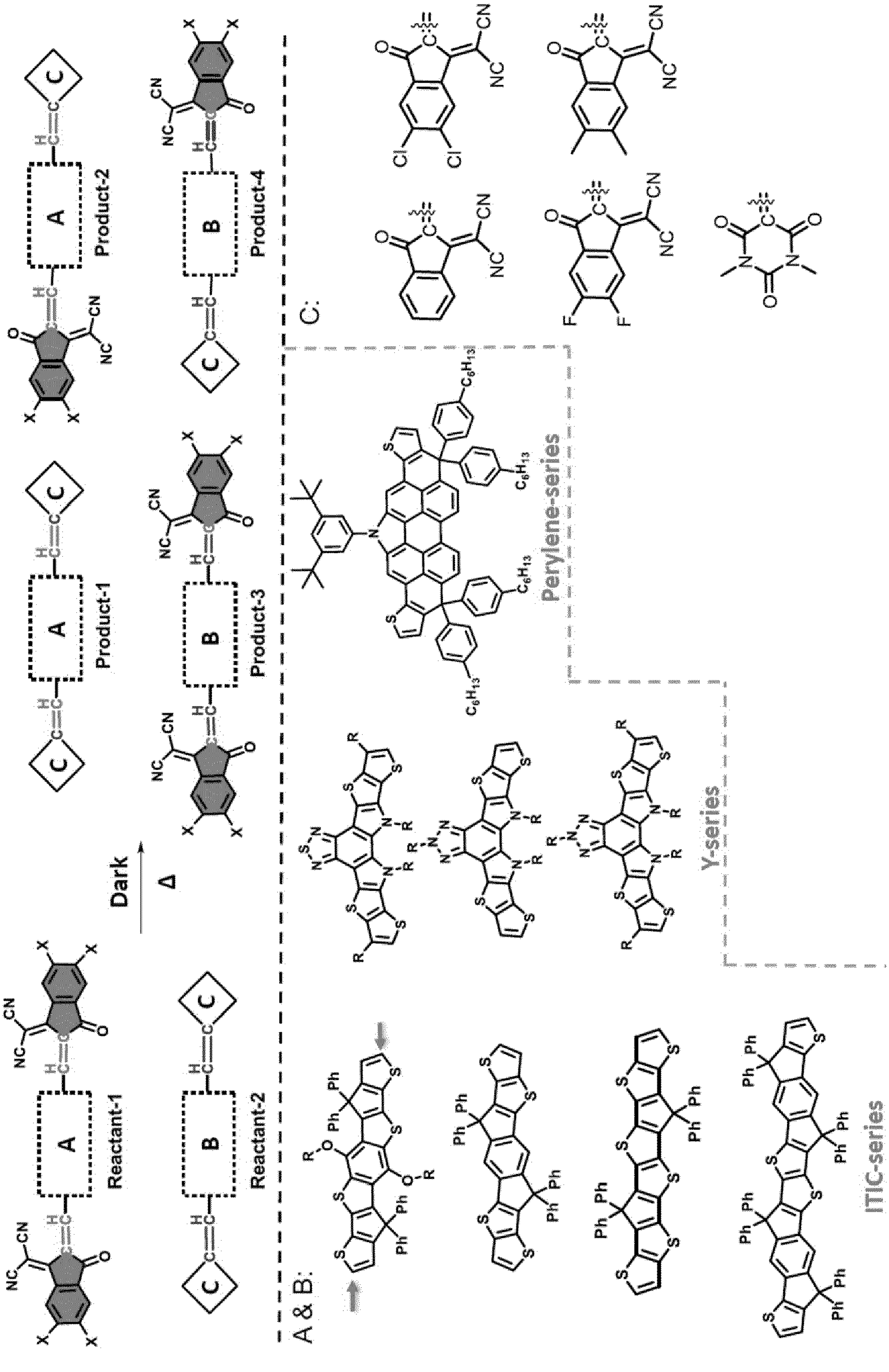


Reactant-1
Reactant-2
Product-1
Product-2
Product-3
Product-4
A & B:
C:
ITIC-series
Y-series
Perylene-series

FIG. 1

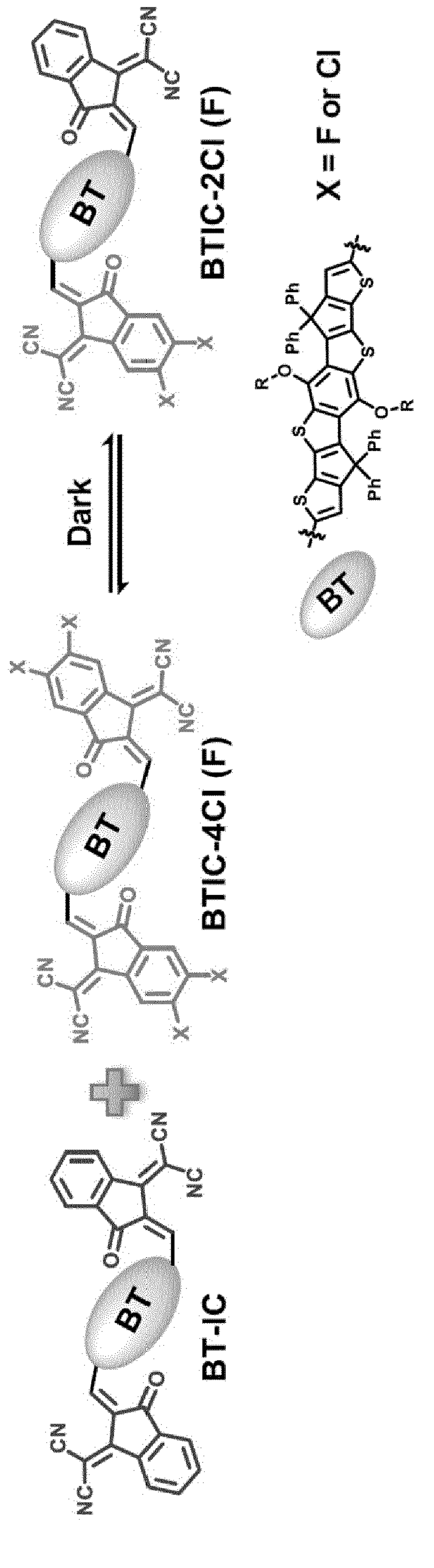


BT-IC

BTIC-4Cl (F)

BTIC-2Cl (F)

X = F or Cl

FIG. 2

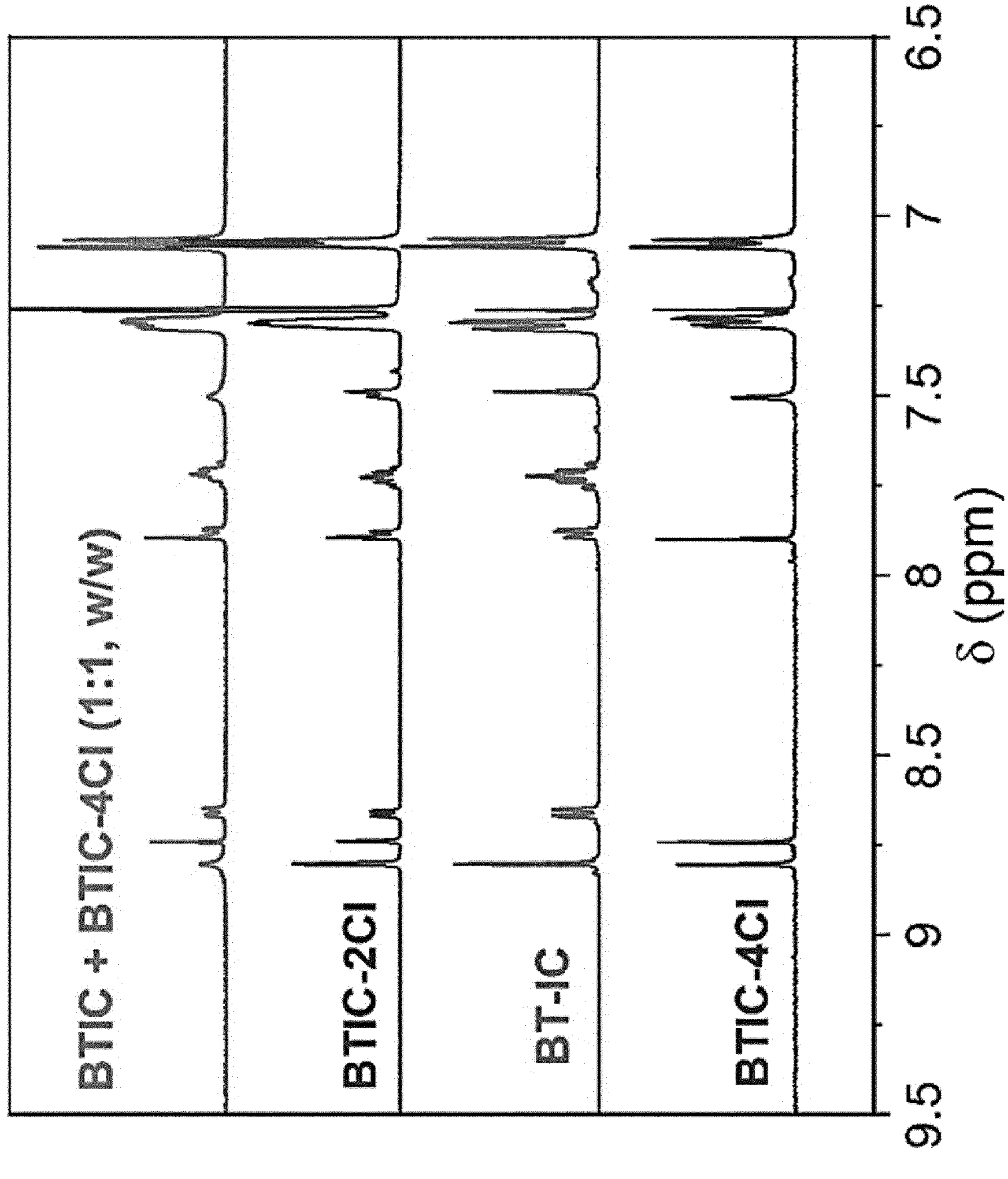


FIG. 3

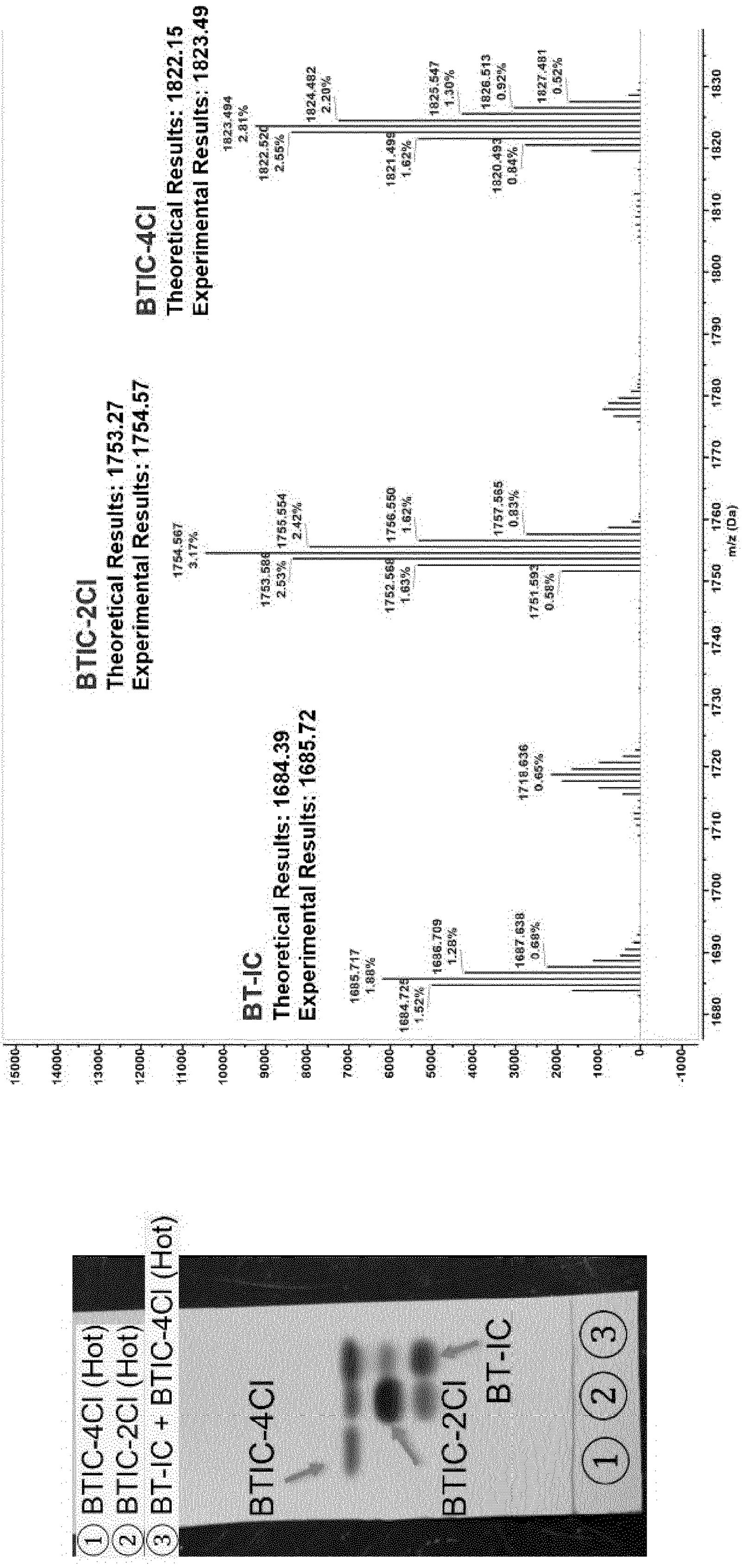

① BTIC-4Cl (Hot)
② BTIC-2Cl (Hot)
③ BT-IC + BTIC-4Cl (Hot)
BTIC-4Cl
BTIC-2Cl
BT-IC
BT-IC
Theoretical Results: 1684.39
Experimental Results: 1685.72
BTIC-2Cl
Theoretical Results: 1753.27
Experimental Results: 1754.57
BTIC-4Cl
Theoretical Results: 1822.15
Experimental Results: 1823.49
m/z (Da)


FIG. 4

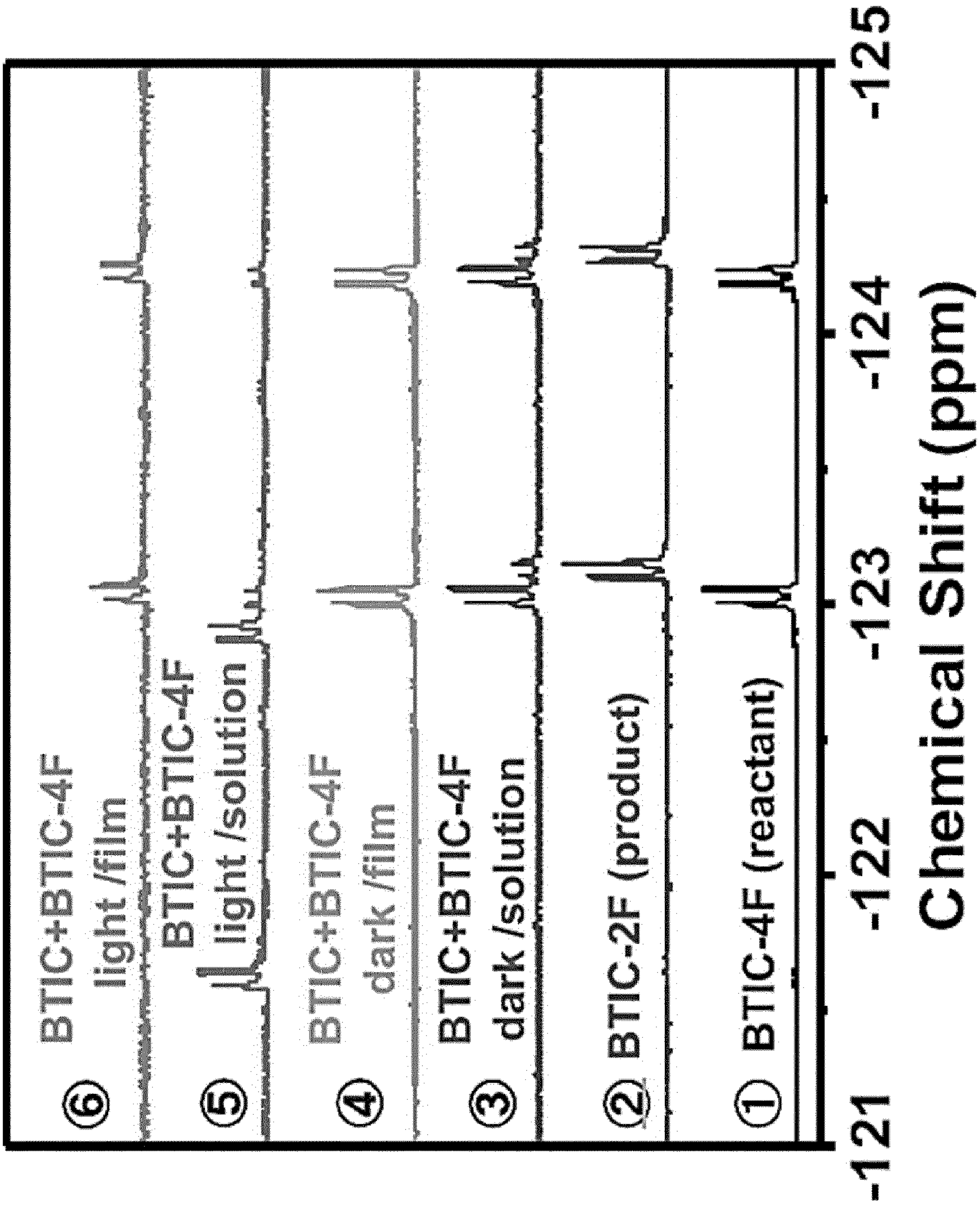
⑥ BTIC+BTIC-4F light /film
⑤ BTIC+BTIC-4F light /solution
④ BTIC+BTIC-4F dark /film
③ BTIC+BTIC-4F dark /solution
② BTIC-2F (product)
① BTIC-4F (reactant)
-121
-122
-123
-124
-125
Chemical Shift (ppm)

FIG. 5

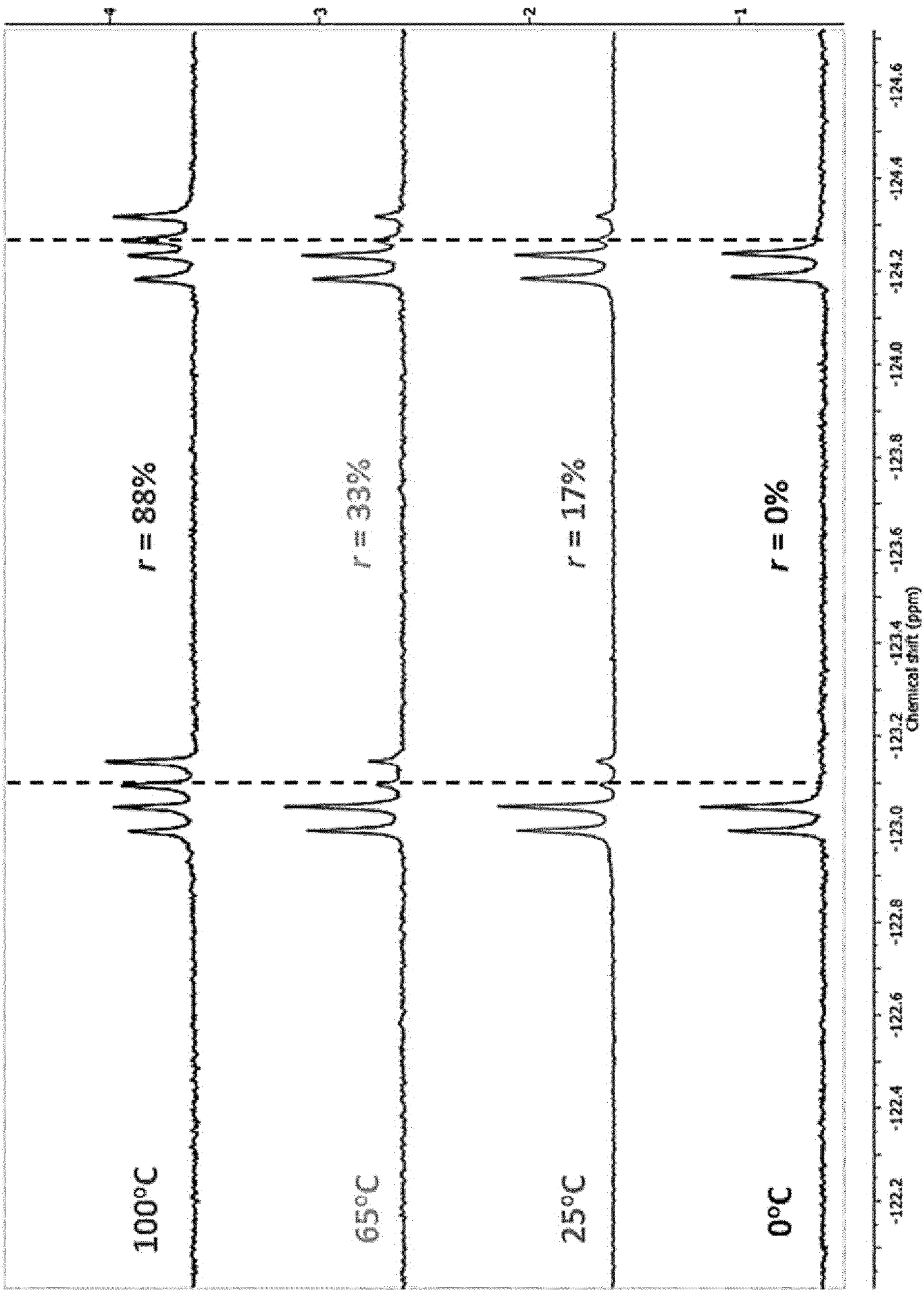
100°C
65°C
25°C
0°C
r = 88%
r = 33%
r = 17%
r = 0%
-122.2
-122.4
-122.6
-122.8
-123.0
-123.2
-123.4
-123.6
-123.8
-124.0
-124.2
-124.4
-124.6
Chemical shift (ppm)
1
2
3
4

FIG. 6

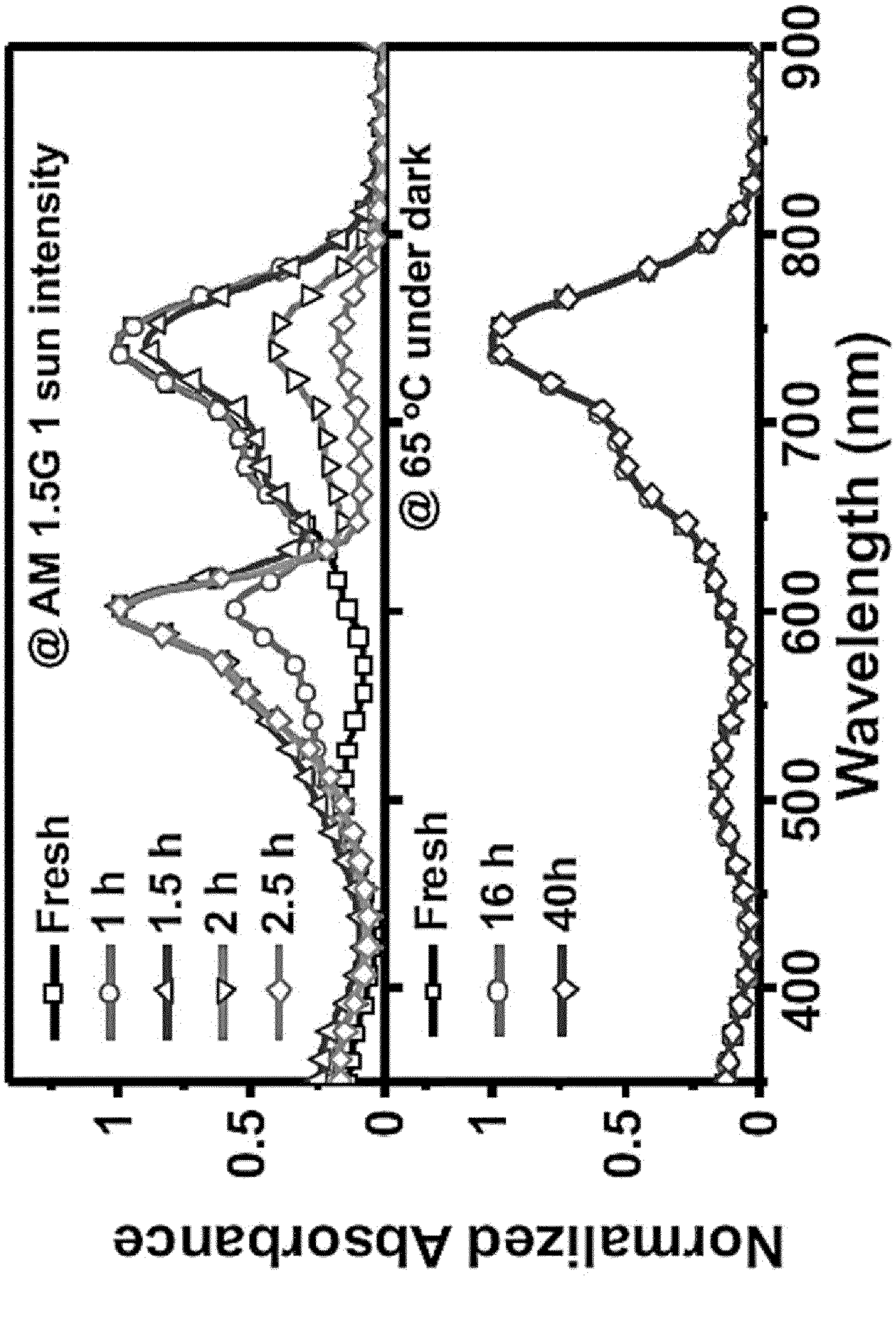

@ AM 1.5G 1 sun intensity
Fresh
1 h
1.5 h
2 h
2.5 h
@ 65 °C under dark
Fresh
16 h
40h
Normalized Absorbance
0
0.5
1
400
500
600
700
800
900
Wavelength (nm)


FIG. 7

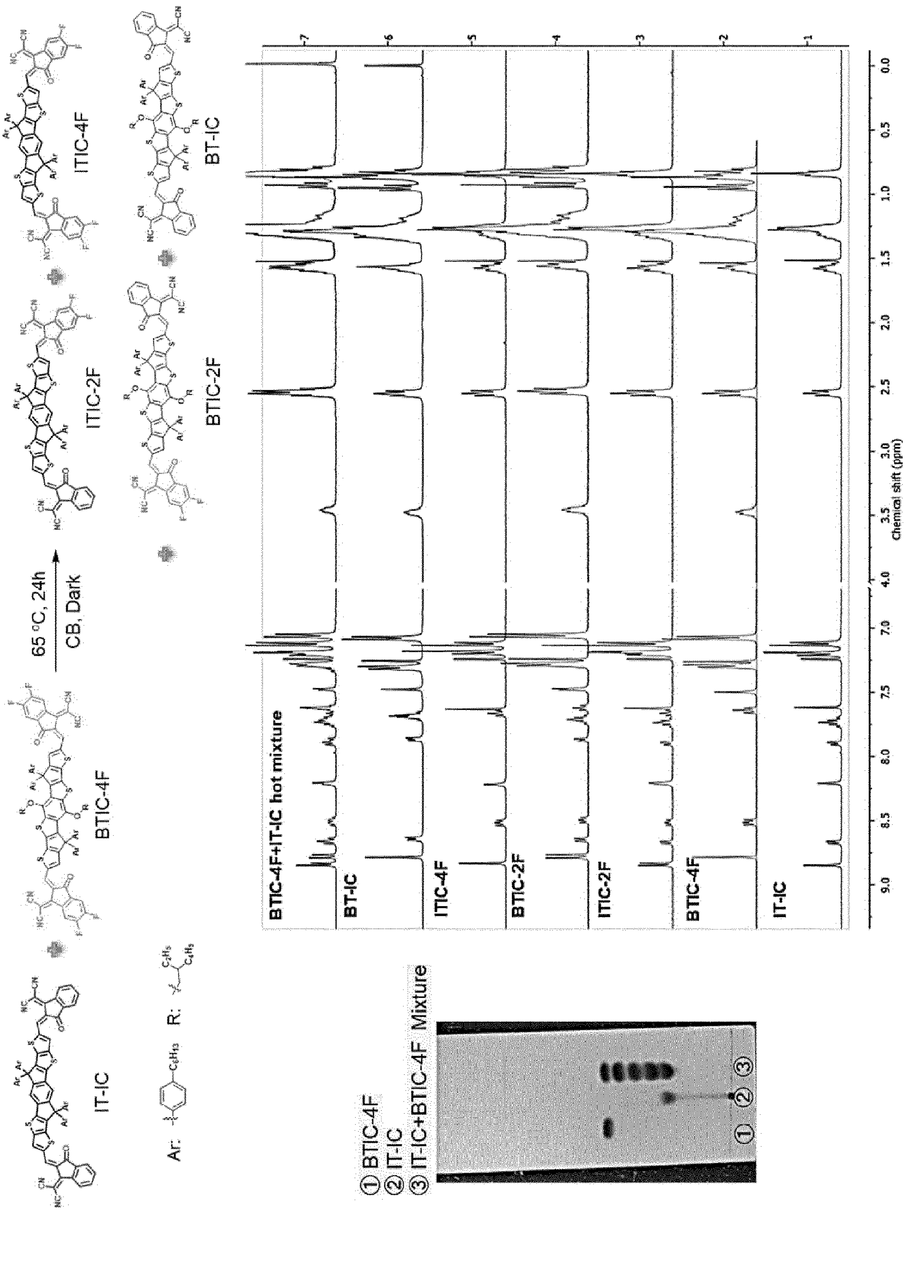
IT-IC
BTIC-4F
65 °C, 24h
CB, Dark
ITIC-2F
ITIC-4F
BTIC-2F
BT-IC
Ar:
R:
BTIC-4F+IT-IC hot mixture
BT-IC
ITIC-4F
BTIC-2F
ITIC-2F
BTIC-4F
IT-IC
Chemical shift (ppm)
① BTIC-4F
② IT-IC
③ IT-IC+BTIC-4F Mixture

FIG. 8

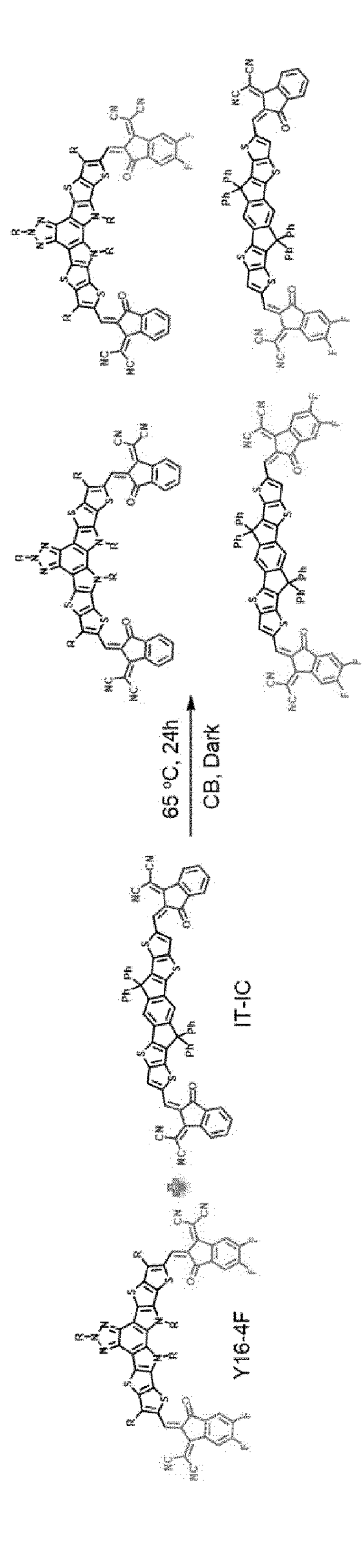
Y16-4F
IT-IC
65 °C, 24h
CB, Dark

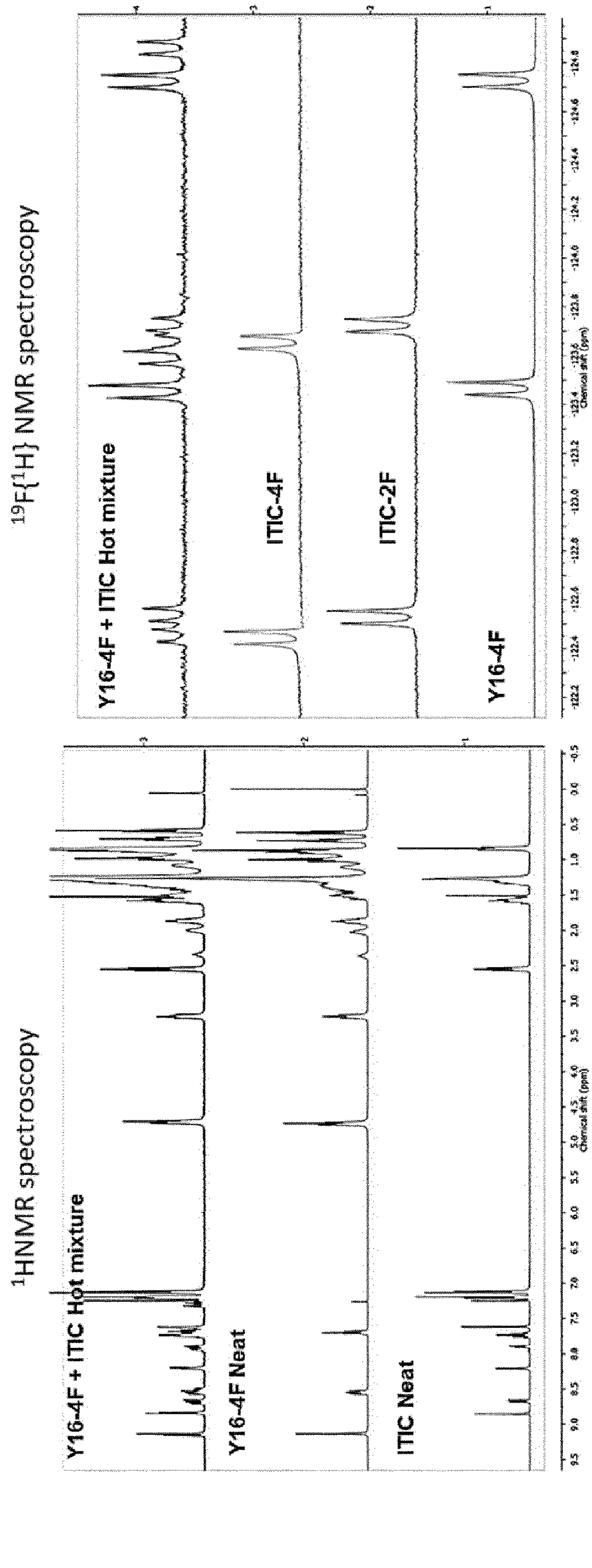
$^1$HNMR spectroscopy
Y16-4F + ITIC Hot mixture
Y16-4F Neat
ITIC Neat
Chemical shift (ppm)
$^{19}$F{$^1$H} NMR spectroscopy
Y16-4F + ITIC Hot mixture
ITIC-4F
ITIC-2F
Y16-4F
Chemical shift (ppm)

FIG. 9

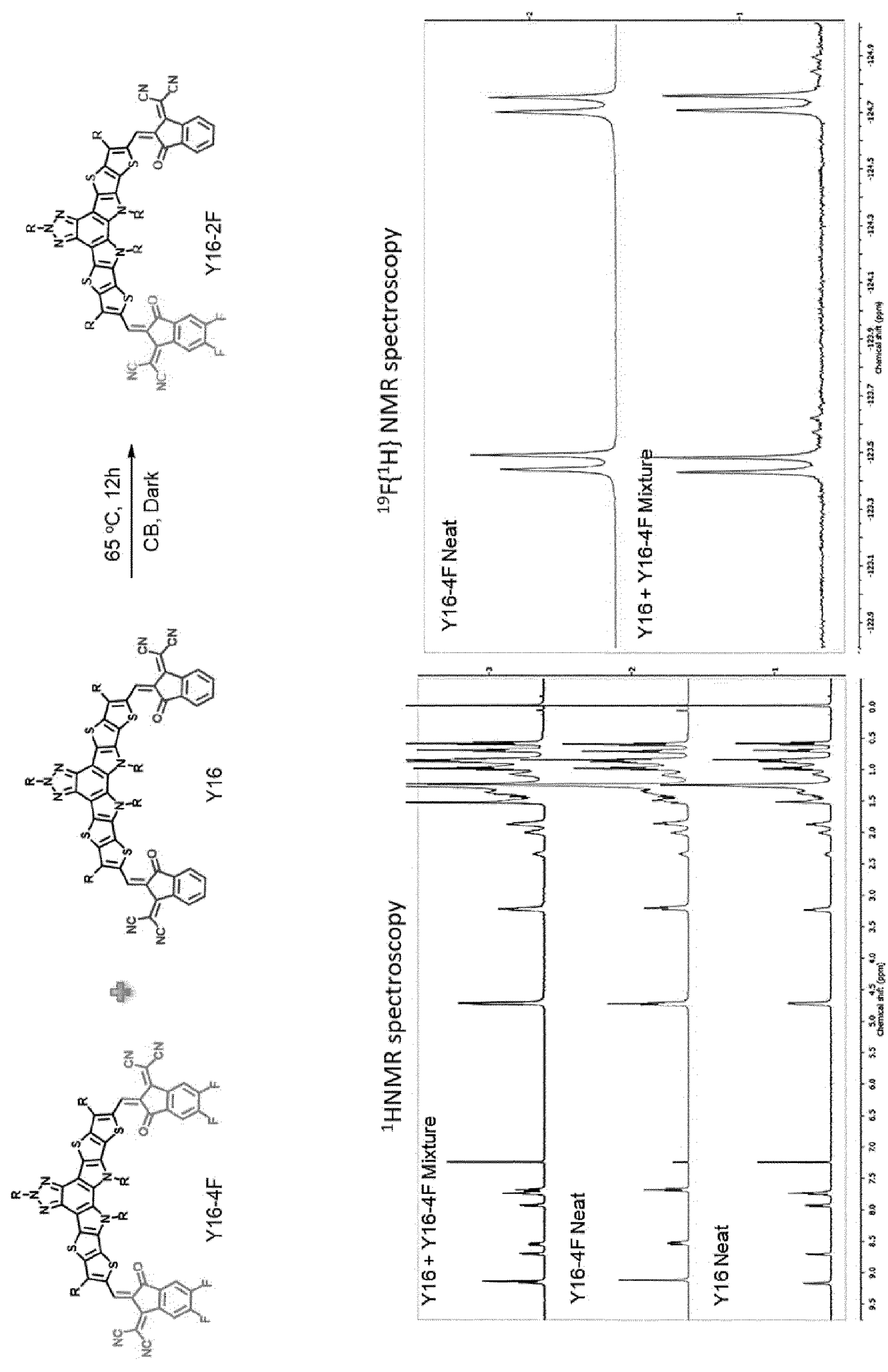
Y16-4F
Y16
65 °C, 12h
CB, Dark
Y16-2F
$^{1}$HNMR spectroscopy
Y16 + Y16-4F Mixture
Y16-4F Neat
Y16 Neat
Chemical shift (ppm)
$^{19}$F{$^{1}$H} NMR spectroscopy
Y16-4F Neat
Y16 + Y16-4F Mixture
Chemical shift (ppm)

FIG. 10

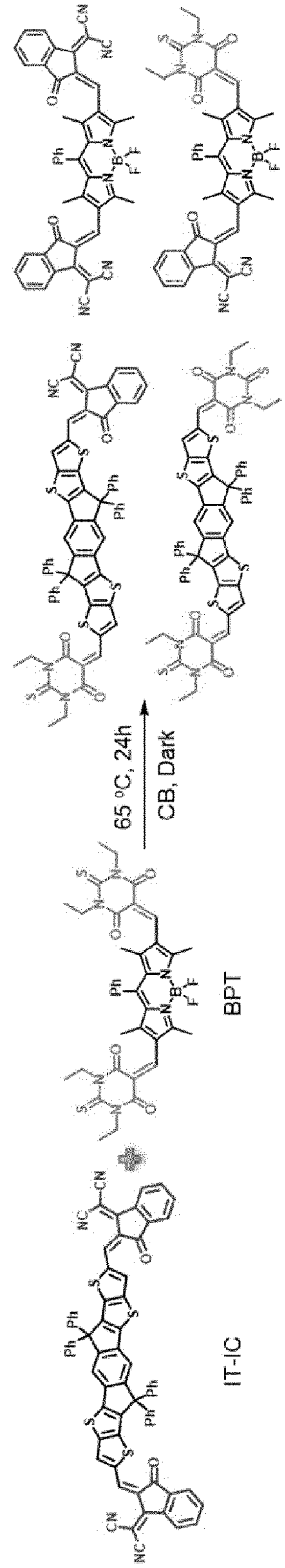


IT-IC

BPT

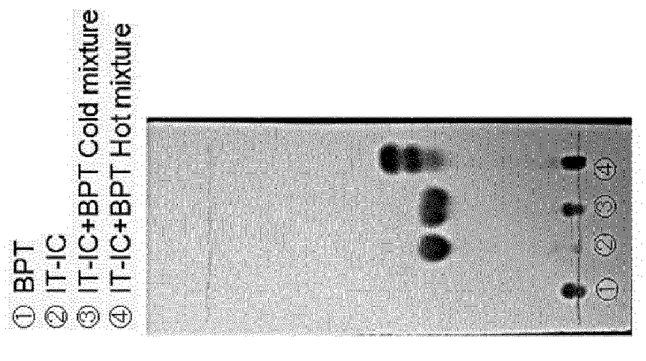


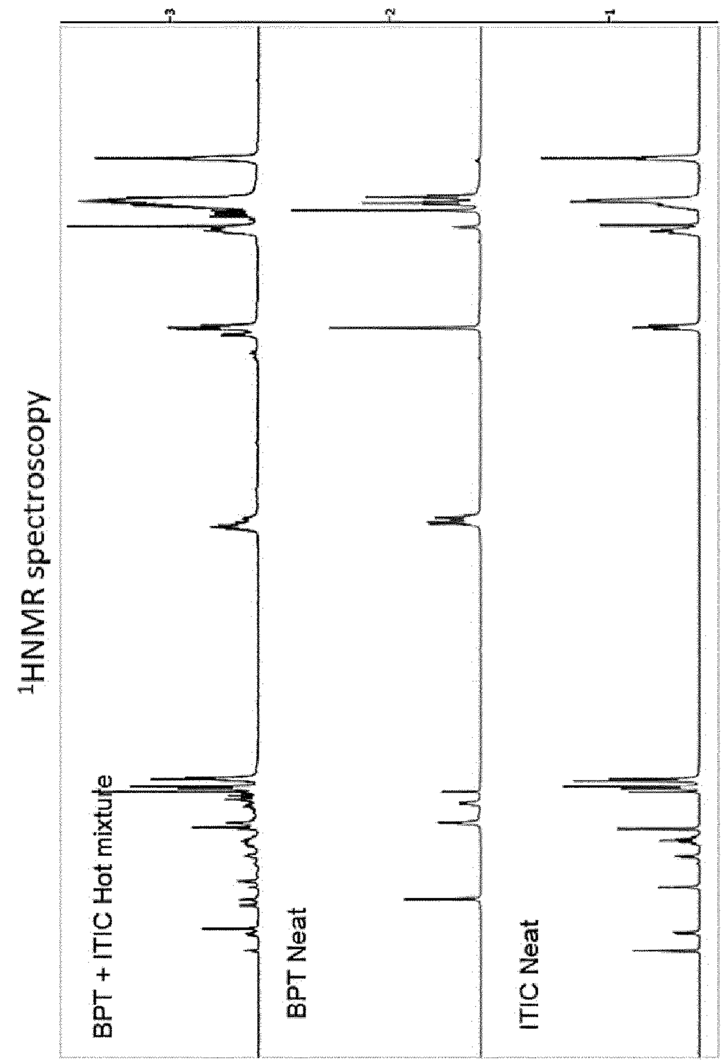


**FIG. 11**

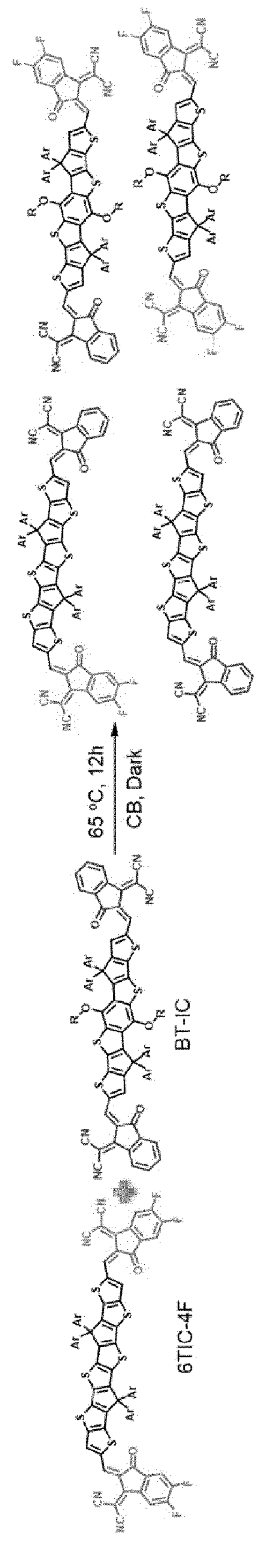


6TIC-4F

BT-IC

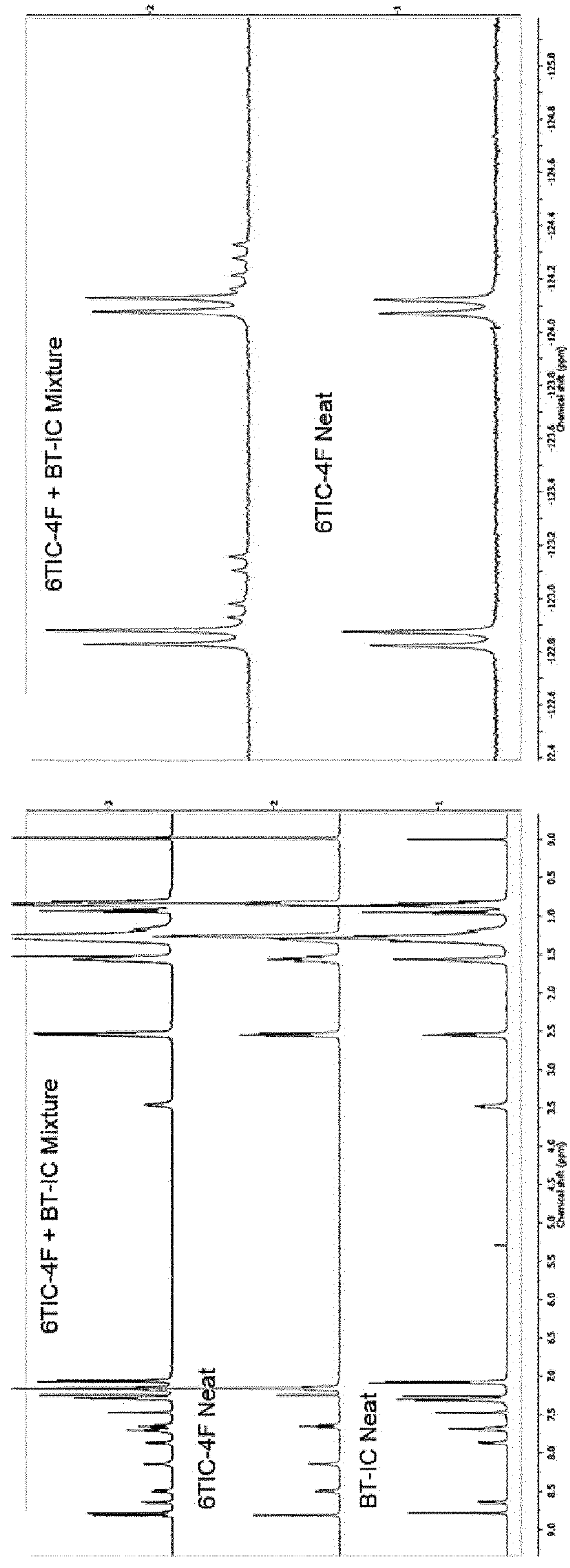


FIG. 12

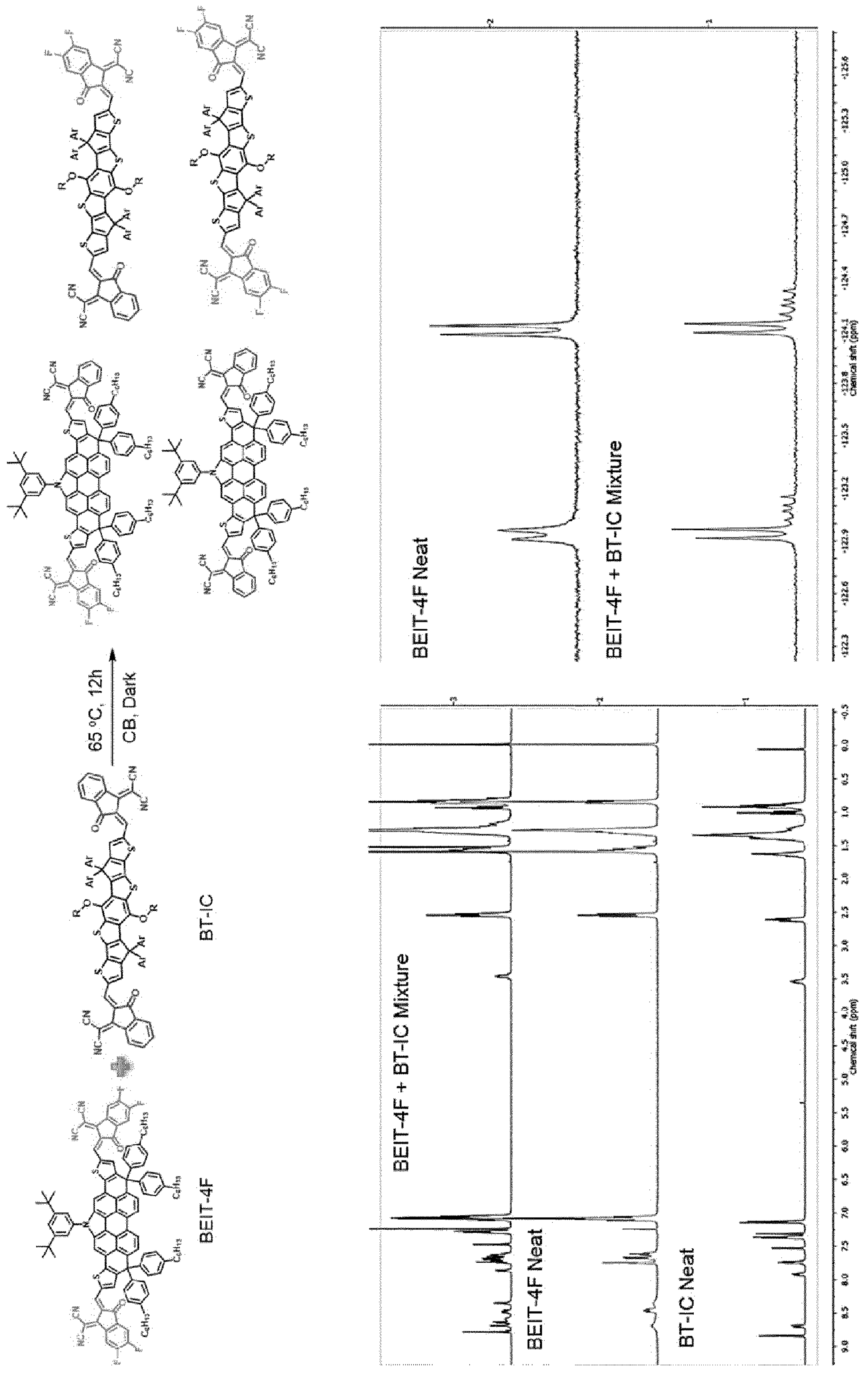
BEIT-4F
BT-IC
65 °C, 12h
CB, Dark
BEIT-4F + BT-IC Mixture
BEIT-4F Neat
BT-IC Neat
Chemical shift (ppm)
BEIT-4F Neat
BEIT-4F + BT-IC Mixture
Chemical shift (ppm)

FIG. 13

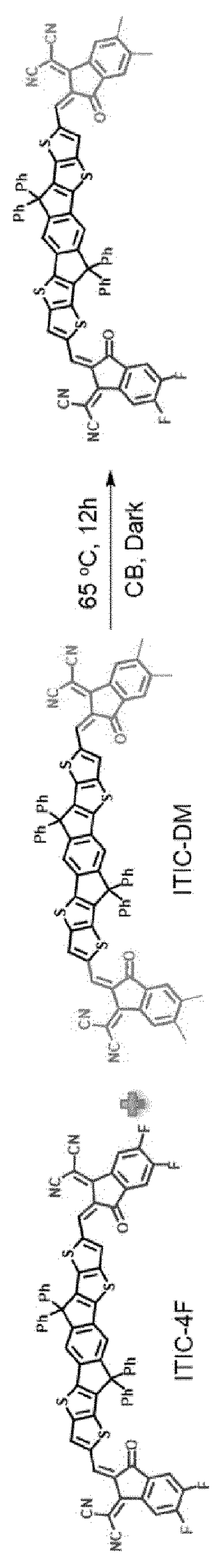


ITIC-4F

ITIC-DM

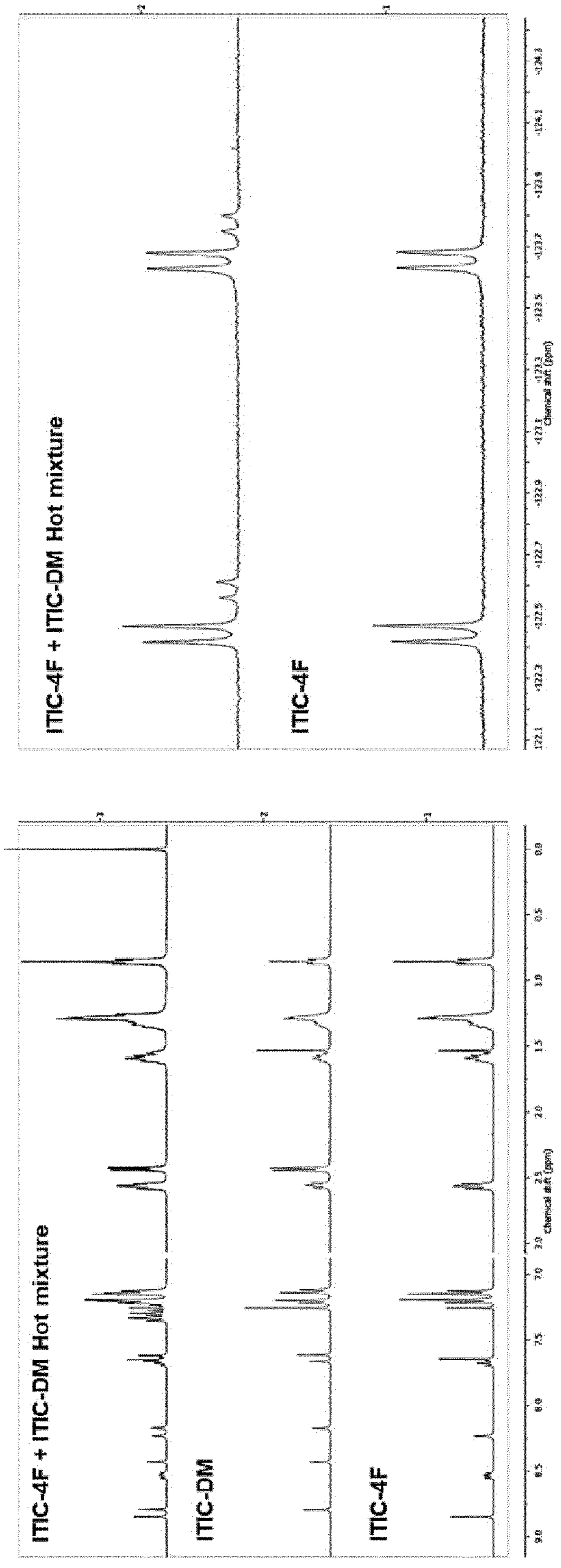


FIG. 14

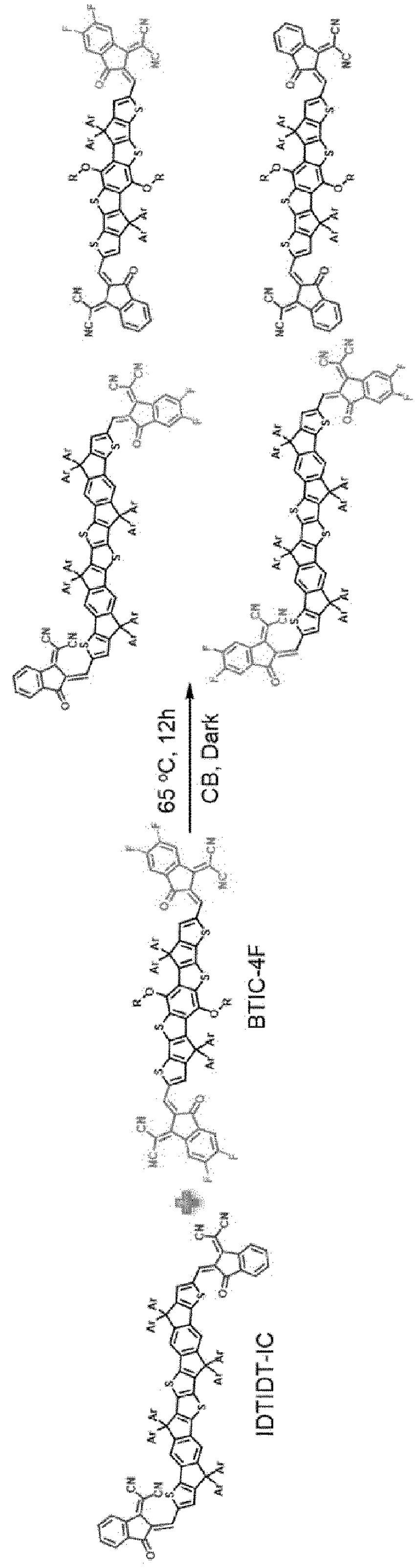
IDTIDT-IC
BTIC-4F
65 °C, 12h
CB, Dark

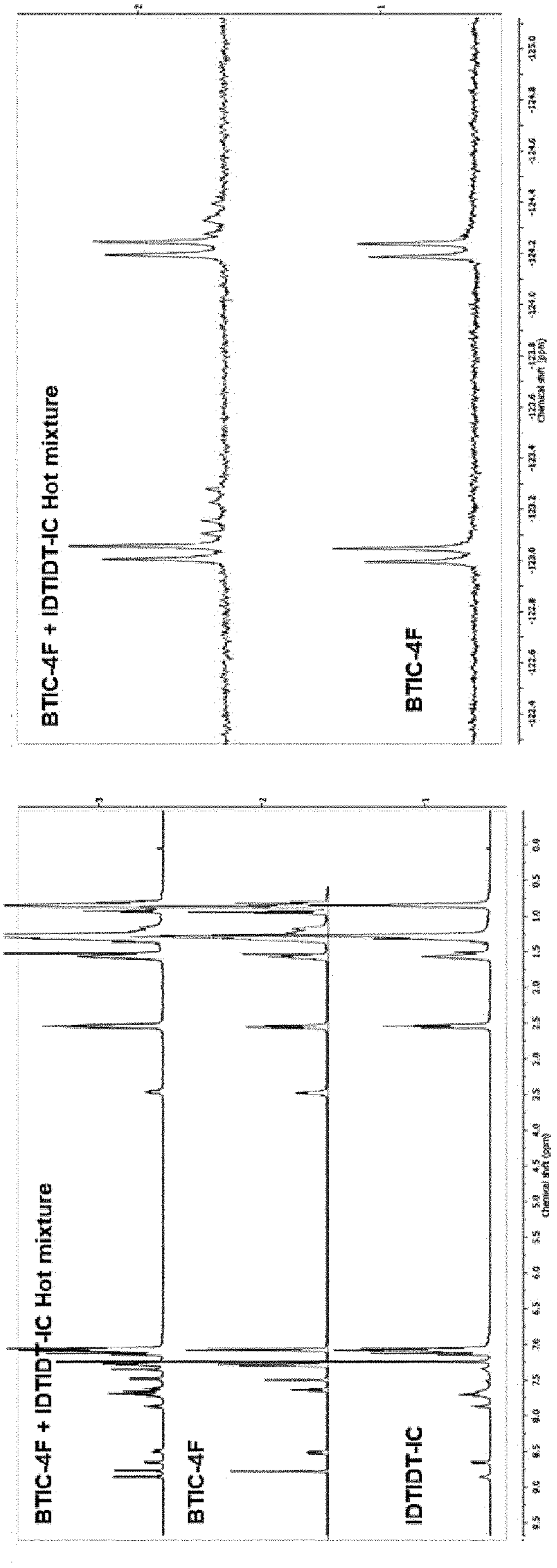
BTIC-4F + IDTIDT-IC Hot mixture
BTIC-4F
IDTIDT-IC
Chemical shift (ppm)
BTIC-4F + IDTIDT-IC Hot mixture
BTIC-4F
Chemical shift (ppm)

FIG. 15

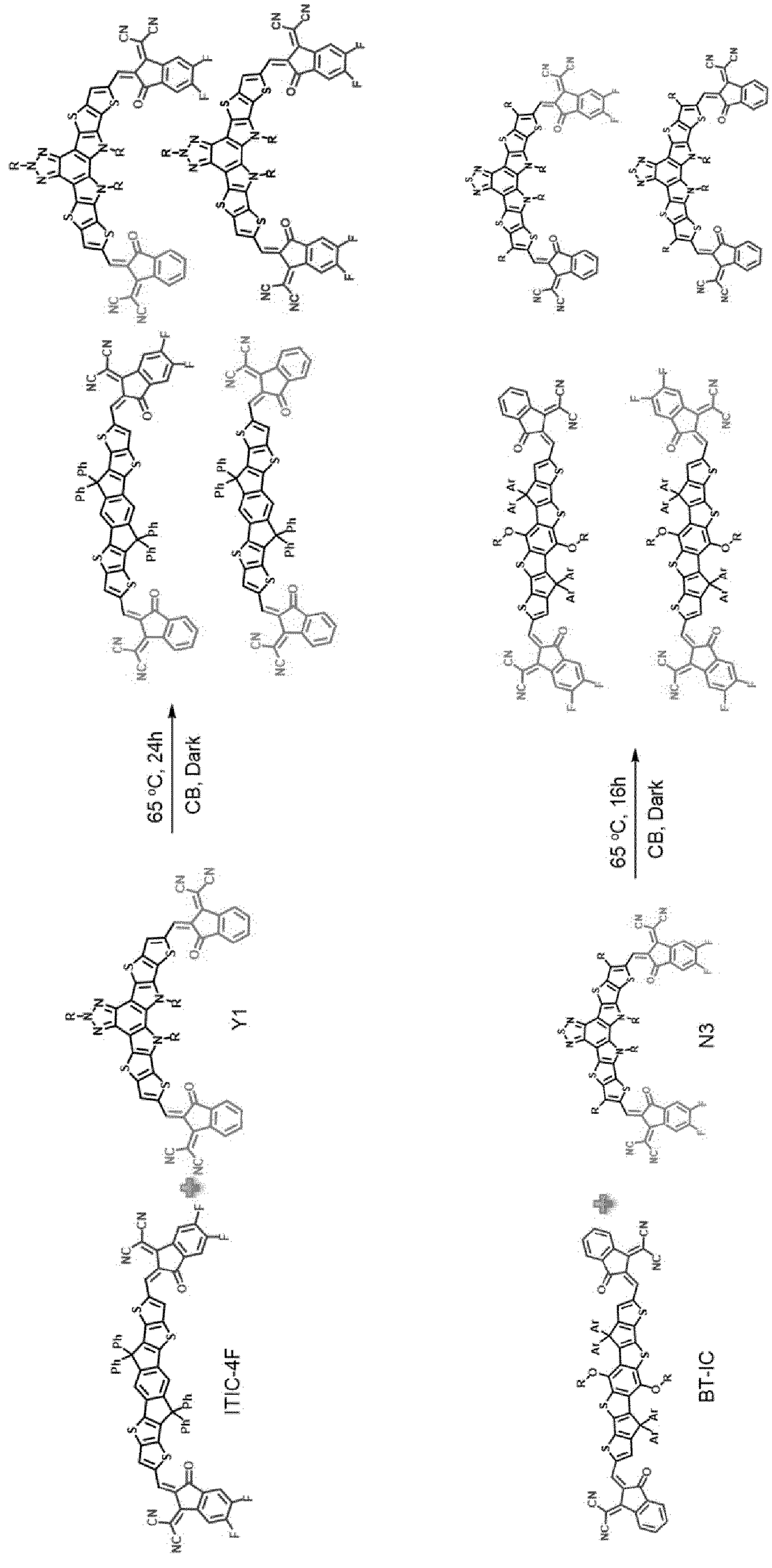


ITIC-4F

Y1

BT-IC

N3

FIG. 16

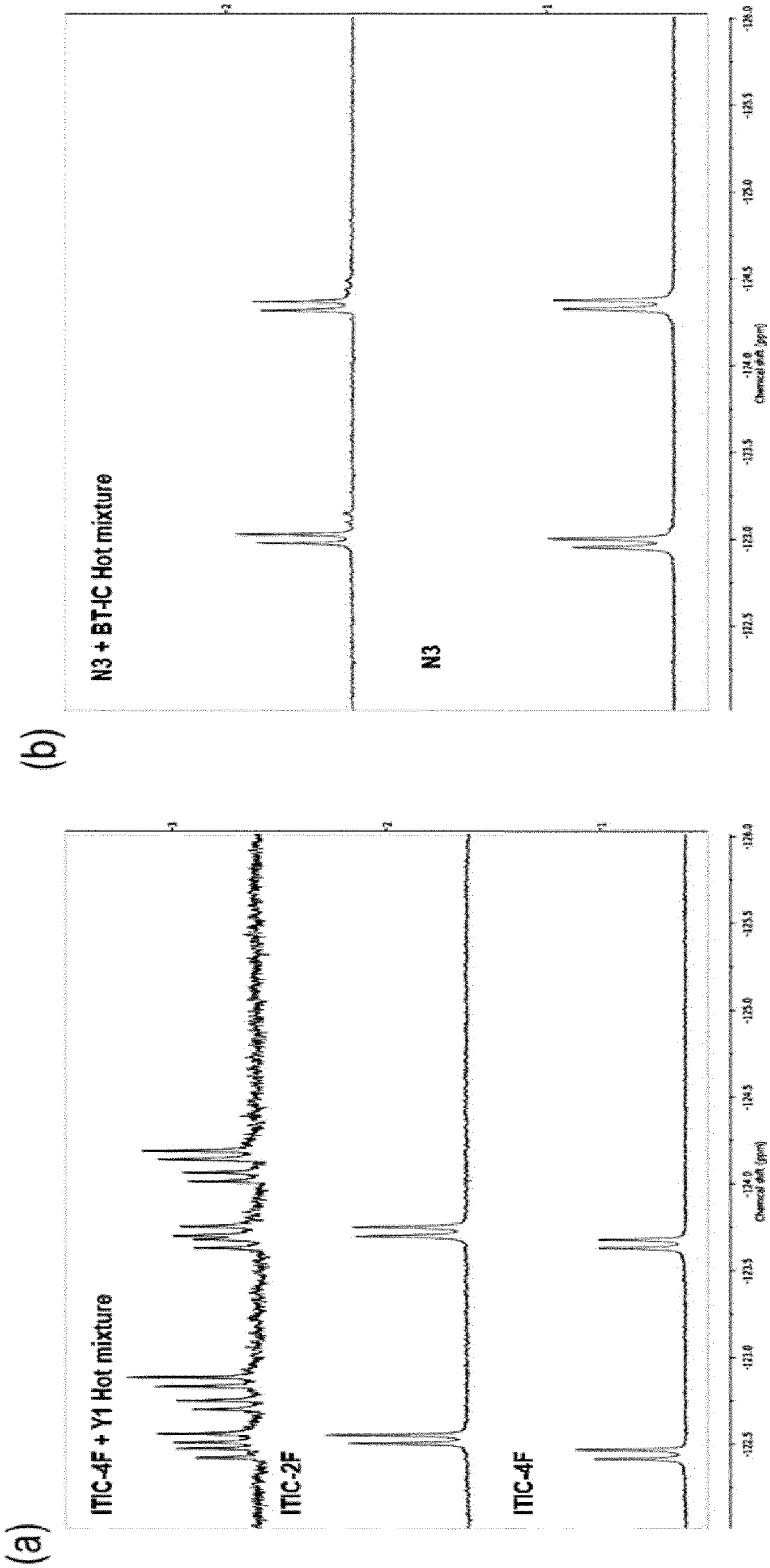
(a)
ITIC-4F + Y1 Hot mixture
ITIC-2F
ITIC-4F
(b)
N3 + BT-IC Hot mixture
N3
Chemical shift (ppm)

FIG. 17

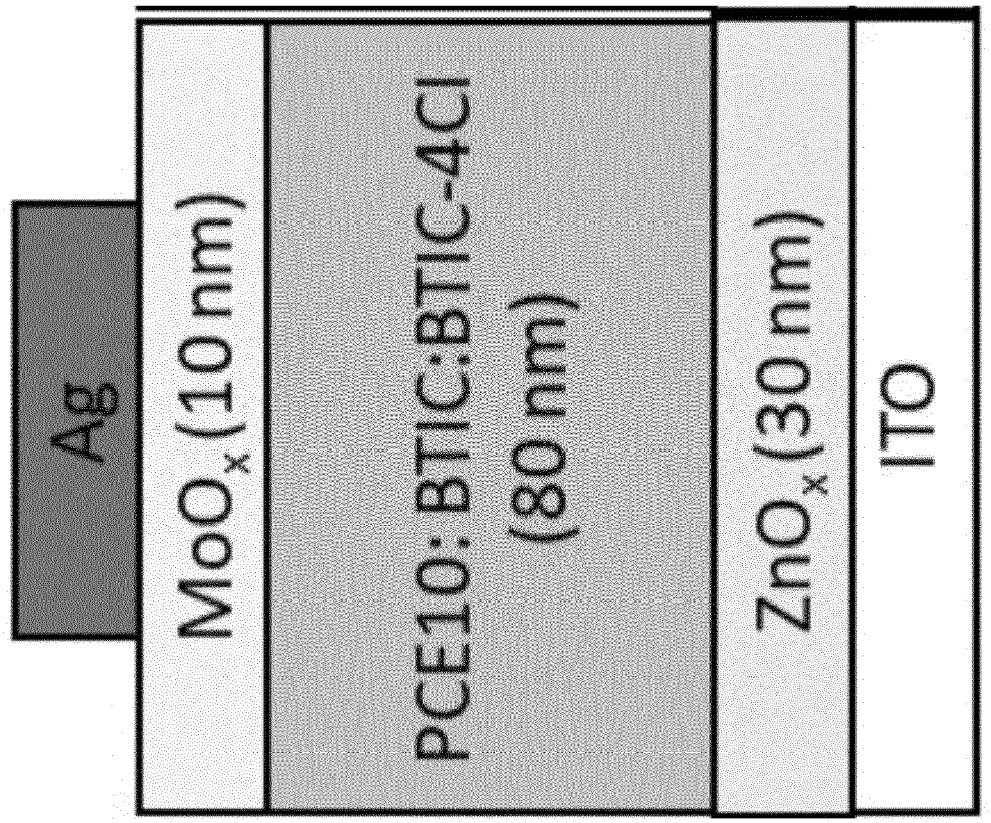


FIG. 18

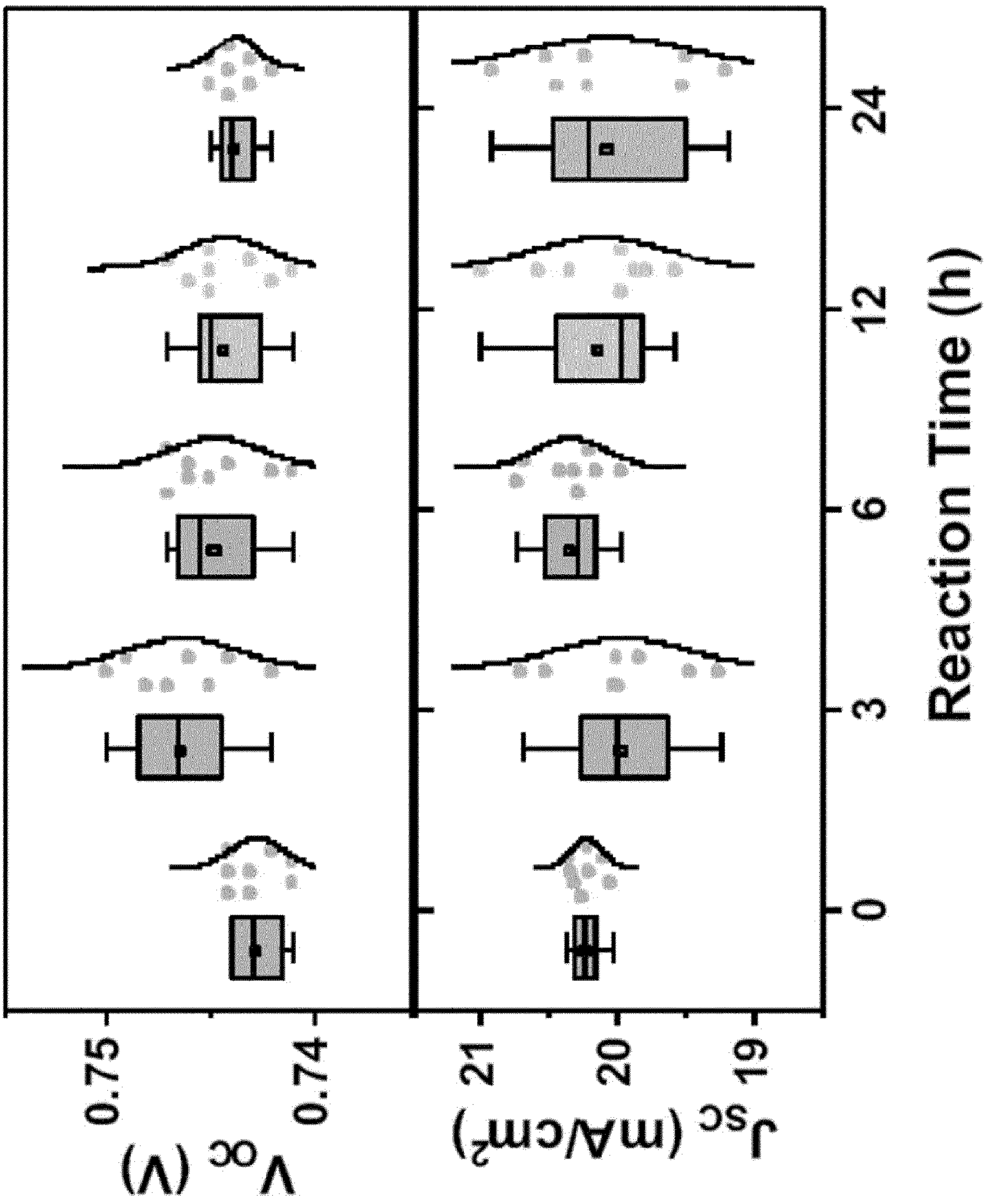


FIG. 19

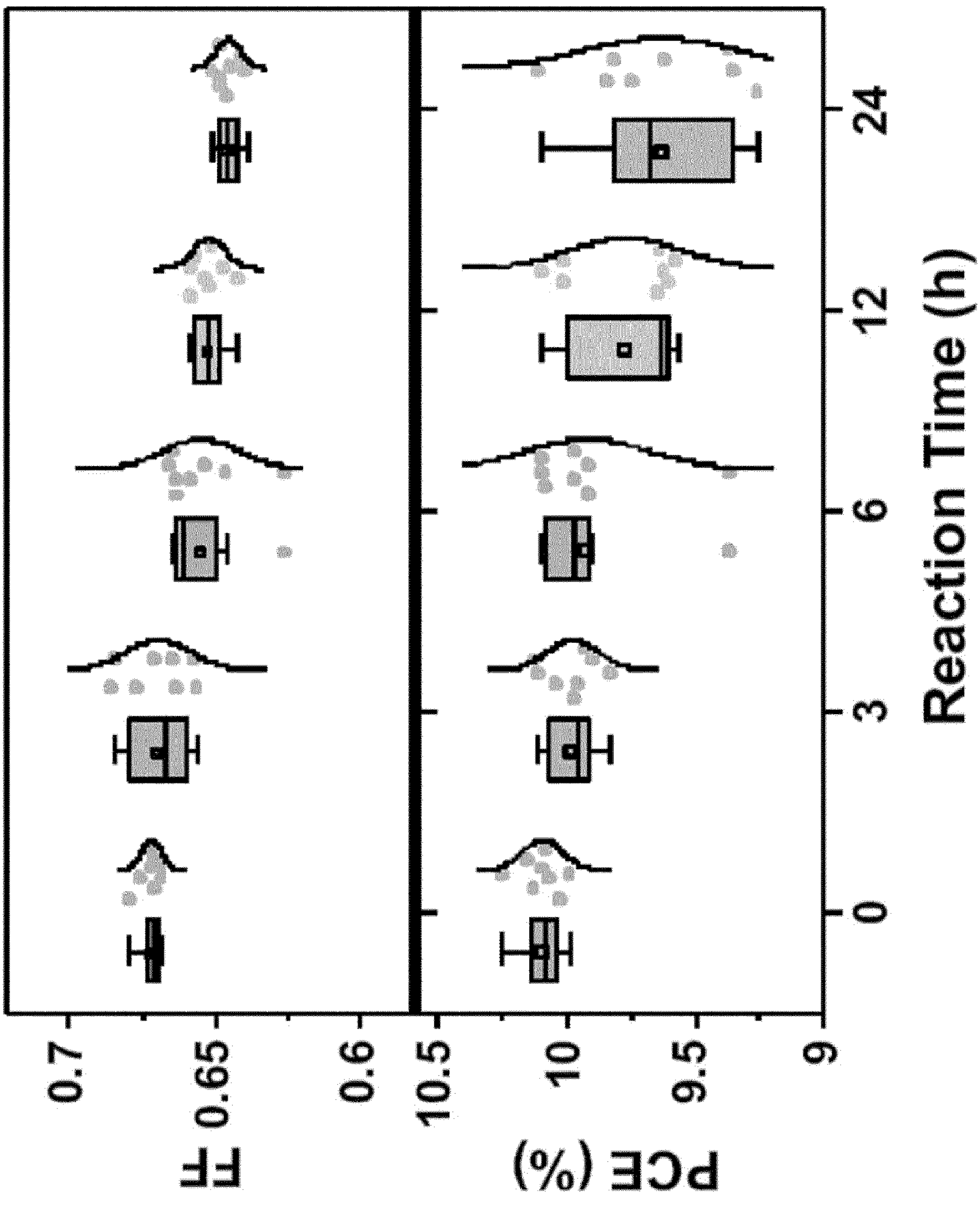


FIG. 20

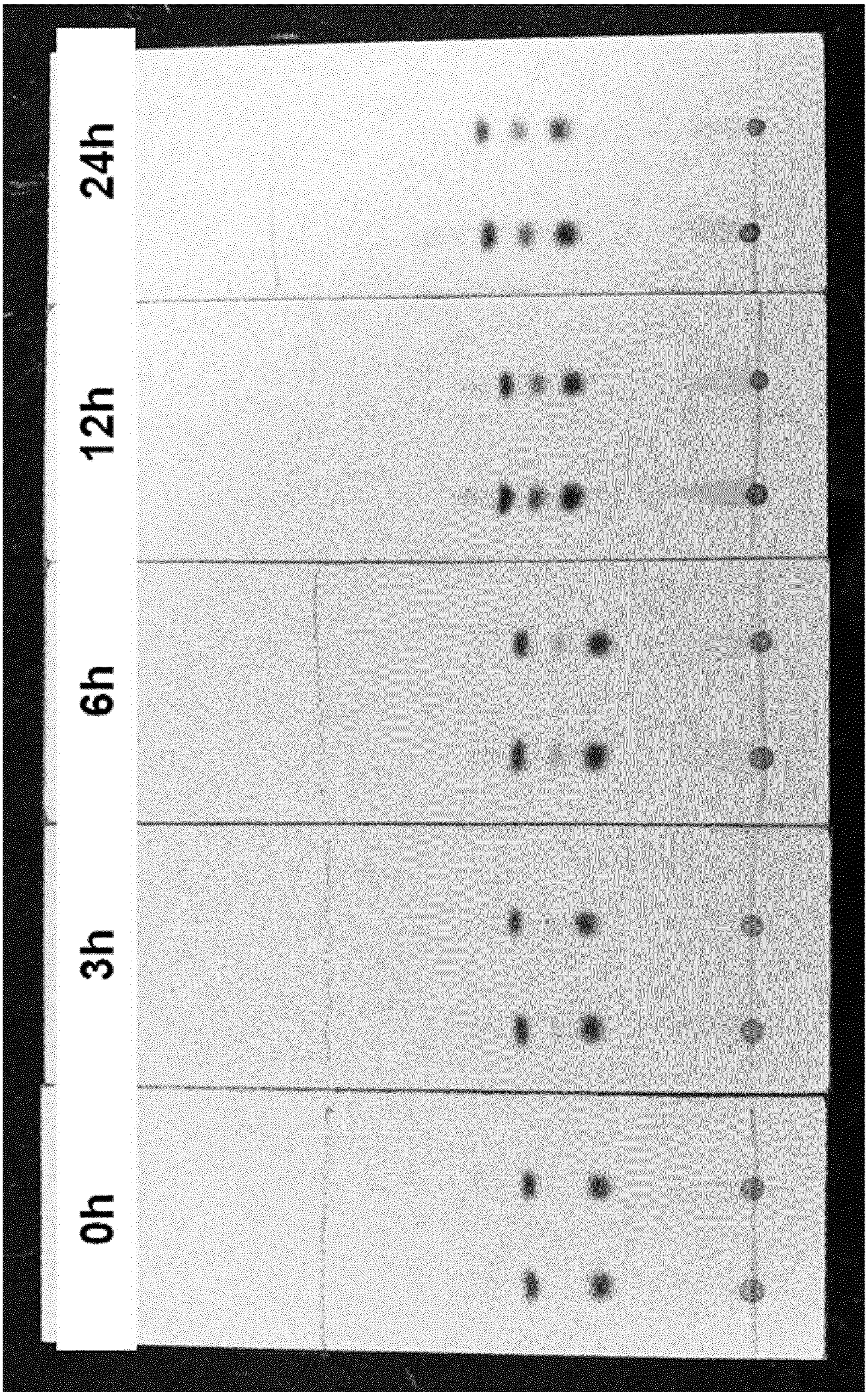


FIG. 21

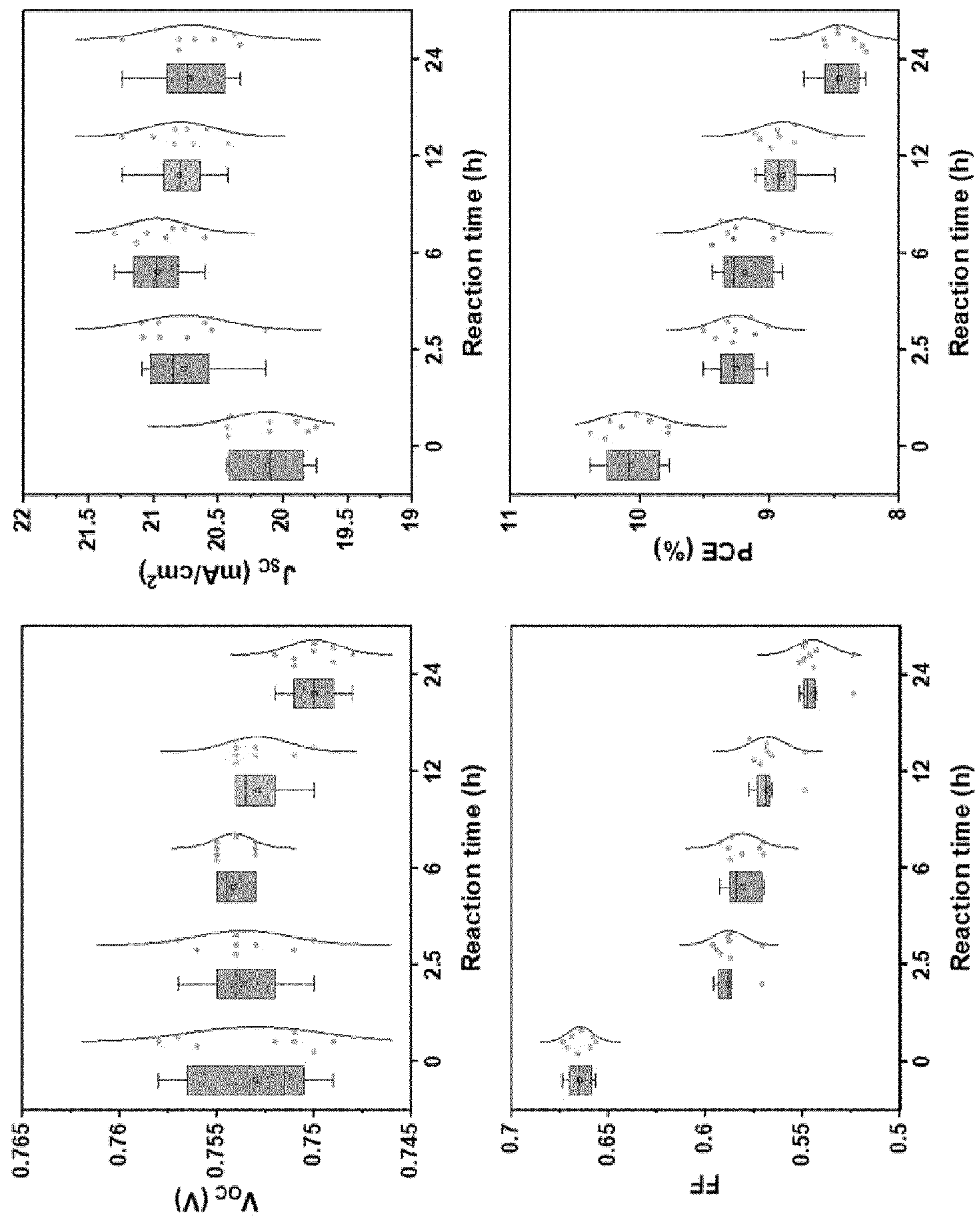

Voc (V)
0.765
0.76
0.755
0.75
0.745
Jsc (mA/cm2)
22
21.5
21
20.5
20
19.5
19
FF
0.7
0.65
0.6
0.55
0.5
PCE (%)
11
10
9
8
0
2.5
6
12
24
Reaction time (h)


FIG. 22

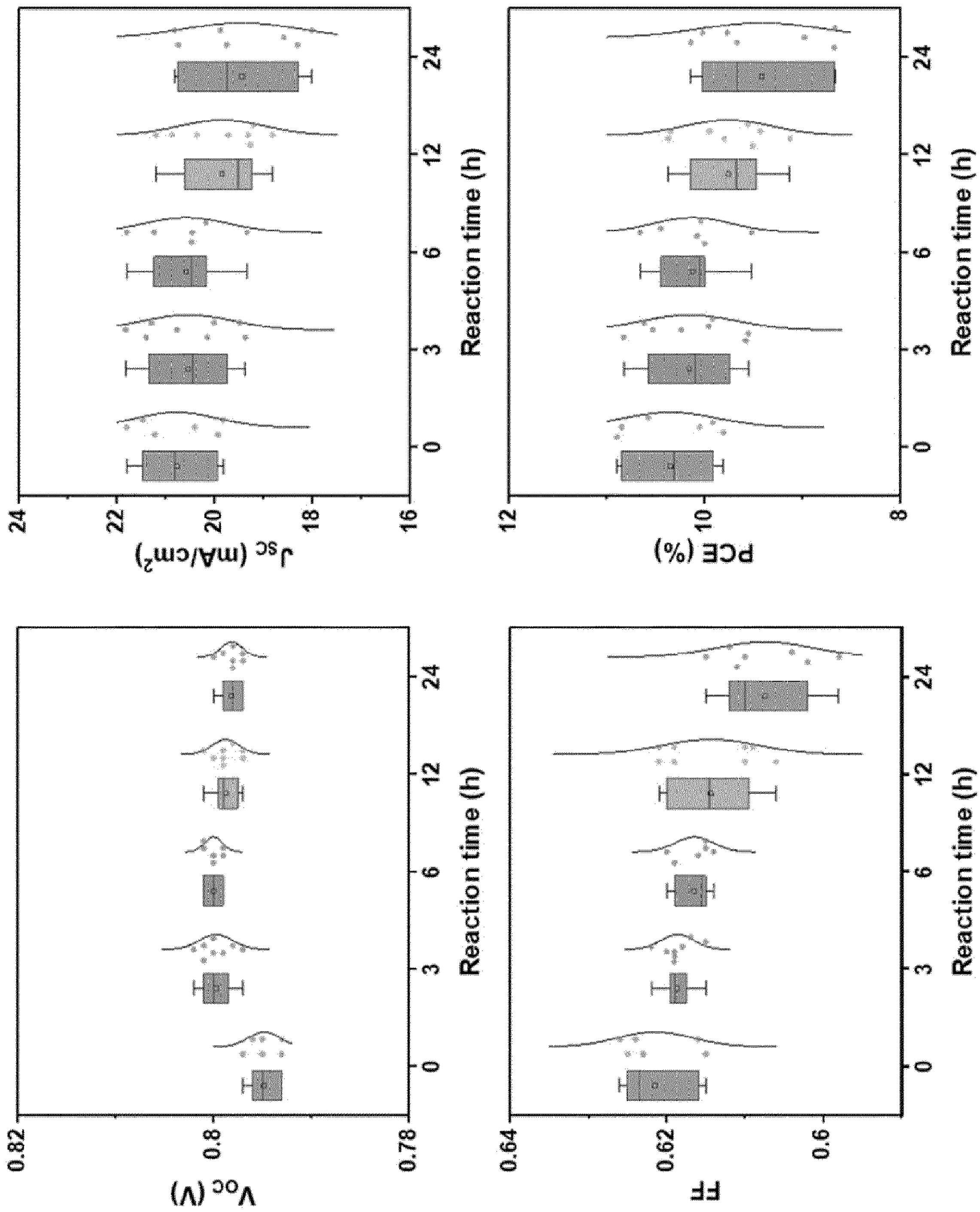

$V_{oc}$ (V)
$J_{sc}$ (mA/cm$^2$)
FF
PCE (%)
Reaction time (h)
Reaction time (h)
Reaction time (h)
Reaction time (h)


FIG. 23

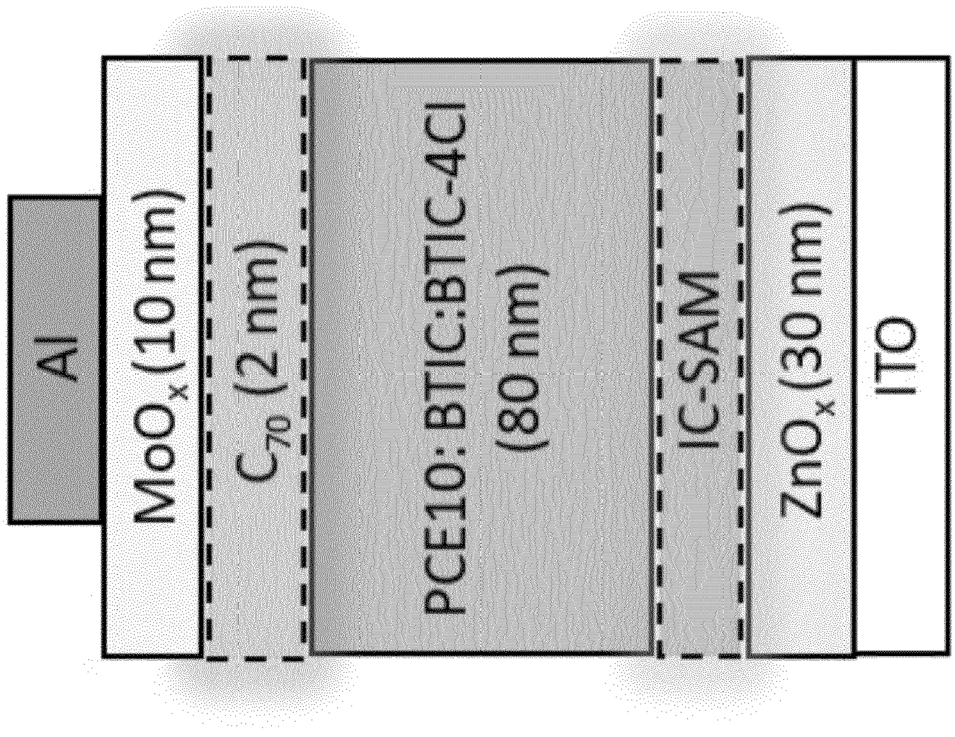
Al
$MoO_x$ (10 nm)
$C_{70}$ (2 nm)
PCE10: BTIC:BTIC-4Cl
(80 nm)
IC-SAM
$ZnO_x$ (30 nm)
ITO

FIG. 24

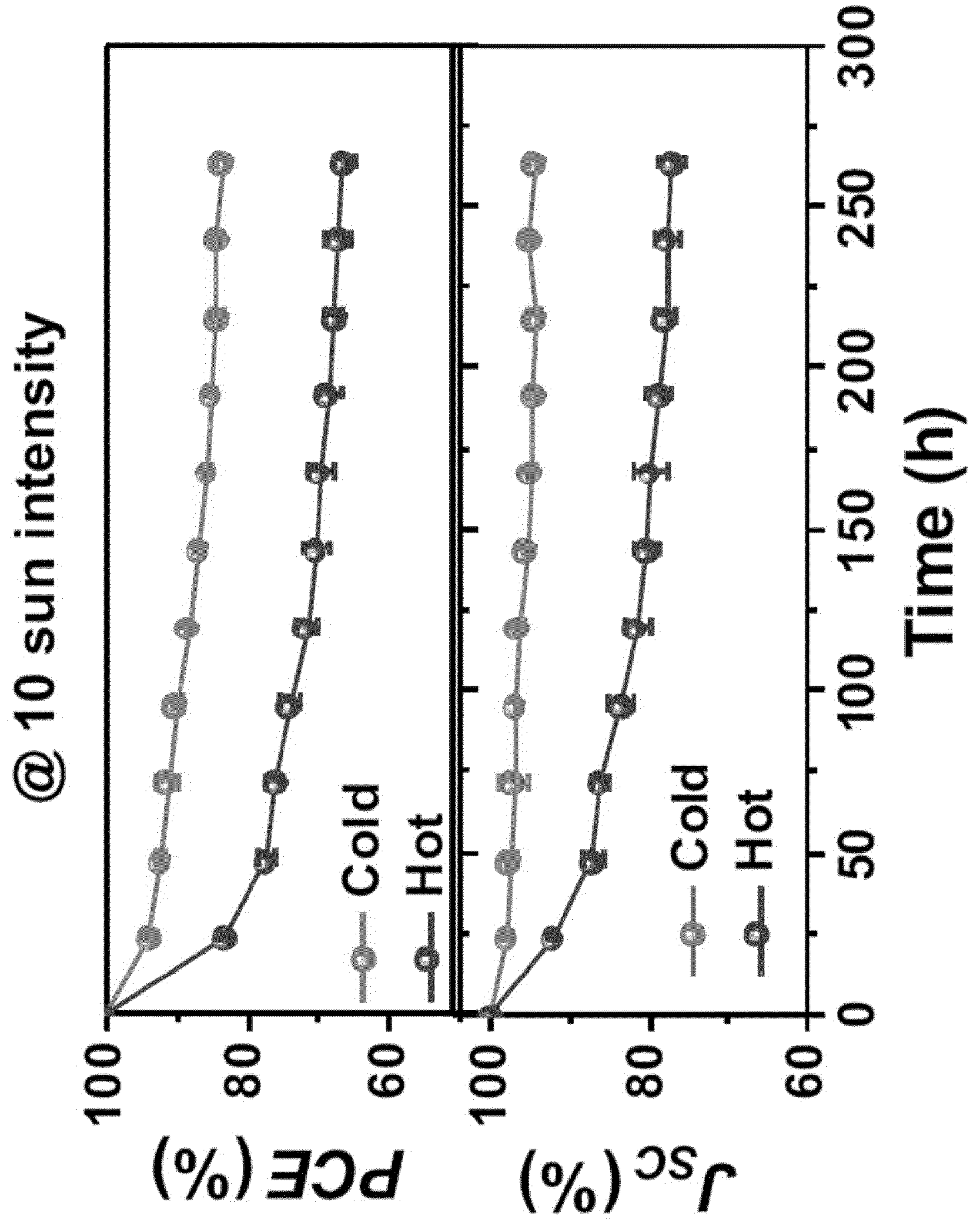
@ 10 sun intensity
PCE (%)
$J_{SC}$ (%)
Cold
Hot
Cold
Hot
100
80
60
100
80
60
0
50
100
150
200
250
300
Time (h)

FIG. 25

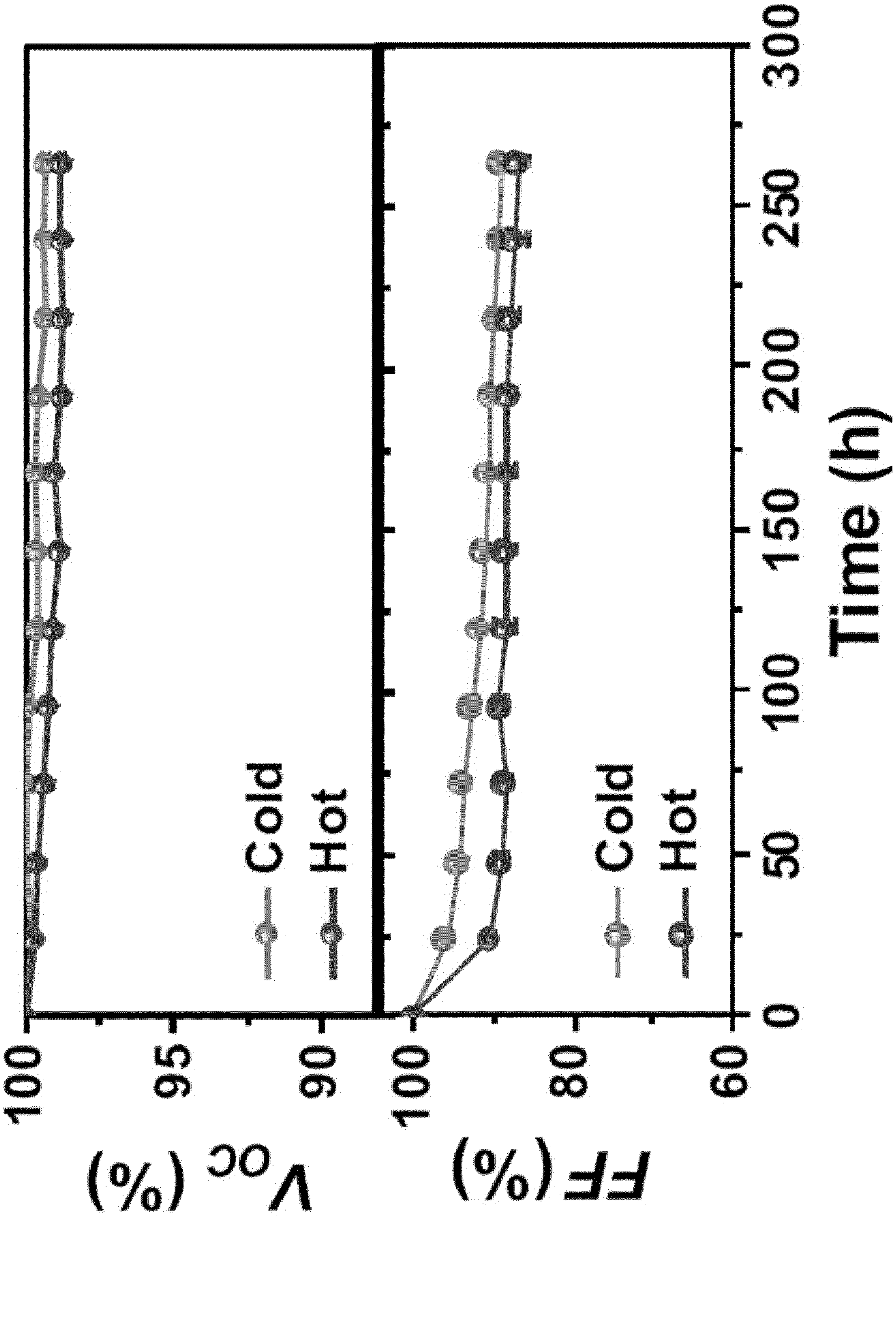

Voc (%)
100
95
90
FF (%)
100
80
60
Cold
Hot
Cold
Hot
0
50
100
150
200
250
300
Time (h)


FIG. 26

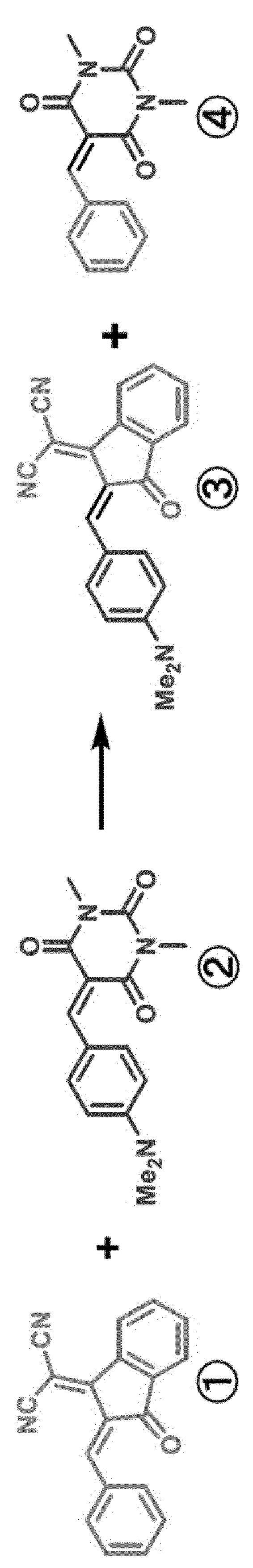

① + ②

③ + ④

FIG. 27

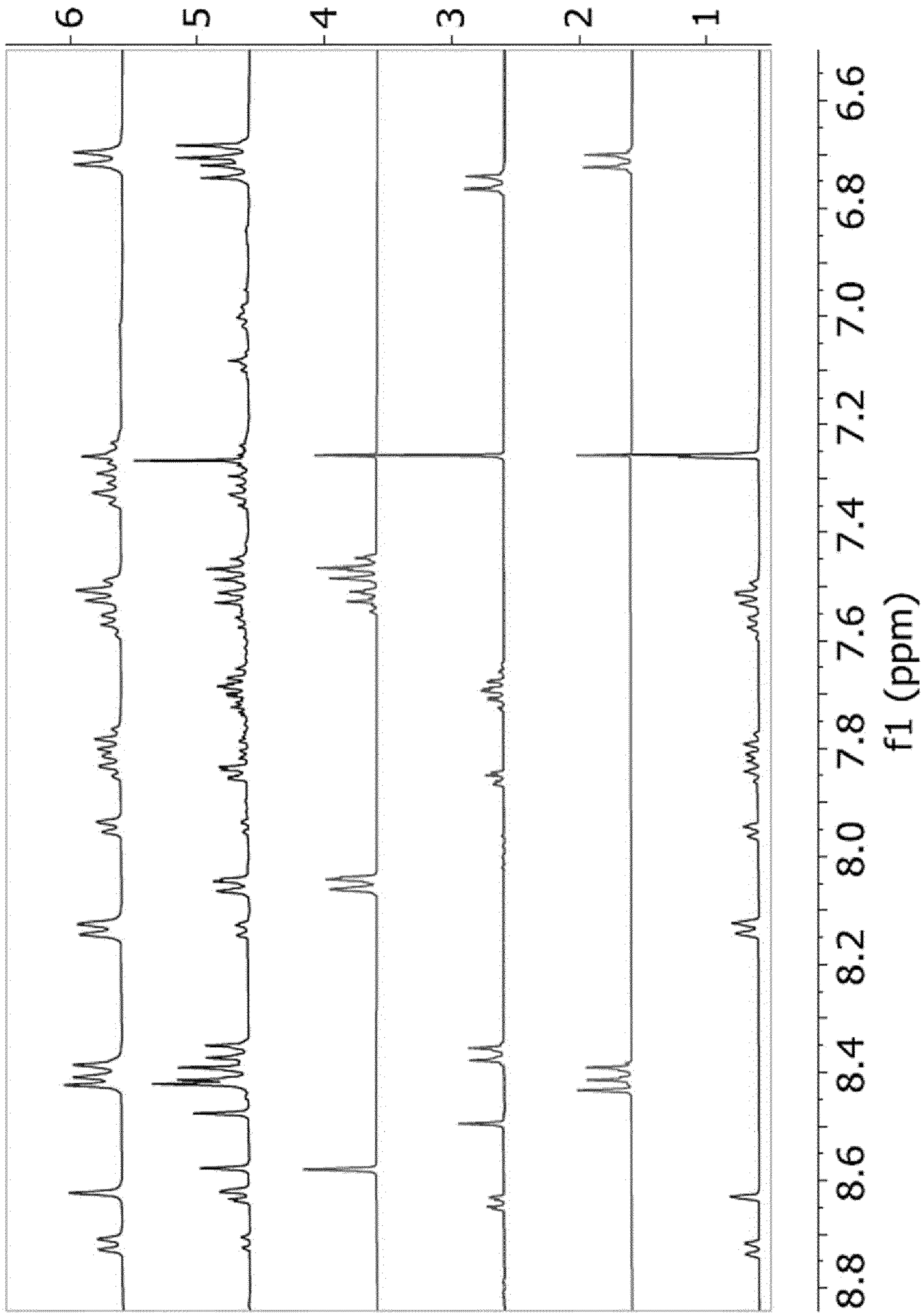


FIG. 28

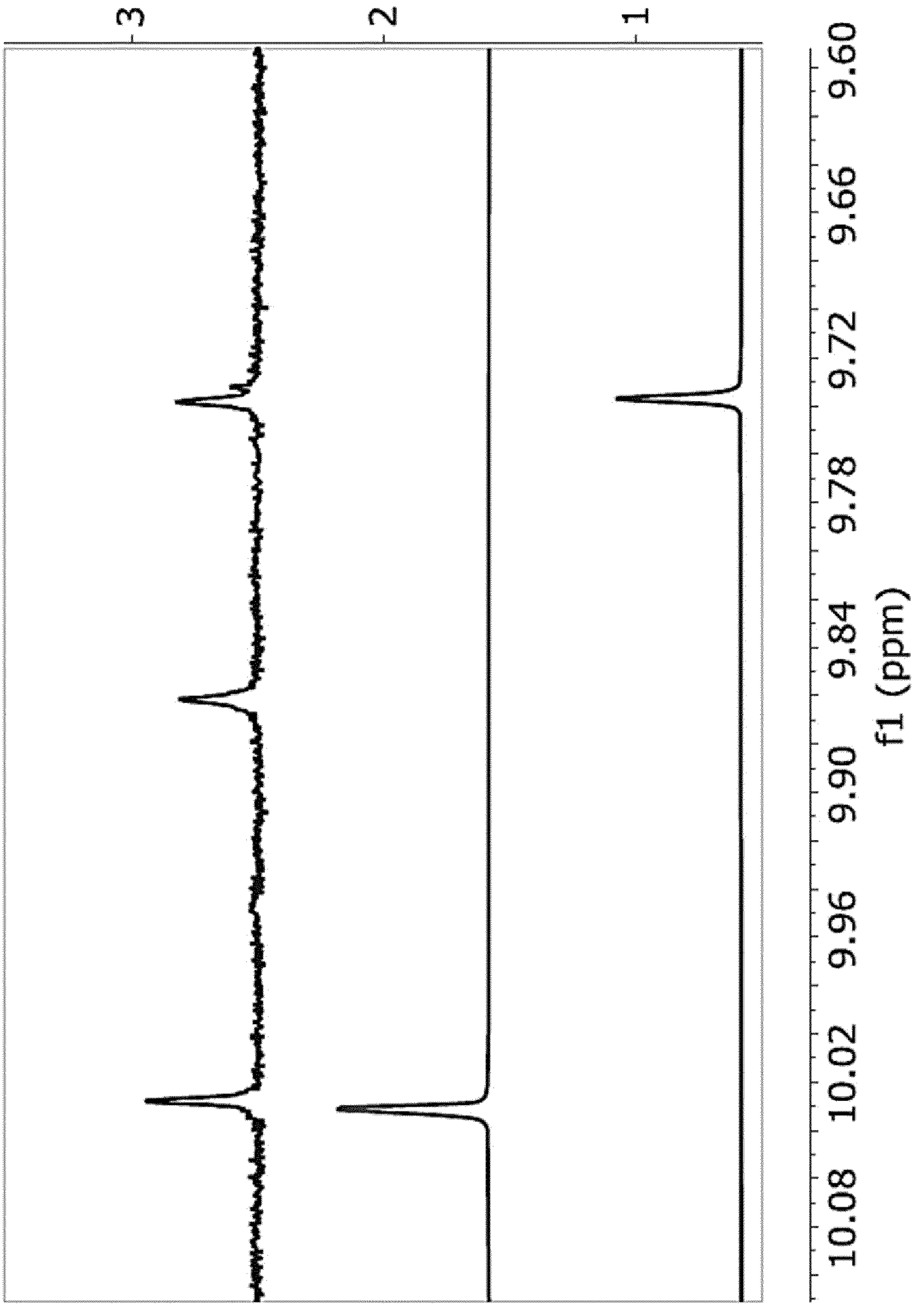


FIG. 29

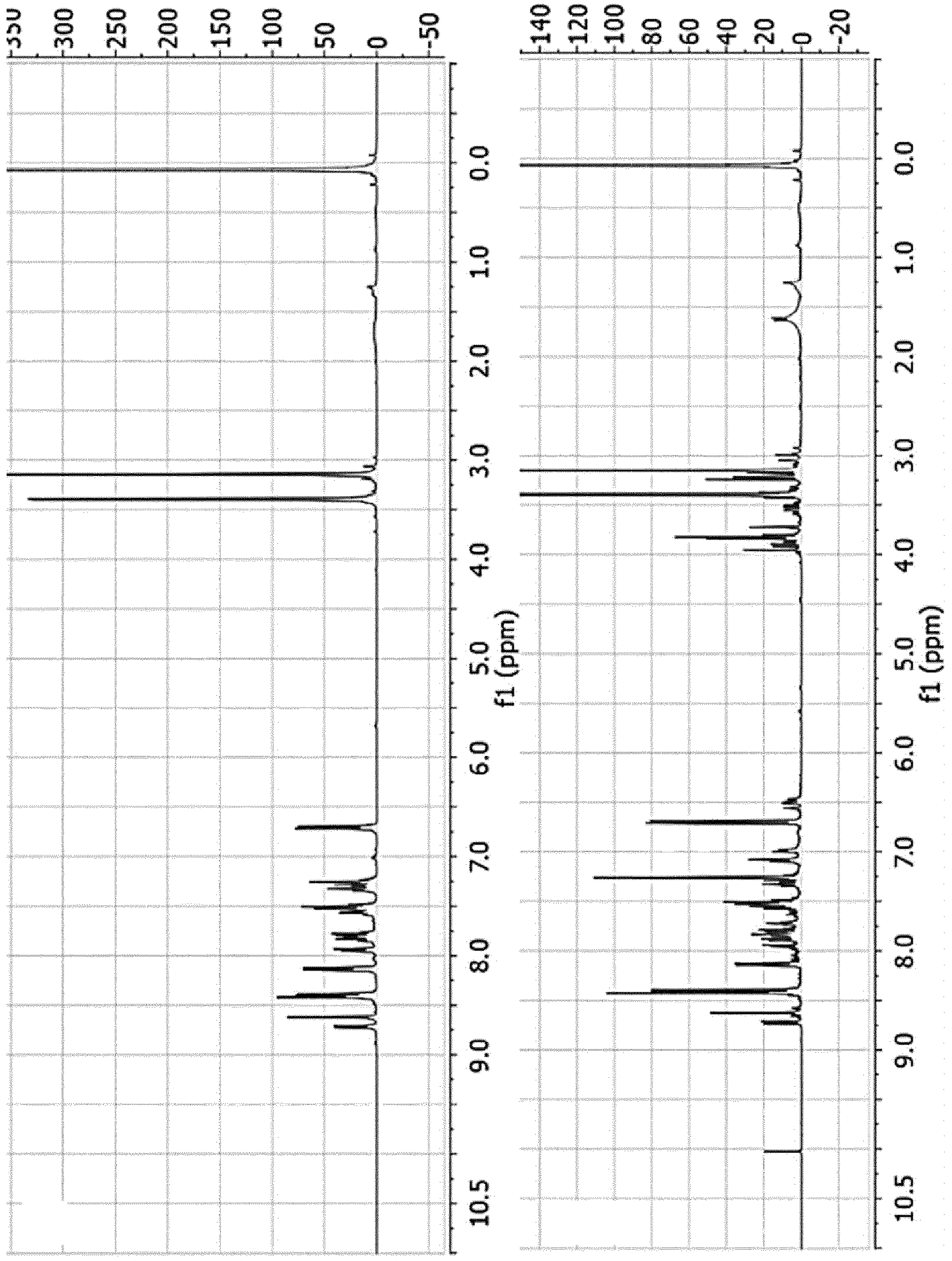


FIG. 30

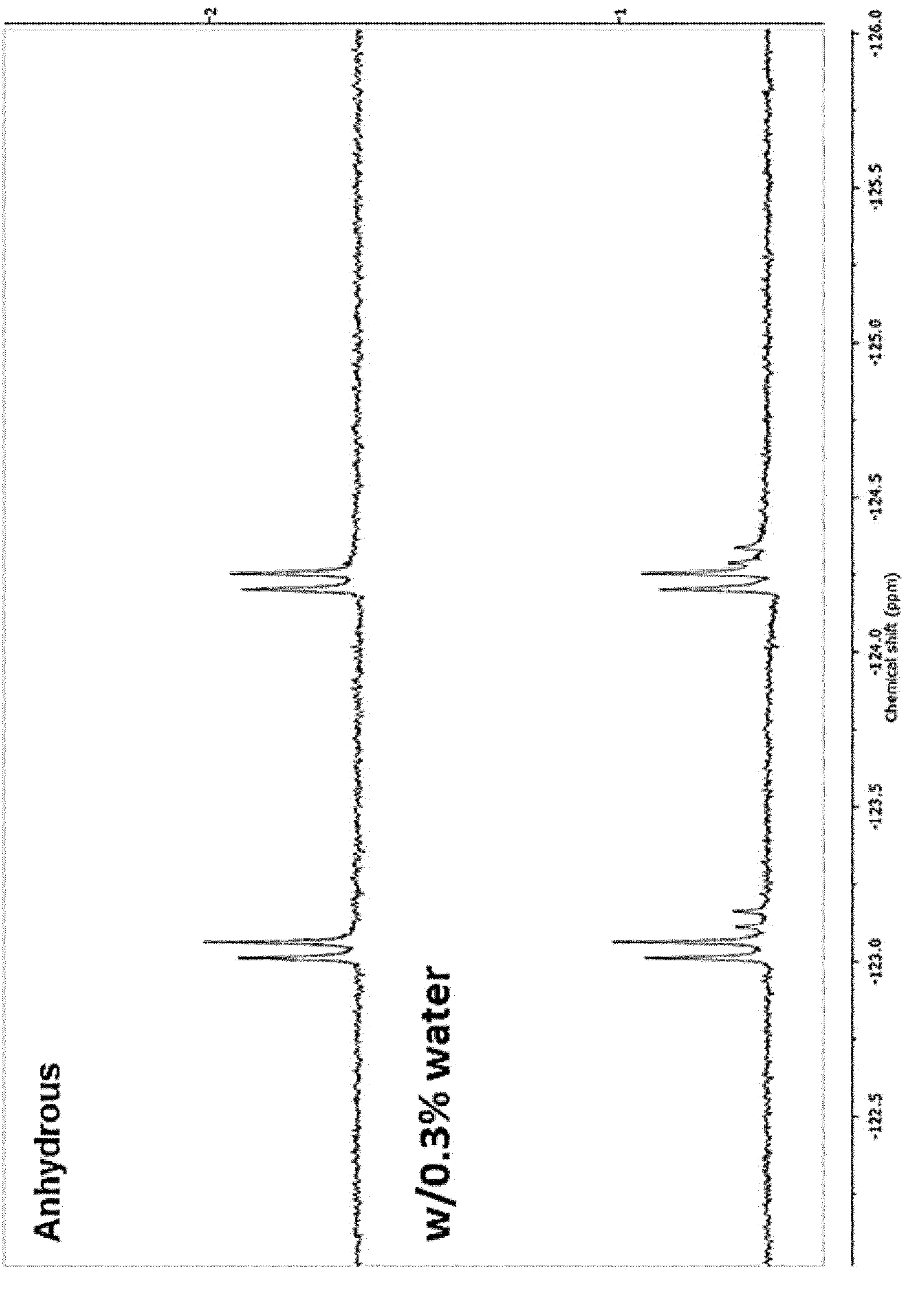
Anhydrous
w/0.3% water
2
1
-126.0
-125.5
-125.0
-124.5
-124.0
-123.5
-123.0
-122.5
Chemical shift (ppm)

FIG. 31

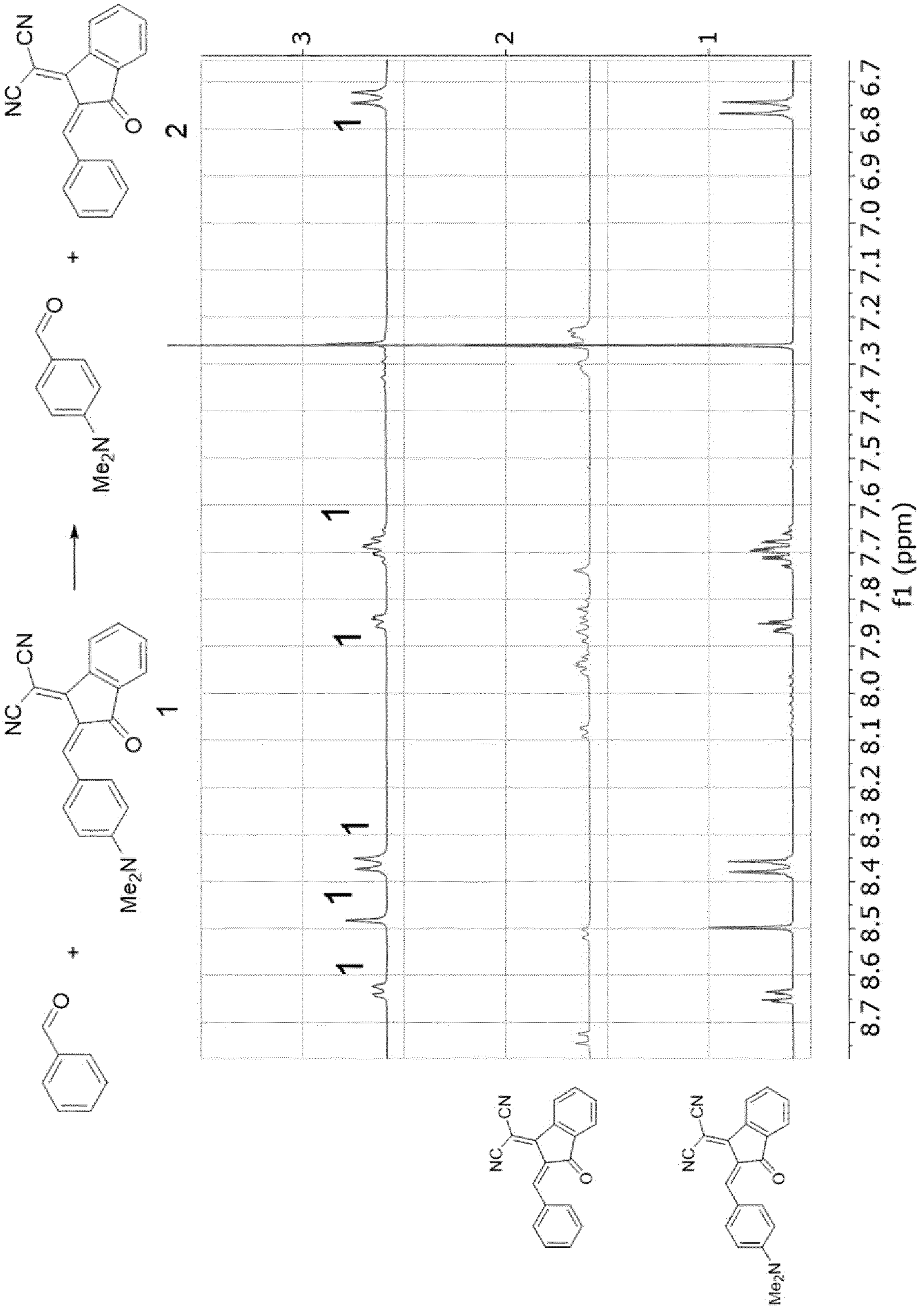
1
2
3
2
1
8.7 8.6 8.5 8.4 8.3 8.2 8.1 8.0 7.9 7.8 7.7 7.6 7.5 7.4 7.3 7.2 7.1 7.0 6.9 6.8 6.7
f1 (ppm)

FIG. 32

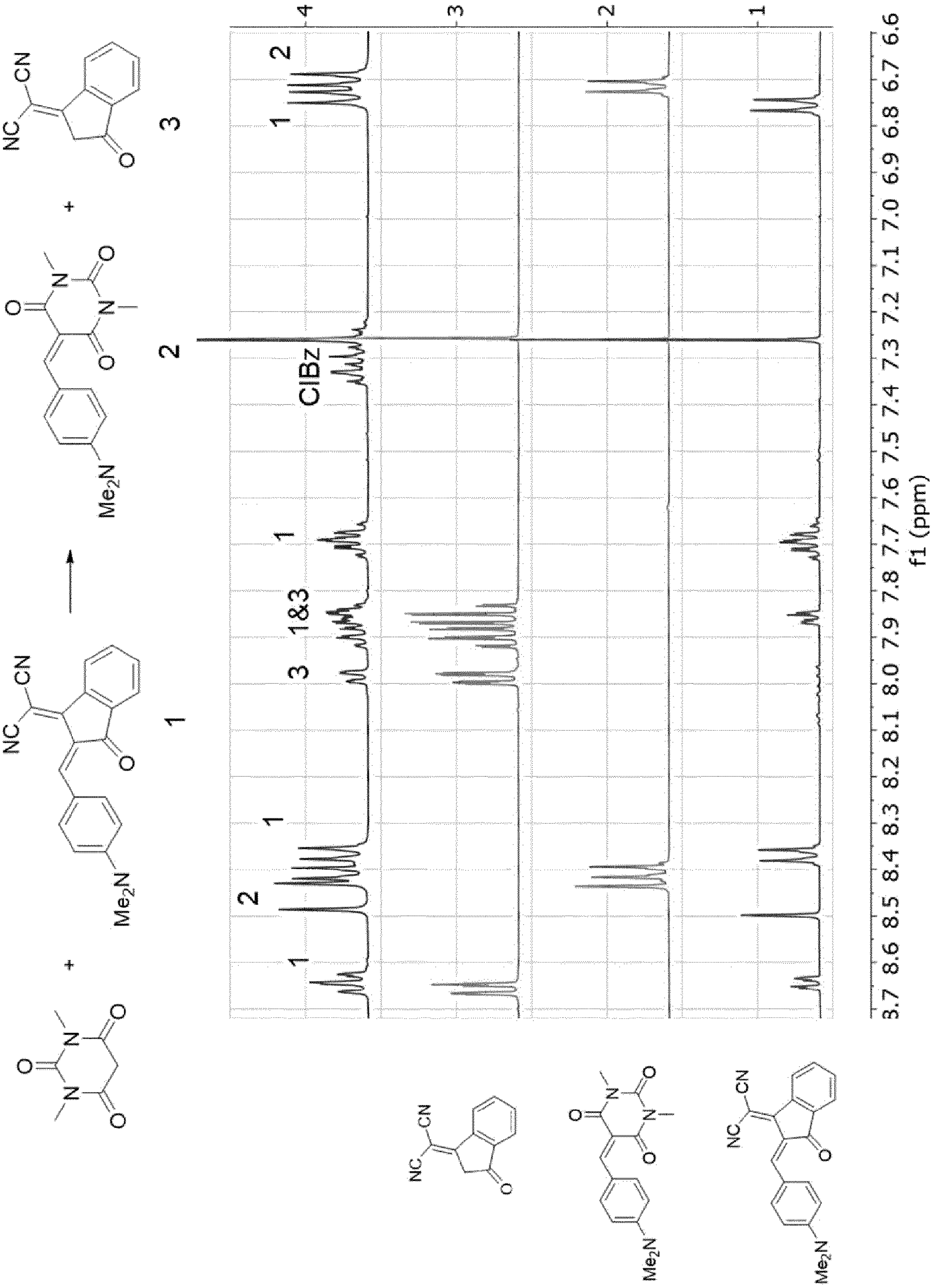
1
2
3
4
3
2
1
2
1
1
1&3
3
1
2
1
ClBz
6.6 6.7 6.8 6.9 7.0 7.1 7.2 7.3 7.4 7.5 7.6 7.7 7.8 7.9 8.0 8.1 8.2 8.3 8.4 8.5 8.6 8.7
f1 (ppm)

FIG. 33

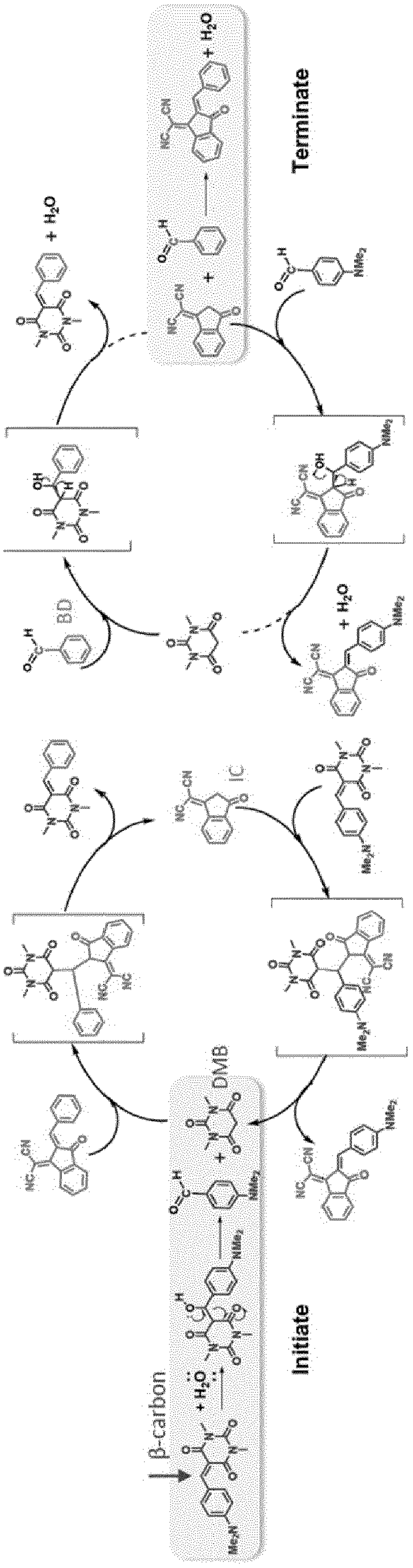


FIG. 34

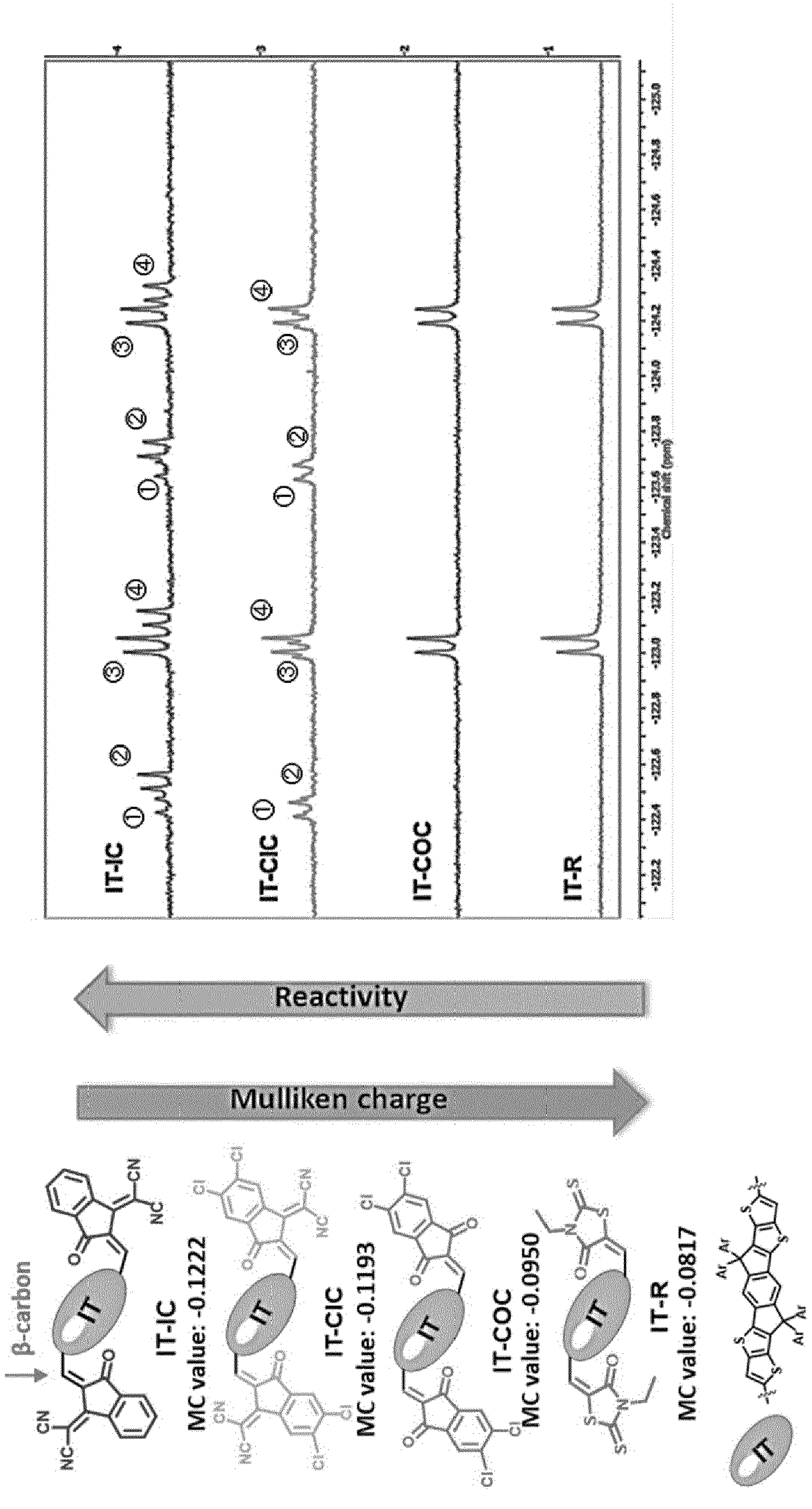
β-carbon
IT-IC
MC value: -0.1222
IT-CIC
MC value: -0.1193
IT-COC
MC value: -0.0950
IT-R
MC value: -0.0817
Mulliken charge
Reactivity
IT-IC
IT-CIC
IT-COC
IT-R
Chemical shift (ppm)

FIG. 35

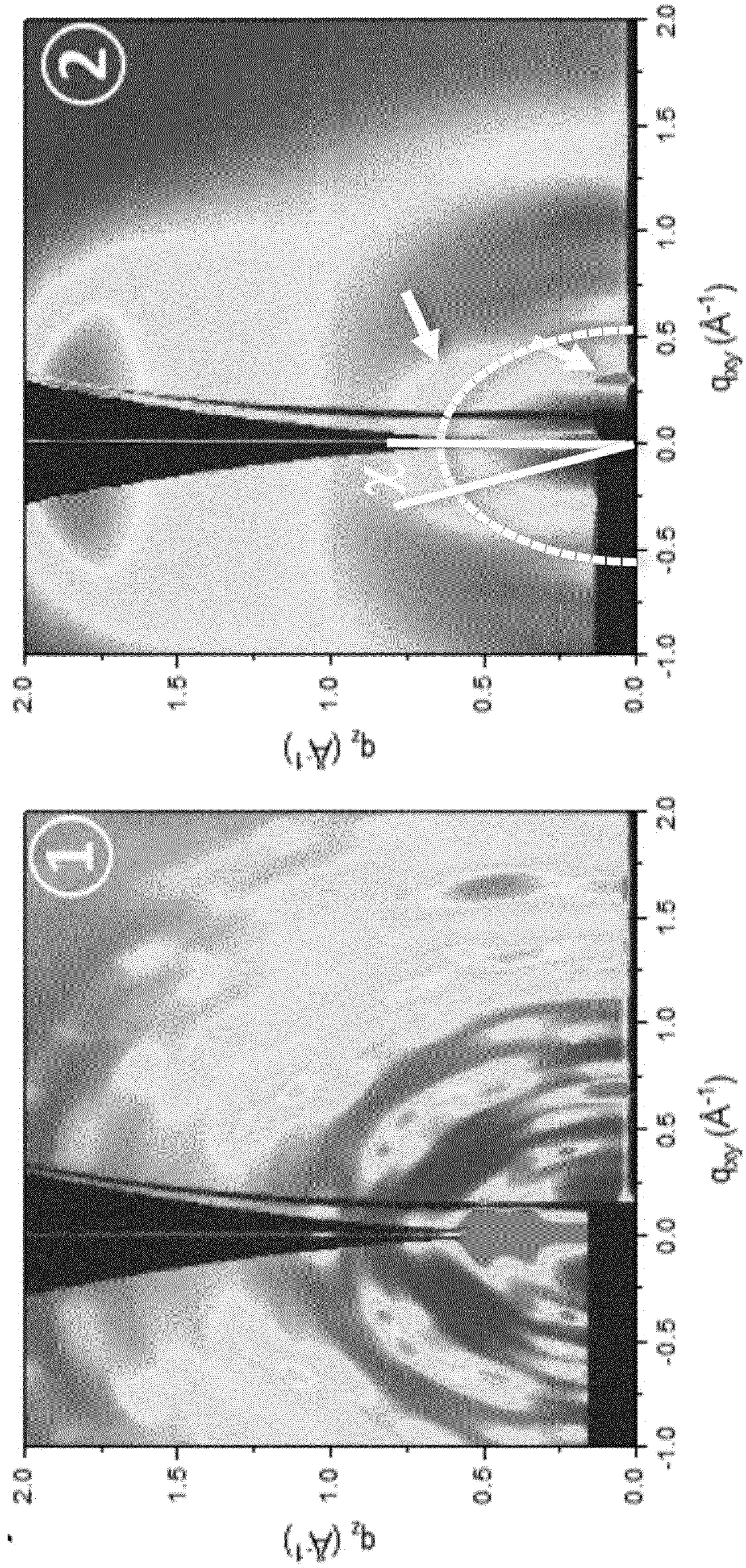


FIG. 36

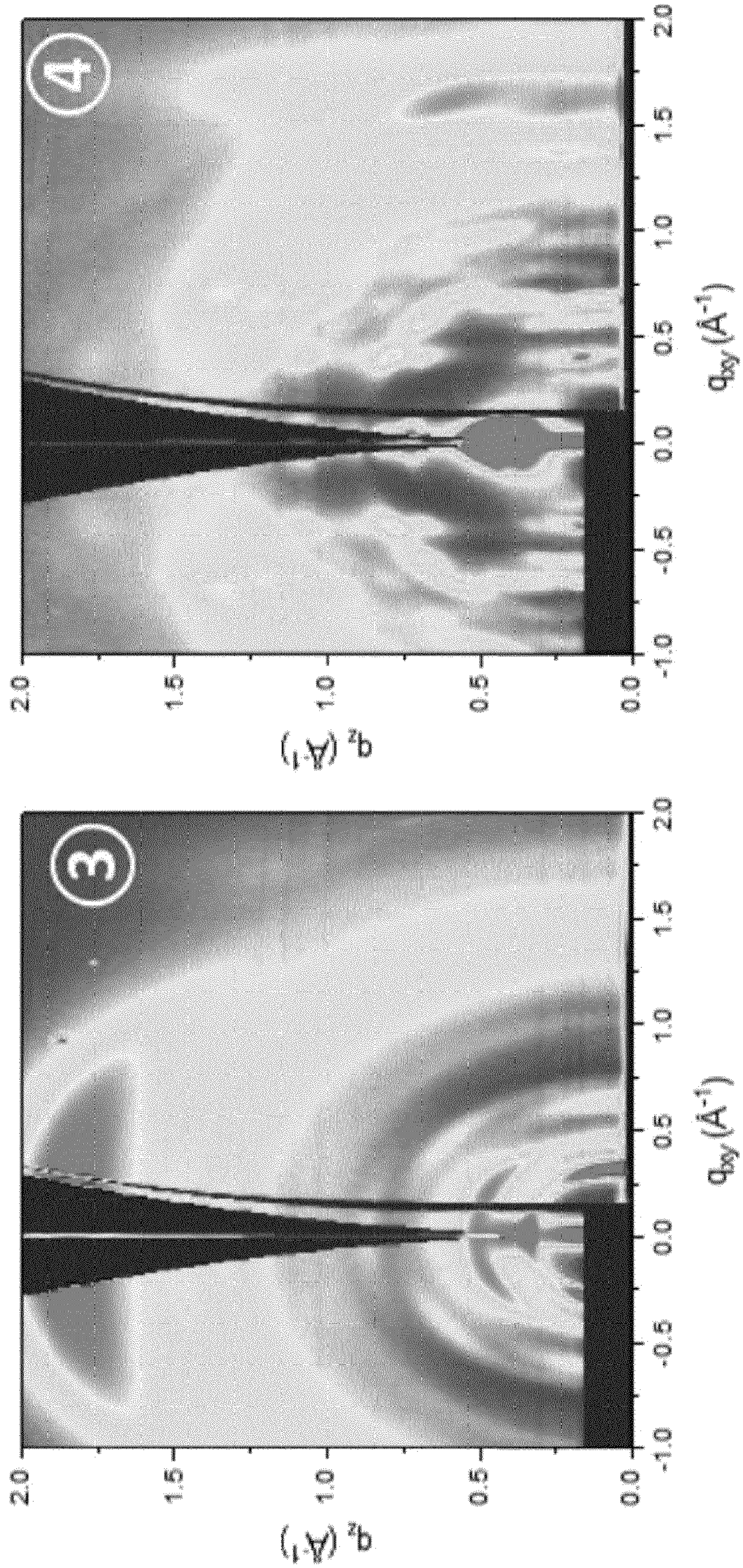


FIG. 37

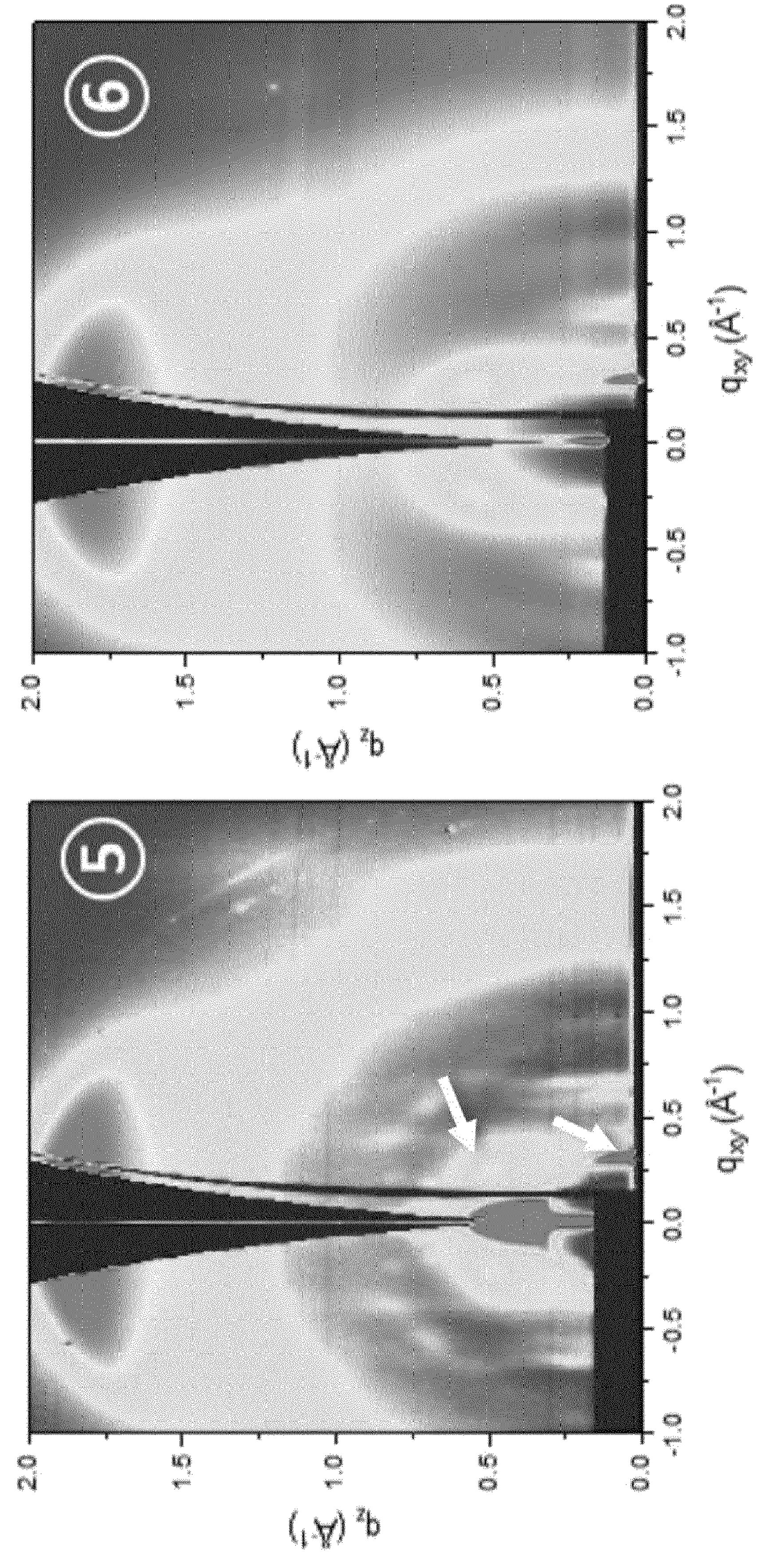


FIG. 38

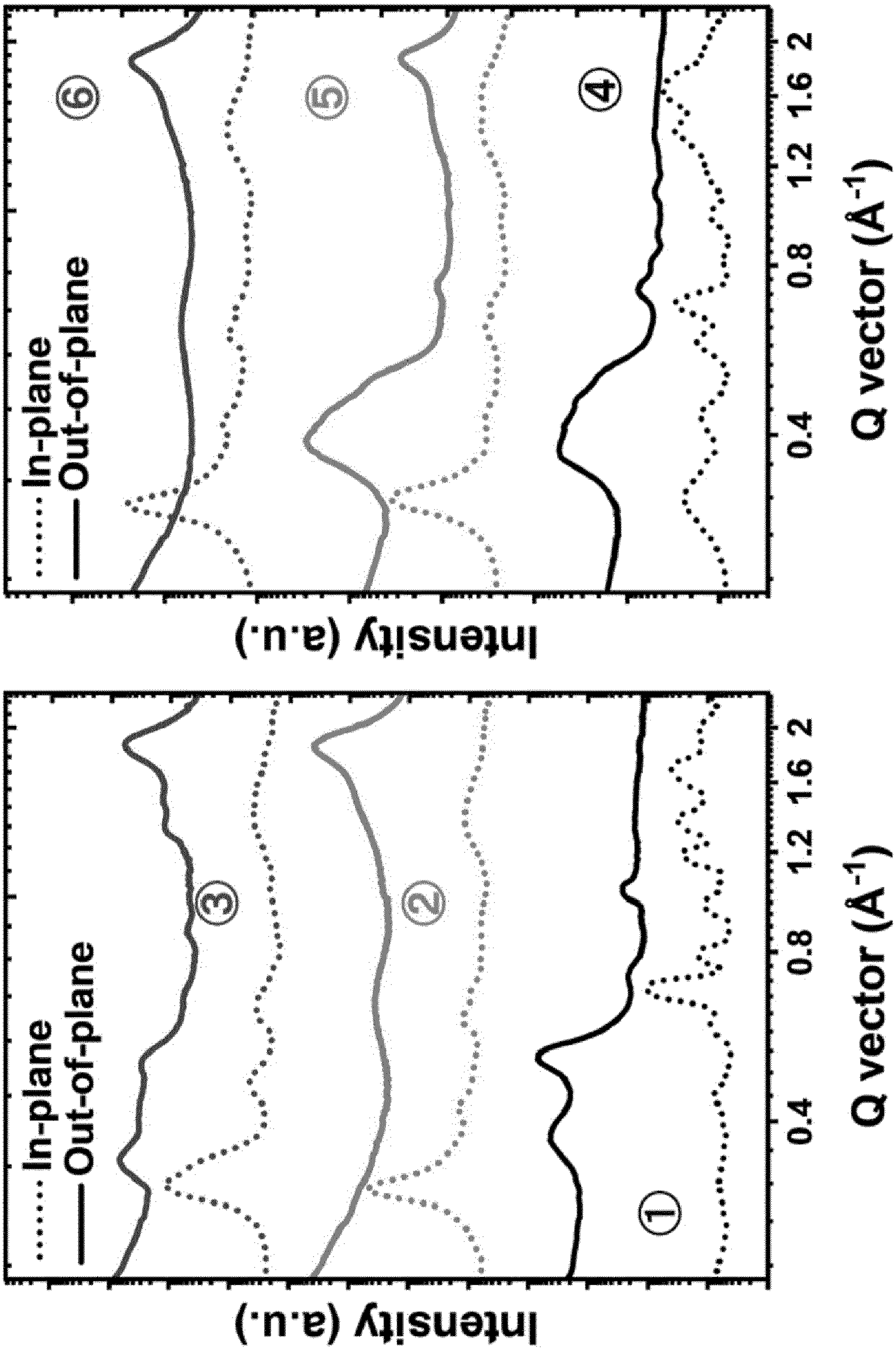
In-plane
Out-of-plane
Intensity (a.u.)
Q vector (Å$^{-1}$)
0.4
0.8
1.2
1.6
2
①
②
③
④
⑤
⑥

FIG. 39

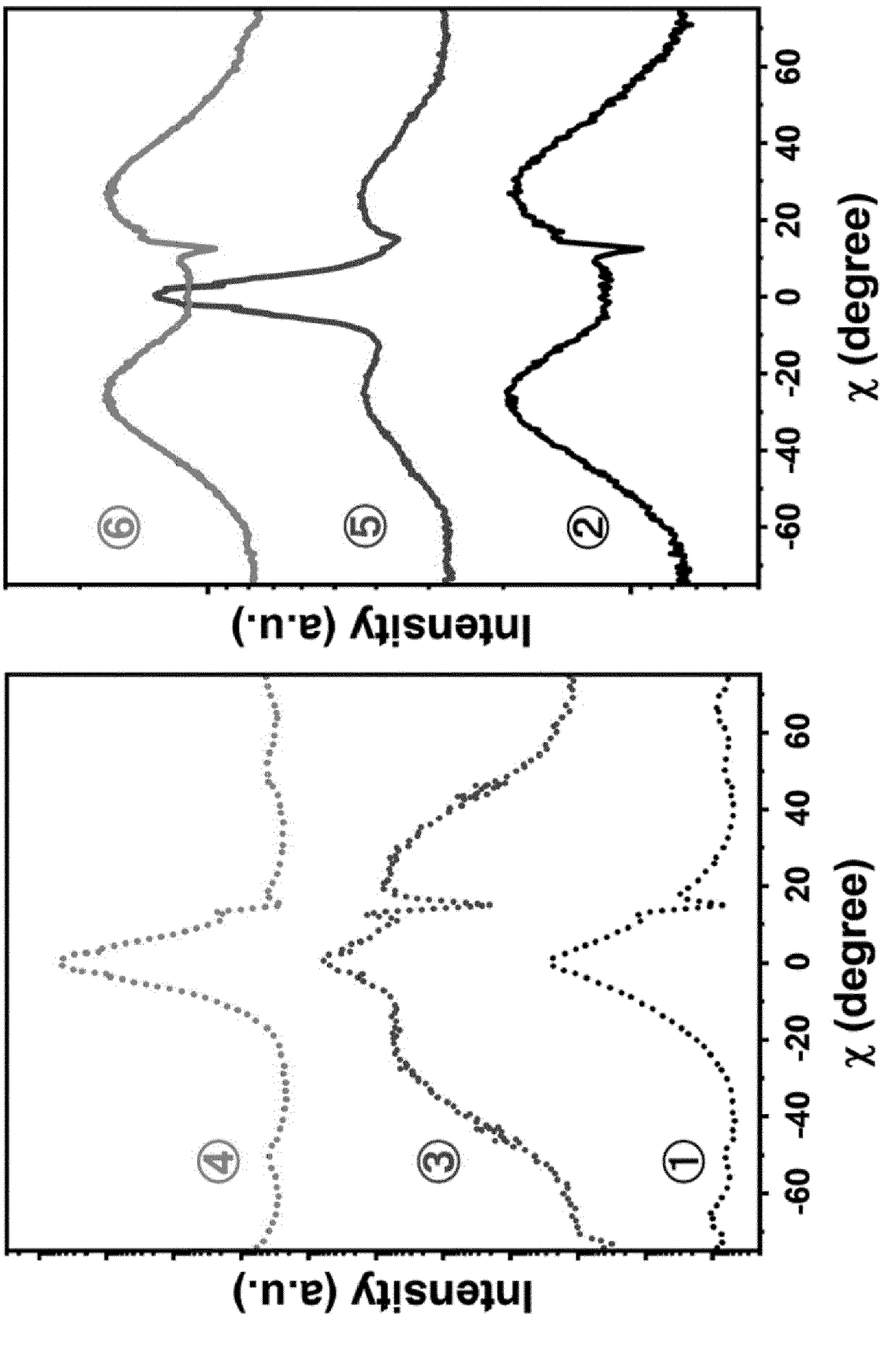


FIG. 40

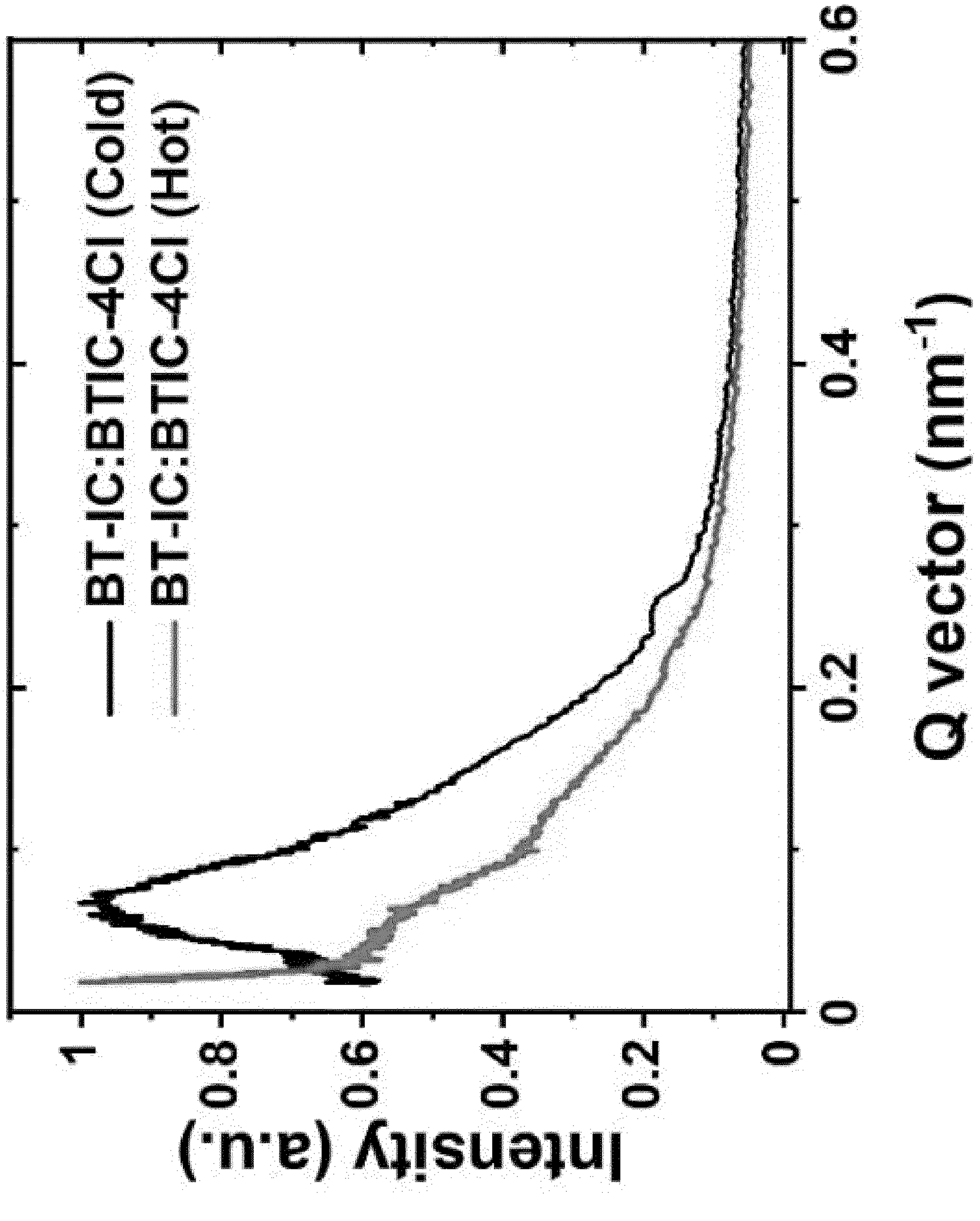
BT-IC:BTIC-4Cl (Cold)
BT-IC:BTIC-4Cl (Hot)
Intensity (a.u.)
Q vector (nm$^{-1}$)
0
0.2
0.4
0.6
0.8
1

FIG. 41

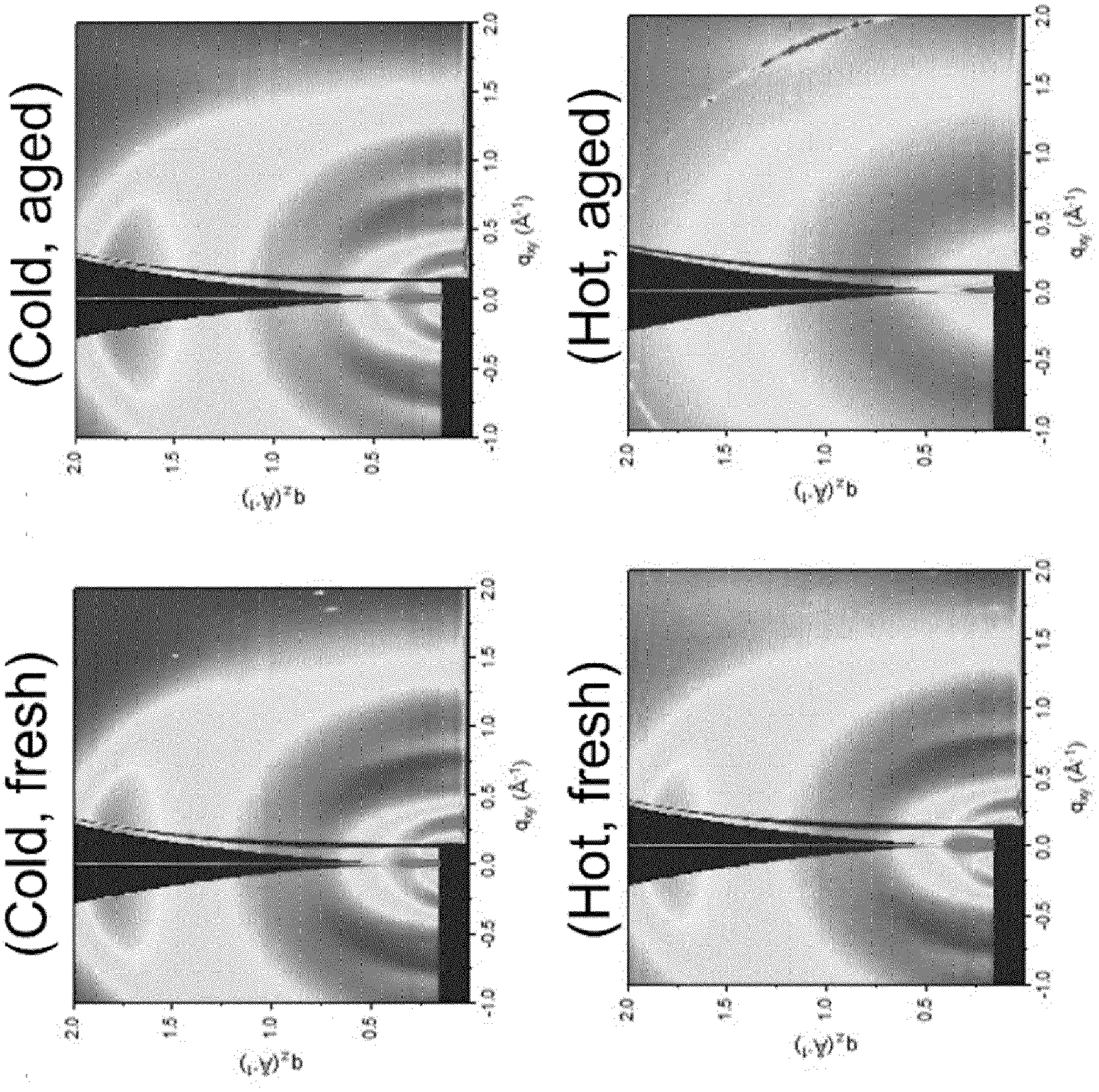
(Cold, fresh)
(Cold, aged)
(Hot, fresh)
(Hot, aged)
$q_z$ (Å$^{-1}$)
$q_{xy}$ (Å$^{-1}$)
2.0
1.5
1.0
0.5
-1.0
-0.5
0.0
0.5
1.0
1.5
2.0

FIG. 42

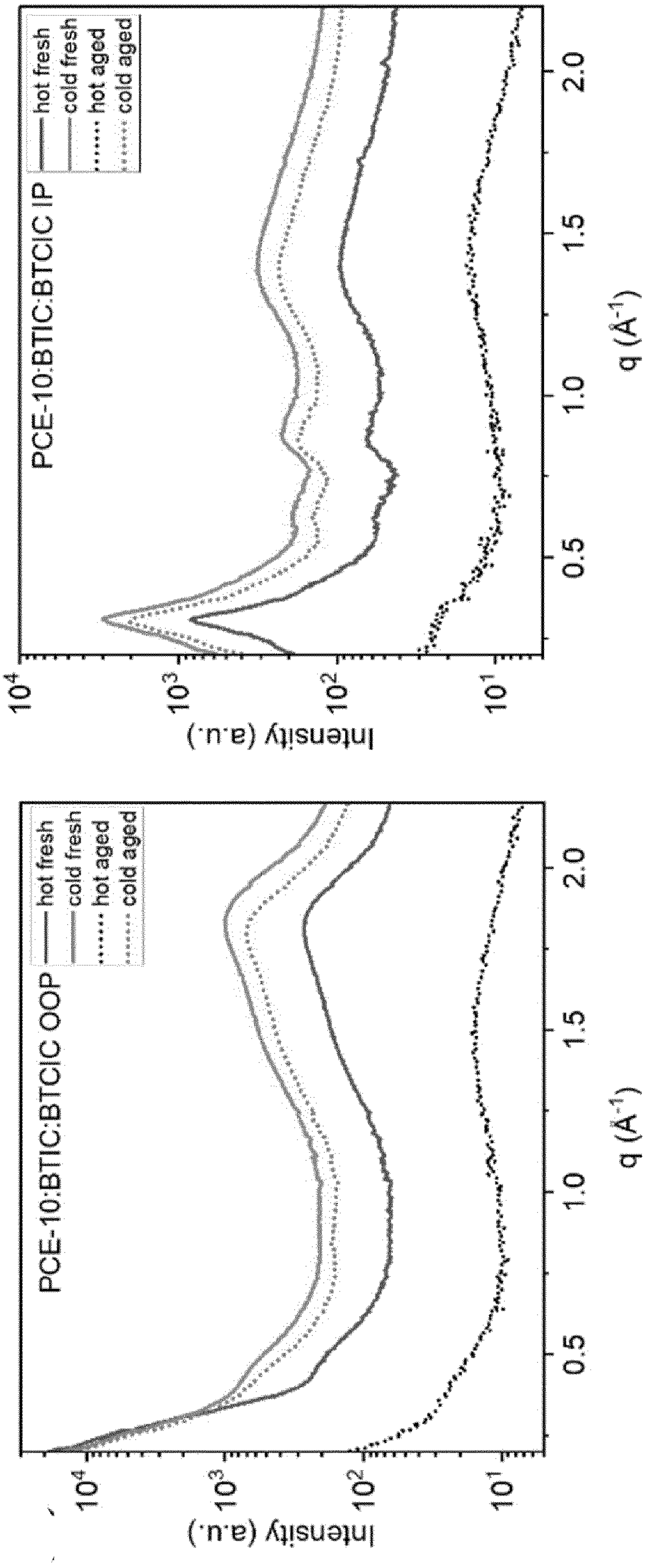
PCE-10:BTIC:BTCIC OOP
hot fresh
cold fresh
hot aged
cold aged
Intensity (a.u.)
q (Å$^{-1}$)
PCE-10:BTIC:BTCIC IP
hot fresh
cold fresh
hot aged
cold aged
Intensity (a.u.)
q (Å$^{-1}$)

FIG. 43

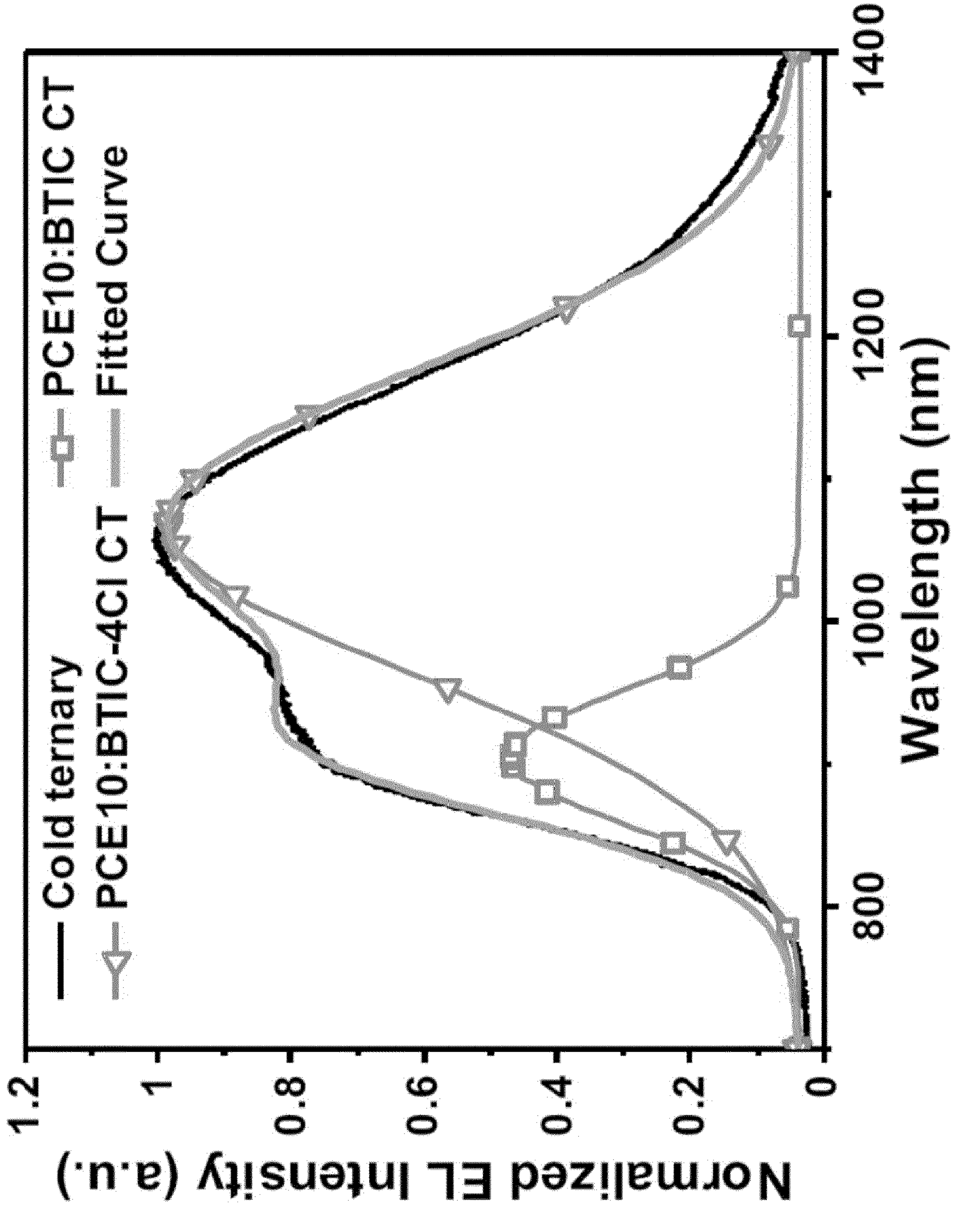
Cold ternary
PCE10:BTIC-4Cl CT
PCE10:BTIC CT
Fitted Curve
Normalized EL Intensity (a.u.)
0
0.2
0.4
0.6
0.8
1
1.2
Wavelength (nm)
800
1000
1200
1400

FIG. 44

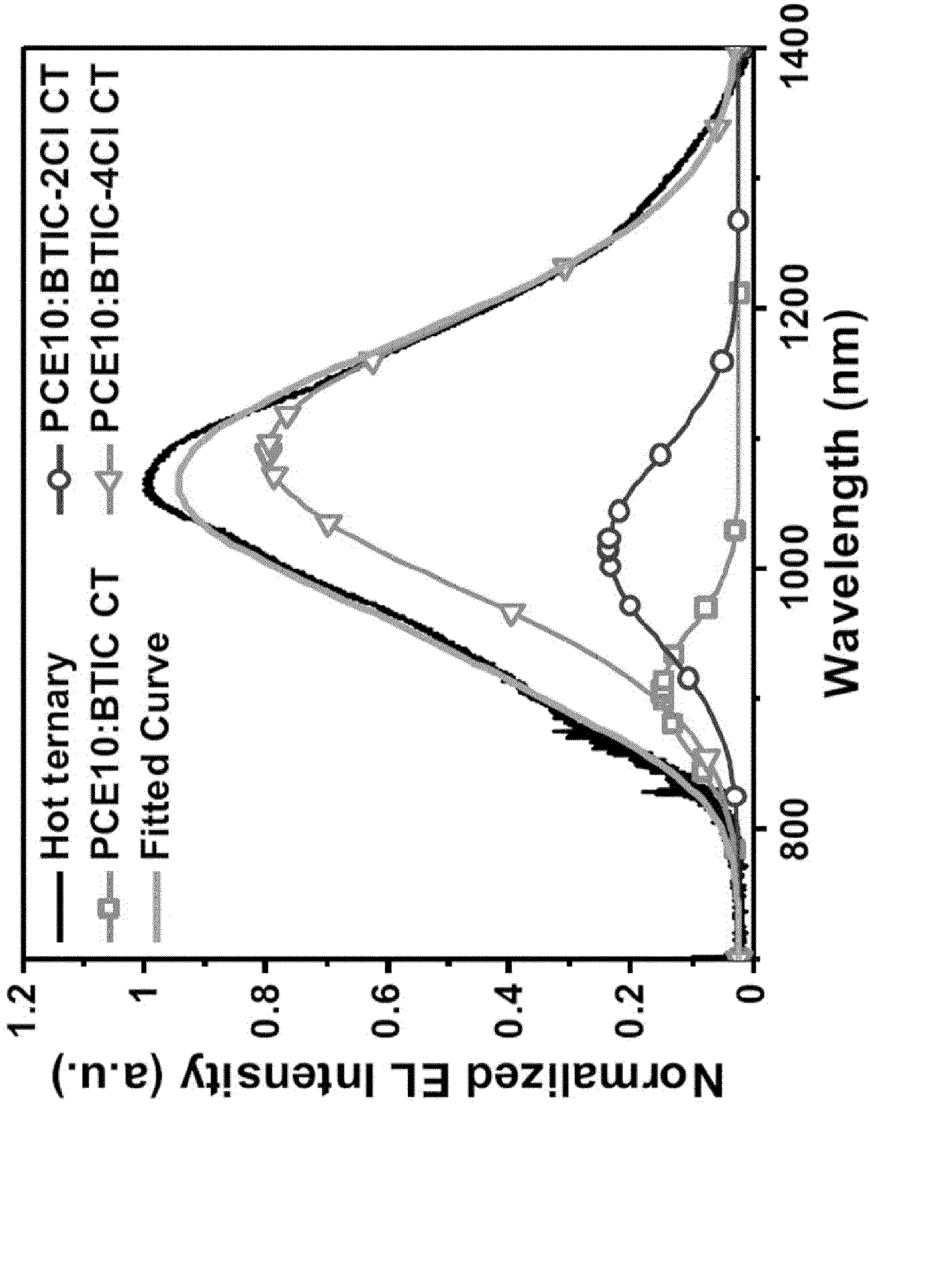
Hot ternary
PCE10:BTIC CT
Fitted Curve
PCE10:BTIC-2Cl CT
PCE10:BTIC-4Cl CT
Normalized EL Intensity (a.u.)
0
0.2
0.4
0.6
0.8
1
1.2
Wavelength (nm)
800
1000
1200
1400

FIG. 45

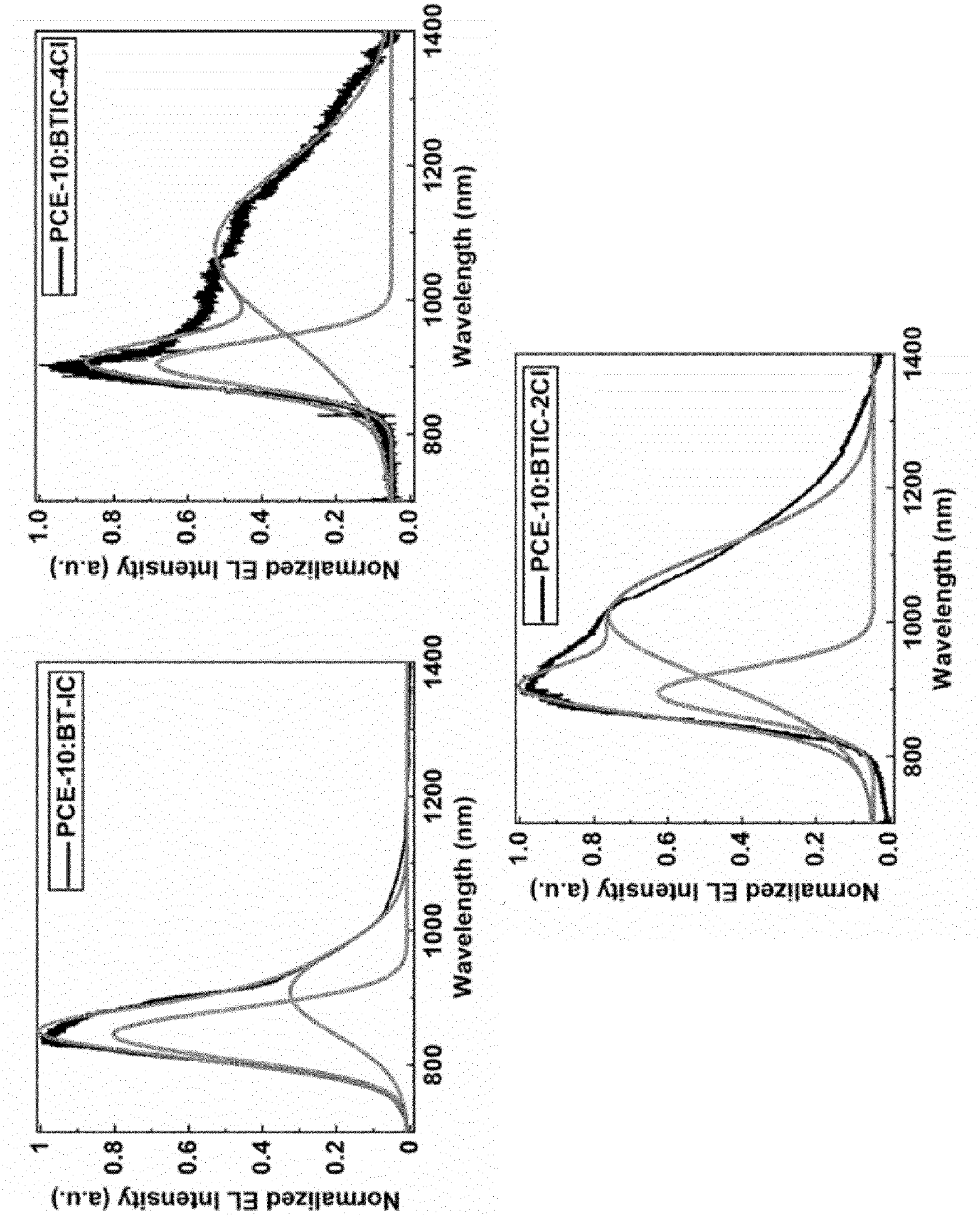
PCE-10:BT-IC
PCE-10:BTIC-4Cl
PCE-10:BTIC-2Cl
Normalized EL Intensity (a.u.)
Wavelength (nm)
800
1000
1200
1400
0
0.2
0.4
0.6
0.8
1
0.0
1.0

FIG. 46

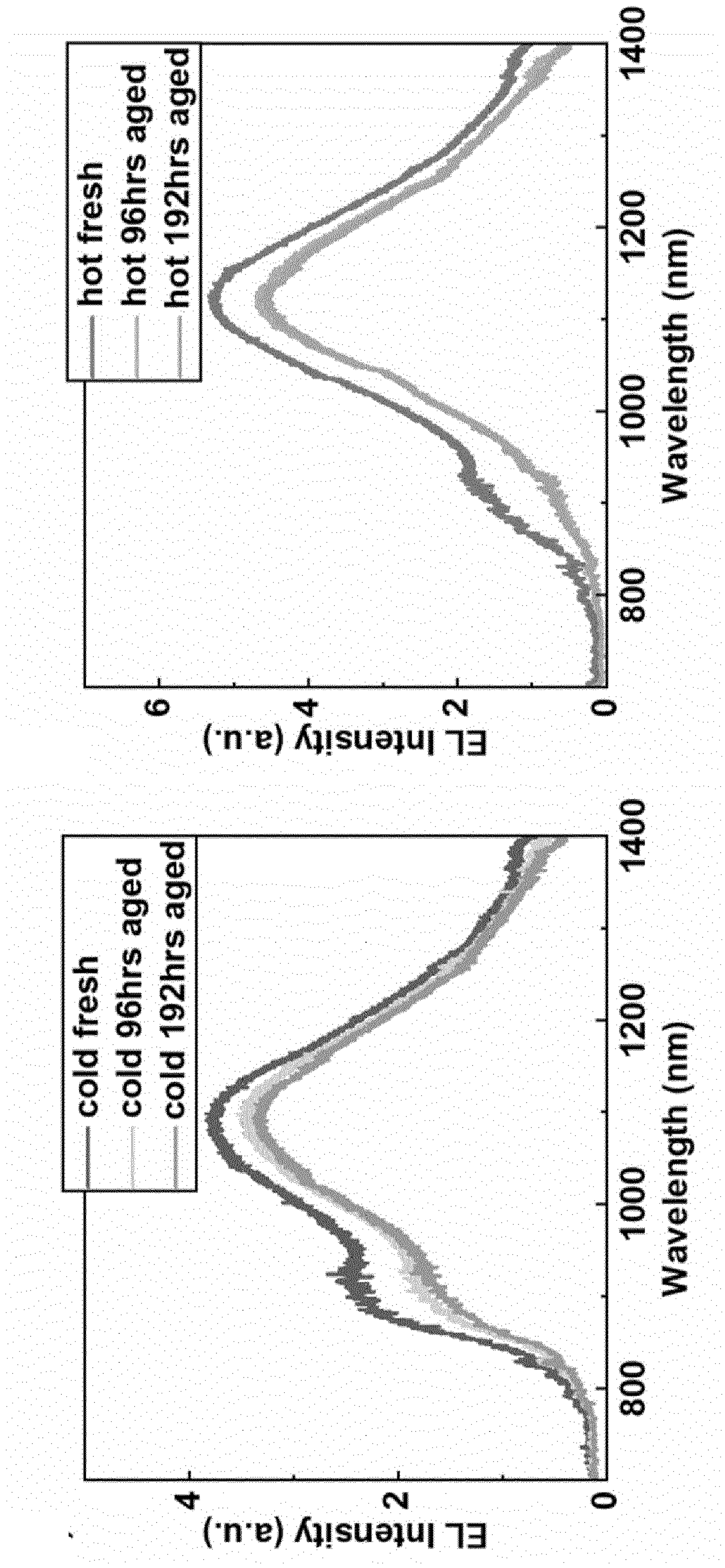
EL Intensity (a.u.)
0
2
4
6
800
1000
1200
1400
Wavelength (nm)
hot fresh
hot 96hrs aged
hot 192hrs aged
EL Intensity (a.u.)
0
2
4
800
1000
1200
1400
Wavelength (nm)
cold fresh
cold 96hrs aged
cold 192hrs aged

FIG. 47

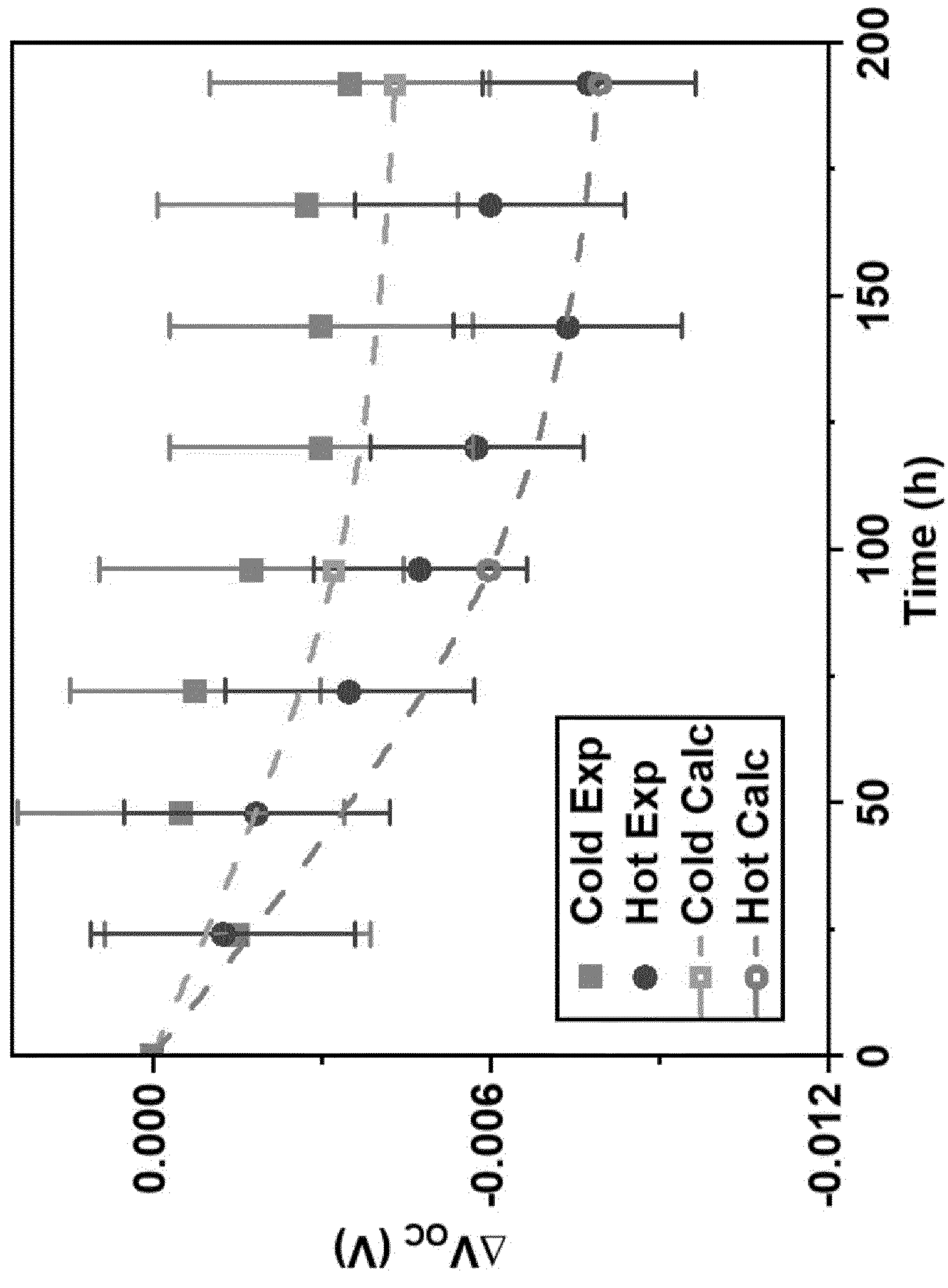
Cold Exp
Hot Exp
Cold Calc
Hot Calc
0.000
-0.006
-0.012
ΔVoc (V)
0
50
100
150
200
Time (h)

FIG. 48

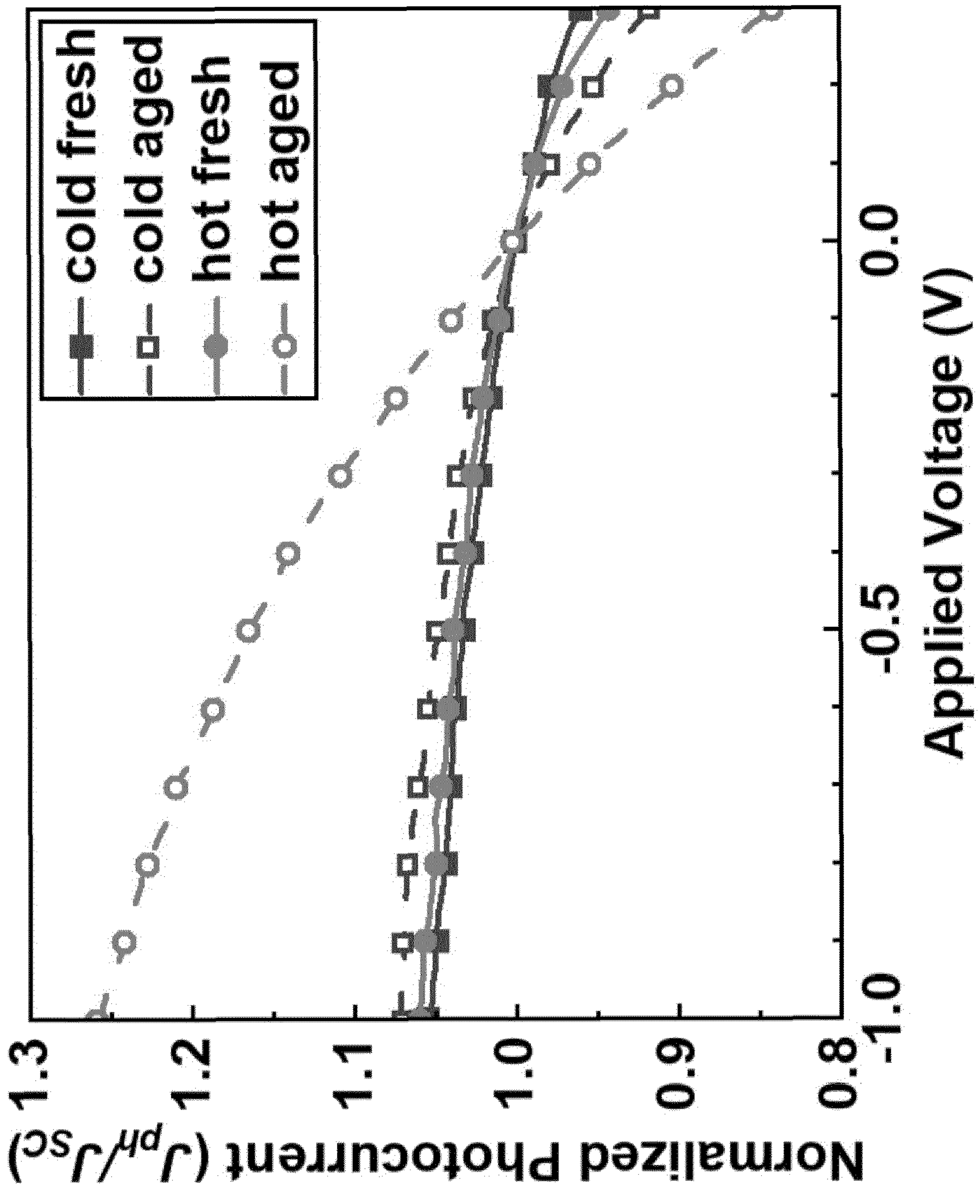
cold fresh
cold aged
hot fresh
hot aged
Normalized Photocurrent ($J_{ph}/J_{SC}$)
1.3
1.2
1.1
1.0
0.9
0.8
-1.0
-0.5
0.0
Applied Voltage (V)

FIG. 49

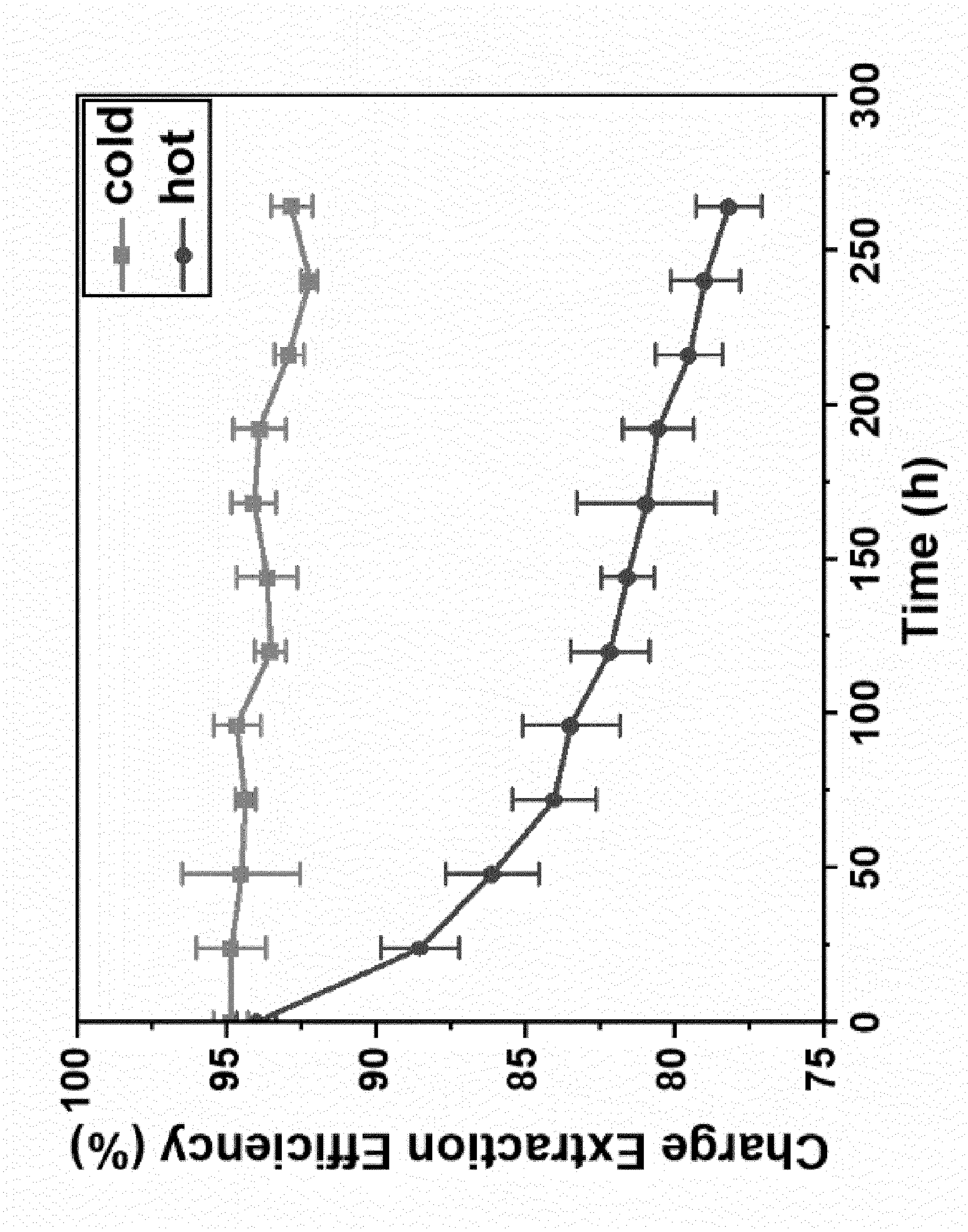
Charge Extraction Efficiency (%)
Time (h)
cold
hot
100
95
90
85
80
75
0
50
100
150
200
250
300

FIG. 50

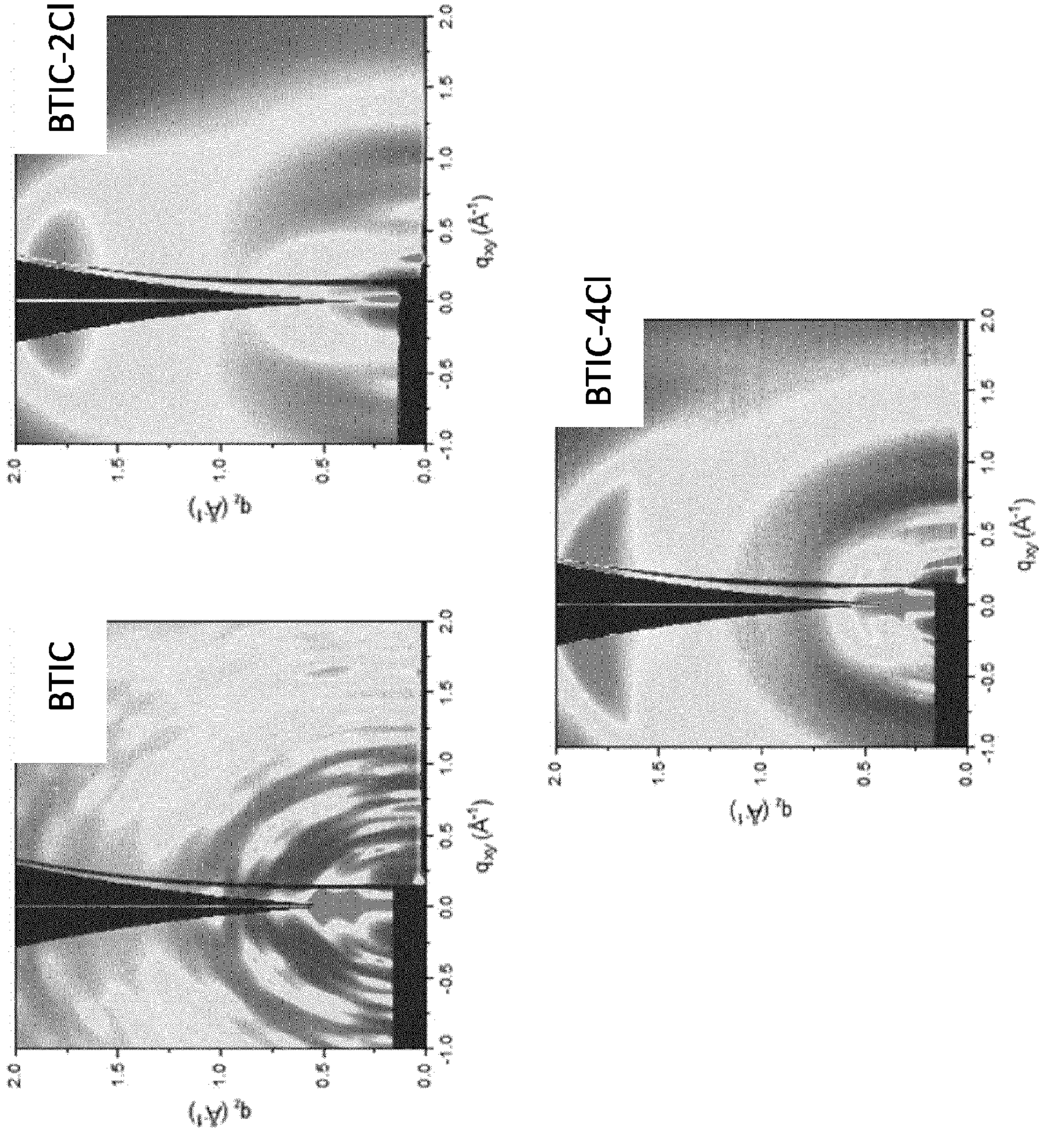


FIG. 51

Europäisches Patentamt
European Patent Office
Office européen des brevets

# EUROPEAN SEARCH REPORT

Application Number
EP 23 21 5041

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/135587 A1 (SMITH AUSTIN [US] ET AL) 5 May 2022 (2022-05-05)<br>* page 59; compound 3rd *<br>* page 60; compound 2nd *<br>* page 186; compound 1st *<br>* page 189; compound 2nd *<br>* page 221; compound 1st *<br>* page 350; compound last *<br>* page 353; compound 1st *<br>* page 358; compound 4th *<br>----- | 1,2,4-7 | INV.<br>H10K85/60<br><br>ADD.<br>H10K30/30 |
| X | CUI YONG ET AL: "Wide-gap non-fullerene acceptor enabling high-performance organic photovoltaic cells for indoor applications",<br>NATURE ENERGY, NATURE PUBLISHING GROUP UK, LONDON,<br>vol. 4, no. 9, 19 August 2019 (2019-08-19), pages 768-775, XP036885033,<br>DOI: 10.1038/S41560-019-0448-5<br>[retrieved on 2019-08-19]<br>* page 773, column 2, line 9; figure 2b; compounds IO-4Cl *<br>----- | 1-8 | TECHNICAL FIELDS SEARCHED (IPC)<br><br>H10K |
| X | CN 114 605 618 A (UNIV TIANJIN)<br>10 June 2022 (2022-06-10)<br>* compounds ZY-4Cl *<br>----- | 1 | |
| A | CN 114 621 275 B (GUANGZHOU FOLLOW SPOT SCIENCE AND TECH LIMITED COMPANY)<br>8 November 2022 (2022-11-08)<br>* page page 8; compounds 2nd, 3rd, 4th and 5th *<br>----- | 1-8 | |

The present search report has been drawn up for all claims

1

| Place of search | Date of completion of the search | Examiner |
|---|---|---|
| The Hague | 9 April 2024 | Fratiloiu, Silvia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 23 21 5041

This annex lists the patent family members relating to the patent documents cited in the above-mentioned European search report. The members are as contained in the European Patent Office EDP file on
The European Patent Office is in no way liable for these particulars which are merely given for the purpose of information.

09-04-2024

| Patent document cited in search report | Publication date | Patent family member(s) | Publication date |
|---|---|---|---|
| US 2022135587 A1 | 05-05-2022 | EP 4241318 A1 | 13-09-2023 |
| | | JP 2023549734 A | 29-11-2023 |
| | | KR 20230117123 A | 07-08-2023 |
| | | US 2022135587 A1 | 05-05-2022 |
| | | US 2022140266 A1 | 05-05-2022 |
| | | US 2022149298 A1 | 12-05-2022 |
| | | WO 2022098917 A1 | 12-05-2022 |
| CN 114605618 A | 10-06-2022 | NONE | |
| CN 114621275 B | 08-11-2022 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 63386569
- US 52653023
- US 8557400 B
- WO 2006095951 A
- US 20110037057 A
- US 6013982 A
- US 6087196 A
- US 6337102 B, Forrest
- US 7431968 B
- US 6294398 B
- US 6468819 B
- US 7968146 B
- US 2007023098 W
- US 2009042829 W


### Non-patent literature cited in the description


- **MATEKER, W. R. et al.** *Energy Environ. Sci.,* 2013, vol. 6, 2529-2537
- **SALZMAN, R. F. et al.** *Org. Electron.,* 2005, vol. 6, 242-246
- **TETREAULT et al.** *Org. Electron.,* 2021, vol. 92, 106091
- **WANG, N. et al.** *Sol. Energy Mater. Sol. Cells,* 2014, vol. 125, 170-175
- **FORREST, S. R. et al.** *J. Appl. Phys.,* 1984, vol. 56, 543-551
- **LU, L. et al.** *Nat. Photonics,* 2014, vol. 8, 716-722
- **GASPARINI, N. et al.** *Nat. Rev. Mater.,* 2019, vol. 4, 229-242
- **YU, R. et al.** *Adv. Energy Mater.,* 2018, vol. 8, 1702814
- **LI, Y. et al.** *J. Am. Chem. Soc.,* 2019, vol. 141, 18204-18210
- **LI, Y. et al.** *Adv. Mater.,* 2018, vol. 30, 1804416
- **CUI, Y. et al.** *Adv. Mater.,* 2021, vol. 33, 2102420
- **ZUO, L. et al.** *Nat. Nanotechnol.,* 2022, vol. 17, 53-60
- **ZHAN, L. et al.** *Adv. Energy Mater.,* 2022, vol. 12, 2201076
- **HULTMARK, S. et al.** *Adv. Funct. Mater.,* 2020, vol. 30, 2005462
- **MING YAN et al.** *Tetrahedron,* 2015, vol. 71, 1425-30
- **ATZRODT et al.** *Angew. Chem. Int. Ed. (Reviews),* 2007, vol. 46, 7744-65
- **LI, Y. et al.** *J. Am. Chem. Soc.,* 2017, vol. 139, 17114-17119
- **LI, Y. et al.** *Energy Environ. Sci.,* 2017, vol. 10, 1610-1620
- **LIN, Y. et al.** *Adv. Mater.,* 2015, vol. 27, 1170-1174
- **DAI, S. et al.** *Adv. Mater.,* 2018, vol. 30, 1706571
- **YUAN, J. et al.** *Nat. Commun.,* 2019, vol. 10, 570
- **LIU, Q. et al.** *Joule,* 2021, vol. 5, 2365-2379
- **HUANG, X. et al.** *Appl. Phys. Lett.,* 2020, vol. 116, 153501
- **LI, Y. et al.** *Nat. Commun.,* 2021, vol. 12, 1-9
- **HUANG, X. et al.** *Joule,* 2022, vol. 6, 1581-1589
- **LI, Y et al.** *Nat. Rev. Mater.,* 2023, vol. 8, 186-201
- **CHE, Y et al.** *Angew. Chemie Int. Ed.,* 2021, vol. 60, 24833-24837
- **LI, M. et al.** *J. Mater. Chem.,* 2019, vol. A7, 8889-8896
- **ZHAO, W. et al.** *J. Am. Chem. Soc.,* 2017, vol. 139, 7148-7151
- **DING, K. et al.** *ACS Appl. Mater. \& Interfaces,* 2022, vol. 14, 5692-5698
- **JIANG, Y. et al.** *Mater. Horiz.,* 2019, vol. 6, 1438-1443
- **LIU, Z.-X. et al.** *Nat. Commun.,* 2021, vol. 12, 3049
- **ALDRICH, T. J. et al.** *J. Am. Chem. Soc.,* 2019, vol. 141, 3274
- **ZHU, X. et al.** *ACS Appl. Energy Mater.,* 2019, vol. 2, 7602-7608
- **XU, S. et al.** *Angew. Chemie Int. Ed.,* 2022, vol. 61, e202202492
- **ZHANG, H. et al.** *Adv. Mater.,* 2018, vol. 30, 1800613
- **CUI, Y et al.** *Nat. Energy,* 2019, vol. 4, 768-775
- **MULLIKEN, R. S.** *J. Chem. Phys.,* 1955, vol. 23, 1833-1840
- **XIAO, L. et al.** *Adv. Funct. Mater.,* 2021, vol. 31, 2105304

- **CAI, Y. et al.** *Adv. Mater.,* 2021, vol. 33, 2101733
- **HUANG, X. et al.** *Mater. Horiz.,* 2020, vol. 7, 244-251
- **AMESON, C. et al.** *Appl. Phys. Lett.,* 2021, vol. 118, 63301
- **BARTESAGHI, D. et al.** *Nat. Commun.,* 2015, vol. 6, 7083
- **DIBB, G. F. A. et al.** *J. Phys. Chem. Lett.,* 2013, vol. 4, 803-808
- **HUANG, J. et al.** *Energy Environ. Sci.,* 2021, vol. 14, 3010-3018
- **HE, L. et al.** *IEEE J. Photovoltaics,* 2019, vol. 9, 1290-1296
- **LIN, Y. et al.** *Adv. Mater.,* 2015, vol. 27, 1170
- **LI, S. et al.** *Adv. Mater.,* 2016, vol. 28, 9423
- **CUI, Y. et al.** *Nat. Energy,* 2019, vol. 4, 768
- **LI, Y. et al.** *Energy Environ. Sci.,* 2017, vol. 10 (7), 1610-1620
- **LI, Y. et al.** *J. Am. Chem. Soc.,* 2017, vol. 139, 17114
- **LI, Y. et al.** *J. Mater. Chem. A,* 2016, vol. 4, 5890
- **SHI, X. et al.** *Adv. Funct. Mater.,* 2018, vol. 28, 1802324
- **LIU, S. et al.** *Nat. Photonics,* 2020, vol. 14, 300
- **LUO, M. et al.** *J. Energy Chem.,* 2020, vol. 42, 169
- **JIANG, K. et al.** *Joule,* 2019, vol. 3, 3020
- **HUANG, X. et al.** *Appl. Phys. Lett,* 2020, vol. 116 (15), 153501
- **BURLINGAME, Q. et al.** *Nature,* 2019, vol. 573, 394-397